(19) Europäisches Patentamt
European Patent Office
Office européen des brevets

(11) EP 4 222 141 B1

(12) **EUROPEAN PATENT SPECIFICATION**

(45) Date of publication and mention of the grant of the patent:
**06.08.2025 Bulletin 2025/32**

(21) Application number: **21811159.9**

(22) Date of filing: **30.09.2021**

(51) International Patent Classification (IPC):
***C07C 309/29*** *(2006.01)* ***C07C 309/32*** *(2006.01)*
***C08F 14/18*** *(2006.01)* ***C08F 14/22*** *(2006.01)*
***C08F 14/26*** *(2006.01)* ***C08F 114/26*** *(2006.01)*
***C08F 214/22*** *(2006.01)* ***C08F 2/26*** *(2006.01)*

(52) Cooperative Patent Classification (CPC):
(C-Sets available)
**C08F 114/26; C07C 317/14; C07C 317/22; C08F 14/18; C08F 14/22; C08F 14/26; C08F 214/22**
(Cont.)

(86) International application number:
**PCT/US2021/052961**

(87) International publication number:
**WO 2022/072693 (07.04.2022 Gazette 2022/14)**

(54) **LOW REACTIVITY HYDROCARBON DISPERSING AGENTS IN THE AQUEOUS POLYMERIZATION OF FLUOROPOLYMERS**

KOHLENWASSERSTOFFE MIT GERINGER REAKTIVITÄT ALS DISPERGIERMITTEL BEI DER WÄSSRIGEN POLYMERISATION VON FLUORPOLYMEREN

HYDROCARBURES À FAIBLE RÉACTIVITÉ EN TANT QU'AGENTS DISPERSANTS DANS LA POLYMÉRISATION AQUEUSE DE FLUOROPOLYMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2020 US 202063086331 P**

(43) Date of publication of application:
**09.08.2023 Bulletin 2023/32**

(73) Proprietor: **The Chemours Company FC, LLC**
**Wilmington, Delaware 19801 (US)**

(72) Inventors:
- **TANG, Phan Linh**
  **West Chester, PA 19382 (US)**
- **DEEMER, Michael**
  **Wilmington, Delaware 19806 (US)**
- **FRANCO, Vincent**
  **Wilmington, Delaware 19805 (US)**
- **FURYK, Steven**
  **Sewell, New Jersey 08080 (US)**
- **GETTY, Stephen James**
  **Wilmington, Delaware 19802 (US)**
- **KHASNIS, Dipti Dilip**
  **Wilmington, Delaware 19808 (US)**
- **LIU, Xiaolin**
  **Landenberg, PA 19350 (US)**
- **MARCHIONE, Alexander Anthony**
  **Blackwood, New Jersey 08012 (US)**
- **MATSNEV, Andrii**
  **The Plains, Ohio 45780 (US)**
- **MESSMORE, Benjamin Weaver**
  **Wilmington, Delaware 19803 (US)**
- **MORKEN, Peter A.**
  **Wilmington, Delaware 19810 (US)**
- **PARRISH, Cameron**
  **Wilmington, Delaware 19803 (US)**
- **SMITH, Adam Paul**
  **Parkesburg, WV 26101 (US)**
- **CAREY, Edward Patrick**
  **Atglen, Pennsylvania 19310 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(74) Representative: **Abitz & Partner**
**Postfach 86 01 09**
**81628 München (DE)**

(56) References cited:
**EP-A2- 3 081 987** **WO-A1-2021/070159**
**JP-A- 2002 308 914**

- **ANONYMOUS: "4-(4-Butan-2-ylphenyl) sulfonylbenzenesulfonate", 25 January 2017 (2017-01-25), pages 1 - 8, XP055879260, Retrieved from the Internet <URL:https:// pubchem.ncbi.nlm.nih.gov/compound/ 123772037> [retrieved on 20220114]**

(52) Cooperative Patent Classification (CPC): (Cont.)

C-Sets
**C08F 14/18, C08F 2/26;**
**C08F 14/22, C08F 2/26;**
**C08F 14/26, C08F 2/26;**
**C08F 214/22, C08F 214/26, C08F 214/28**

## Description

### FIELD

This invention relates to the aqueous emulsion polymerization of fluoropolymers and more particularly to the use of low reactivity hydrocarbon dispersing agents in the aqueous emulsion polymerization of fluoropolymers.

### BACKGROUND

As a replacement for fluorosurfactants as the stabilizing surfactant in the aqueous emulsion polymerization of fluoropolymers, the use of hydrocarbon surfactants has been disclosed. For fluoropolymers that are not perfluorinated, i.e., contain either a hydrocarbon monomer such an olefin or a hydrocarbon-containing fluoromonomer such as vinylidene fluoride (VF2), a variety of processes using hydrocarbon surfactants have been disclosed. For example, U.S. Patent 7,122,610 (Wille et al.) demonstrates the use of certain alkane sulfonates, sulfones, and disulfones in the polymerization to form non-elastomeric fluoropolymers containing at least 71 wt% vinylidene fluoride (VF2). U.S. Patents 8,080,621, 8,158,734, and 8,338,518 (Amin-Sanayei et al.) disclose the use of various nonionic surfactants containing segments of polyethylene glycol, polypropylene glycol and/or polytetramethylene glycol for the polymerization of VF2 homopolymers and copolymers. U.S. Patents 6,512,063 and 7,521,513 (Tang) discloses the use of hydrocarbon anionic surfactants such as sodium octyl sulfonate to polymerize fluoroelastomers that contain VF2 or olefin monomers.

However, issues arise when hydrocarbon surfactants are attempted for use for the manufacture of perfluoropolymers. For the polymerization of polytetrafluoroethylene (PTFE), Puts et al., in Chem. Rev. 2019, 119, 1763-1805, at page 1779 reports a number of problems with hydrocarbon surfactants. This reference states:
"The primary issue with using hydrocarbon dispersants (surfactants) is the probability of the formation of low-molecular-weight polymers due to hydrogen abstraction by the growing fluoromacroradical. Furthermore, the affinity of hydrocarbons for TFE and its polymers is limited, so the dispersive effects of hydrocarbon agents are not satisfactory."

Instability of the dispersion and undesirably large dispersion particle size are also described by Puts et al. as problems with the use of hydrocarbon surfactants for PTFE polymerization. Because hydrocarbon surfactants are prone to react with the initiator and/or the propagating fluoropolymer radical in the polymerization process, these reactions slow the rate of polymerization or can prevent polymerization altogether and significantly reduce the average molecular weight of the polymerized product.

For perfluoropolymers such as polytetrafluoroethylene (PTFE) and copolymers of tetrafluoroethylene (TFE) with hexafluoropropylene (HFP) and copolymers of TFE with perfluoro(alkyl vinyl ether), processes have been disclosed for the replacement of fluorosurfactants with hydrocarbon surfactants that employ additional process steps to control the difficulties of using hydrocarbon surfactants. WO2012/064846 A1, WO2012/064858 A1, and WO2012/064841 A1 (Brothers et al.) disclose processes using hydrocarbon surfactants that employ a nucleation step early in the process followed by a stabilization step that utilizes the delayed addition and metering of hydrocarbon surfactant as the polymerization is carried out. Treatments to reduce telogenic behavior of hydrocarbon surfactants are also disclosed by Brothers et al. Although the Brothers et al. processes employing the additional process steps and/or treatments for use of hydrocarbon surfactants can very successfully be used for the manufacture of fluoropolymers including perfluoropolymers, the Brothers et al. processes must be carefully controlled and do not enable polymerization processes to be run similarly to existing processes using fluorosurfactants.

JP 2002 308914 A discloses the polymerization of fluorinated olefins using fluorinated alkyl sulfonates as dispersing agents, whereby the fluorinated alkyl groups have a ratio of $CH_3$ groups to the sum of $CH_3$, $CH_2$ and CH groups that is of up to 50%.

### SUMMARY

The invention is based on the discovery that a class of hydrocarbon dispersing agents have low reactivity with the polymerization initiator and/or the propagating fluoropolymer radical in the emulsion polymerization of fluoromonomers. Embodiments of the process of the invention employing the low reactivity hydrocarbon dispersing agents can provide polymerization rates suitable for commercial production, fluoropolymer dispersions with high solids contents, and/or fluoropolymers with the desired average molecular weight for commercial use. The benefits provided by the present invention can be achieved with many different types of fluoropolymers including perfluoropolymers. Moreover, the process of the invention can be carried out if desired like existing processes that use fluorosurfactants, e.g., surfactant can be pre-charged into the aqueous medium prior to or simultaneous with commencement of polymerization.

A process in accordance with the invention polymerizes at least one fluoromonomer in an aqueous medium containing initiator and hydrocarbon dispersing agent to form an aqueous dispersion of particles of fluoropolymer. The hydrocarbon dispersing agent comprises a compound of formula I:

$$R - (XZ)_n \qquad I$$

wherein R is a hydrophobic hydrocarbon moiety that comprises one or more saturated or unsaturated, non-cyclic or cyclic aliphatic groups, the percentage of total $CH_3$ groups in relation to the total of $CH_3$, $CH_2$ and CH groups in said one or more aliphatic groups being at least 70%, said hydrophobic moiety being free of siloxane units;
wherein each X may be the same or different and represents an ionic hydrophilic moiety;
wherein each Z may be a same or different and represents one or more counter ions for said ionic hydrophilic moiety; and
wherein n is 1 to 3.

In one embodiment of the invention, the hydrophobic hydrocarbon moiety of the hydrocarbon dispersing agent is entirely aliphatic, and the hydrocarbon dispersing agent has a molecular weight without a counter ion Z of at least 170 g/mol.

In another embodiment of the invention, the hydrophobic hydrocarbon moiety of the hydrocarbon dispersing agent contains at least one aromatic group and the hydrocarbon dispersing agent has a molecular weight without a counter ion Z of at least 215 g/mol.

Preferably in the process of the invention, the hydrophobic hydrocarbon moiety in the hydrocarbon dispersing agent comprises no more than 3 consecutive $CH_2$ groups, more preferably, no more than 2 consecutive $CH_2$ groups, and most preferably, no consecutive $CH_2$ groups.

Preferably in the process of the invention, the hydrophobic hydrocarbon moiety comprises at least 3 $CH_3$ groups, more preferably, at least 4 $CH_3$ groups. One preferred range for the number of $CH_3$ groups in the hydrophobic hydrocarbon moiety is 3 to 12.

The ionic hydrophilic moiety X is preferably selected from the group consisting of acid groups and salts thereof, quaternary ammonium groups, amine-oxide groups, and tetrazole groups. In one preferred embodiment, the hydrophilic moiety and counter ion XY is a group of the formula $-A^{-}-Y^{+}$ wherein $A^{-}$ is carboxylate, sulfonate, sulfate, phosphonate, or phosphate, and wherein $Y^{+}$ is hydrogen, ammonium, quaternary ammonium, nitrogen heterocycle, alkali metal, or alkaline earth. Most preferably, wherein $A^{-}$ is sulfonate.

In a preferred embodiment of the invention, the hydrophobic hydrocarbon moiety is free of halogens. In another preferred embodiment, the one or more aliphatic groups in the hydrophobic hydrocarbon moiety are non-cyclic or cyclic alkyl groups.

In another preferred embodiment, the hydrophobic hydrocarbon moiety comprises one or more aromatic groups. In the embodiment that includes one or more aromatic groups, the one or more aromatic groups have a Hammett sigma plus value of greater than -1.0, preferably a Hammett sigma plus value of greater than -0.8, most preferably a Hammett sigma plus value of greater than 0.

In the embodiment that includes one or more aromatic groups, the hydrophobic hydrocarbon moiety is preferably free of methyl groups directed bonded to an aromatic group. It is also preferable in such embodiment for the hydrophobic hydrocarbon moiety to be free of benzylic hydrogen atoms. It is also preferable for the hydrophobic hydrocarbon moiety to be free of phenolic hydroxy groups.

In a preferred embodiment of the present invention, the hydrophobic hydrocarbon moiety of the hydrocarbon dispersing agent comprises 8 to 50 carbon atoms, preferably 10 to 40 carbon atoms, more preferably 12 to 30 carbon atoms, most preferably, 12 to 25 carbon atoms.

In a preferred embodiment of the present invention, the hydrophobic hydrocarbon moiety of the hydrocarbon dispersing agent is free of carbon-hydrogen bonds with bond dissociation energies to yield a hydrocarbon radical of less than 100 kcal/mol.

In a preferred embodiment of the present invention, hydrocarbon dispersing agent has a t-butyl hydroperoxide oxidative loss of less than 10 wt%.

In a preferred embodiment of the present invention, hydrocarbon dispersing agent comprises a substituted moiety of the formula:

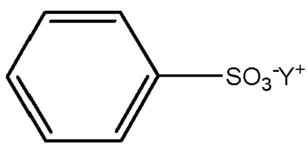

wherein $Y^{+}$ is hydrogen, ammonium, quaternary ammonium, nitrogen heterocycle, alkali metal, or alkaline earth.

In another preferred embodiment, initiator contained in the aqueous medium in the process is organic peroxide.

In a preferred embodiment of the present invention, the hydrocarbon dispersing agent is a compound of formula II:

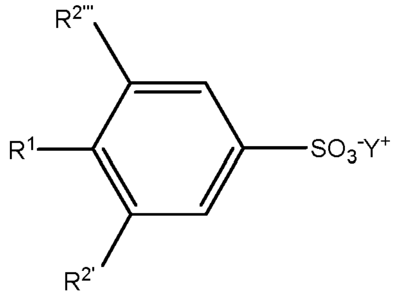

II

wherein $R^{2'}$ and $R^{2''}$ are the same or different and are saturated or unsaturated, non-cyclic or cyclic aliphatic groups having 4 to 16 carbon atoms with the percentage of total $CH_3$ groups in relation to the total of $CH_3$, $CH_2$ and CH groups in the $R^{2'}$ and $R^{2''}$ groups being at least 70%, or $R^{2'}$ and $R^{2''}$ may be joined together to form an saturated or unsaturated aliphatic ring that may contain ether or ester linkages, with the proviso that the percentage of total $CH_3$ groups in relation to the total of $CH_3$, $CH_2$ and CH groups in said ring is at least 70%:

wherein $R^1$ is hydrogen, methoxy, ethoxy or phenoxy; and

wherein $Y^+$ is hydrogen, ammonium, quaternary ammonium, nitrogen heterocycle, alkali metal, or alkaline earth.

Preferably, in the compound of formula II, $R^{2'}$, and $R^{2''}$ are the same or different and are t-butyl-, t-butoxy, 2,3,3-trimethyl-2-butyl, or 2,3,3-trimethyl-2-butoxy, or -CO(O)C(CH3)3, and wherein $R^1$ is hydrogen or methoxy, and wherein $Y^+$ is hydrogen, ammonium, or alkali metal.

In another preferred embodiment, in the compound of formula II, $R^{2'}$ and $R^{2''}$ are the same or different and are t-butyl or 2,3,3-trimethyl-2-butyl, $R^1$ is hydrogen, and $Y^+$ is hydrogen, ammonium, or alkali metal.

In another preferred embodiment, in the compound of formula II, $R^{2'}$ and $R^{2''}$ are the same or different and are t-butyl or 2,3,3-trimethyl-2-butyl, $R^1$ is methoxy and $Y^+$ is hydrogen, ammonium, or alkali metal.

In another preferred embodiment, in the compound of formula II, $R^{2'}$ and $R^{2''}$ are both t-butyl, $R^1$ is hydrogen or methoxy, and $Y^+$ is hydrogen, ammonium, or alkali metal.

In a preferred embodiment of the present invention, the hydrocarbon dispersing agent is a compound of the formula III:

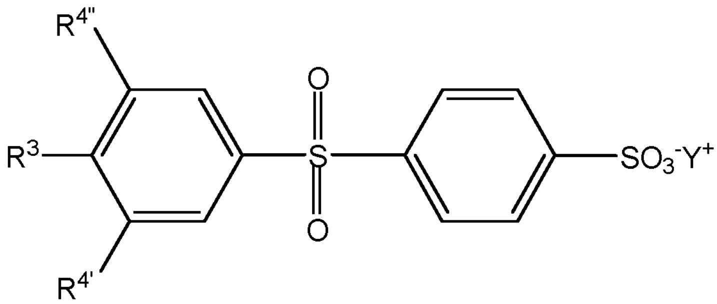

III

wherein $R^3$, $R^{4'}$, and $R^{4''}$ are the same or different and are hydrogen or saturated or unsaturated, non-cyclic or cyclic aliphatic groups having 4 to 16 carbon atoms with the percentage of total $CH_3$ groups in relation to the total of $CH_3$, $CH_2$ and CH groups the $R^3$, $R^{4'}$, and $R^{4''}$ groups being at least 70%, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth.

Preferably, in the compound of formula III, $R^3$, $R^{4'}$, and $R^{4''}$ are the same or different and are hydrogen, t-butyl, t-butoxy, 2,3,3-trimethyl-2-butyl, or 2,3,3-trimethyl-2-butoxy, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth.

In a preferred embodiment, in the compound of formula III, $R^3$ is t-butyl or 2,3,3-trimethyl-2-butyl, $R^{4'}$ and $R^{4''}$ are hydrogen, and $Y^+$ is hydrogen, ammonium, or alkali metal.

In another preferred embodiment, in the compound of formula III, $R^3$ is t-butyl and $R^{4'}$ and $R^{4''}$ are hydrogen, and $Y^+$ is hydrogen, ammonium, or alkali metal.

In another preferred embodiment, in the compound of formula III, $R^{4'}$ and $R^{4''}$ are the same or different and are t-butyl or 2,3,3-trimethyl-2-butyl, $R^3$ is hydrogen and $Y^+$ hydrogen, ammonium, or alkali metal.

In another preferred embodiment, in the compound of formula III, $R^{4'}$ and $R^{4''}$ are t-butyl, $R^3$ is hydrogen and $Y^+$ is hydrogen, ammonium, and alkali metal.

Disclosed, but not claimed, is also an aqueous composition that comprises a hydrocarbon dispersing agent of the formula II:

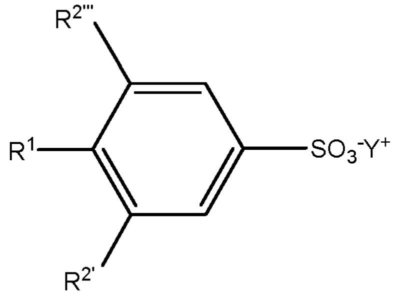

II

wherein $R^{2'}$ and $R^{2''}$ are the same or different and are saturated or unsaturated, non-cyclic or cyclic aliphatic groups having 4 to 16 carbon atoms with the percentage of total $CH_3$ groups in relation to the total of $CH_3$, $CH_2$ and CH groups in the $R^2$ and $R^3$ groups being at least 70%, or may be joined together to form an saturated or unsaturated aliphatic ring that may contain ether or ester linkages, with the proviso that the percentage of total $CH_3$ groups in relation to the total of $CH_3$, $CH_2$ and CH groups in said ring is at least 70%;
wherein $R^1$ is hydrogen, methoxy, ethoxy or phenoxy; and
wherein $Y^+$ is hydrogen, ammonium, quaternary ammonium, nitrogen heterocycle, alkali metal, or alkaline earth.

Preferably, in the aqueous composition of the compound of formula II, $R^{2'}$, and $R^{2''}$ are the same or different and are t-butyl-, t-butoxy, 2,3,3-trimethyl-2-butyl, 2,3,3-trimethyl-2-butoxy, or $-CO(O)C(CH_3)_3$, and wherein $R^1$ is hydrogen, methoxy, or phenoxy, and wherein $Y^+$ is a cation selected from the group consisting of hydrogen, ammonium, or alkali metal.

Preferably, in the aqueous composition of the compound of formula II, $R^{2'}$ and $R^{2''}$ are the same or different and are t-butyl or 2,3,3-trimethyl-2-butyl, $R^1$ is hydrogen, and $Y^+$ is hydrogen, ammonium, or alkali metal.

In the compound of formula II, $R^{2'}$ and $R^{2''}$ are the same or different and are t-butyl- or 2,3,3-trimethyl-2-butyl, and $R^1$ is methoxy.

In the compound of formula II, $R^{2'}$ and $R^{2''}$ are both t-butyl, $R^1$ is hydrogen, or methoxy, and $Y^+$ is hydrogen, ammonium, or alkali metal.

The present disclosure also provides but does not claim a compound of the formula III:

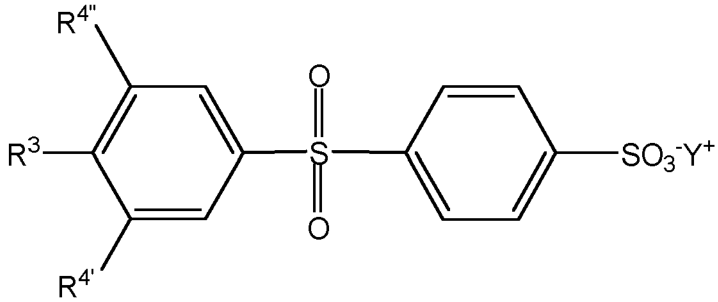

III

wherein $R^3$, $R^{4'}$, and $R^{4''}$ are the same or different and are hydrogen or saturated or unsaturated, non-cyclic or cyclic aliphatic groups having 4 to 16 carbon atoms with the percentage of total $CH_3$ groups in relation to the total of $CH_3$, $CH_2$ and CH groups the $R^3$, $R^{4'}$, and $R^{4''}$ groups being at least 70%, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and
$Y^+$ is hydrogen, ammonium, quaternary ammonium, nitrogen heterocycle, alkali metal, or alkaline earth.

Preferred are compounds wherein $R^3$, $R^{4'}$, and $R^{4''}$ are the same or different and are hydrogen, t-butyl, t-butoxy, 2,3,3-trimethyl-2-butyl, or 2,3,3-trimethyl-2-butoxy, with the provisio that at least one of $R^3$, $R^{4'}$, and $R^{4''}$ is not hydrogen

and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth.

Preferably $R^3$ is t-butyl or 2,3,3-trimethyl-2-butyl, $R^{4'}$ and $R^{4''}$ are hydrogen, and $Y^+$ is hydrogen, ammonium, or alkali metal.

Also preferably $R^3$ is t-butyl, $R^{4'}$ and $R^{4''}$ are hydrogen, and $Y^+$ is hydrogen, ammonium, or alkali metal.

Further preferably $R^{4'}$ and $R^{4''}$ are the same or different and are t-butyl or 2,3,3-trimethyl-2-butyl, $R^3$ is hydrogen and $Y^+$ is hydrogen, ammonium, and alkali metal.

Finally it is also preferred that $R^{4'}$ and $R^{4''}$ are t-butyl, $R^3$ is hydrogen and $Y^+$ is hydrogen, ammonium, and alkali metal.

## BRIEF DESCRIPTION OF THE DRAWINGS

FIG. 1 is graphical representation of the effect of increasing dispersing agent concentration on monomer feed rate for selected comparative and inventive dispersing agents in a process for polymerizing fluoroelastomer as illustrated in Polymerization Example 14.

## DETAILED DESCRIPTION

### Fluoromonomer/Fluoropolymer

The process of the invention polymerizes fluoromonomer in an aqueous medium to form an aqueous dispersion of fluoropolymer particles. By "fluoromonomer" is meant a monomer which contains fluorine, preferably an olefinic monomer with at least one fluorine or a fluoroalkyl group attached to a doubly bonded carbon. Other monomers can be employed in the polymerization that are not fluorinated.

The fluoromonomer and the fluoropolymer obtained therefrom each preferably contain at least 35 wt% fluorine, more preferably at least 50 wt% fluorine. Preferred fluoromonomers useful to produce fluoropolymers in accordance with the invention may be selected from the group consisting of tetrafluoroethylene (TFE), hexafluoropropylene (HFP), chlorotrifluoroethylene (CTFE), trifluoroethylene, hexafluoroisobutylene, perfluoroalkyl ethylene, fluorovinyl ethers, vinyl fluoride (VF), vinylidene fluoride (VF2), perfluoro-2,2-dimethyl-1,3-dioxole (PDD), perfluoro-2-methylene-4-methyl-1,3-dioxolane (PMD), perfluoro(allyl vinyl ether) and perfluoro(butenyl vinyl ether) and mixtures thereof. A preferred perfluoroalkyl ethylene monomer is perfluorobutyl ethylene (PFBE). Preferred fluorovinyl ethers include perfluoro(alkyl vinyl ether) monomers (PAVE) such as perfluoro(propyl vinyl ether) (PPVE), perfluoro(ethyl vinyl ether) (PEVE), and perfluoro(methyl vinyl ether) (PMVE). Non-fluorinated olefinic comonomers such as ethylene (E) and propylene (P) can be copolymerized with the fluoromonomers.

Fluorovinyl ethers also include those useful for introducing functionality into fluoropolymers. These include $CF_2=CF-(O-CF_2CFR_f)_a-O-CF_2CFR'_fSO_2F$, wherein $R_f$ and $R'_f$ are independently selected from F, Cl or a perfluorinated alkyl group having 1 to 10 carbon atoms, a = 0, 1 or 2. Monomers of this type are disclosed in U.S. Patent No. 3,282,875 ($CF_2=CF-O-CF_2CF(CF_3)-O-CF_2CF_2SO_2F$, perfluoro(3,6-dioxa-4-methyl-7-octenesulfonyl fluoride)), and in U.S. Patent Nos. 4,358,545 and 4,940,525 ($CF_2=CF-O-CF_2CF_2SO_2F$). Another example is $CF_2=CF-O-CF_2-CF(CF_3)-O-CF_2CF_2CO_2CH_3$, methyl ester of perfluoro(4,7-dioxa-5-methyl-8-nonenecarboxylic acid), disclosed in U.S. Patent No. 4,552,631. Similar fluorovinyl ethers with functionality of nitrile, cyanate, carbamate, and phosphonic acid are disclosed in U.S. Patent Nos. 5,637,748, 6,300,445, and 6,177,196.

In one preferred process in accordance with the invention, the process produces a dispersion of perfluoropolymer particles. By "perfluoropolymer" is meant that the monovalent substituents on the carbon atoms forming the chain or backbone of the polymer are all fluorine atoms, with the exception of C-H moiety resulting from minor amounts of comonomer or C-H moiety in end groups or pendant group structure. Preferably, the comonomer, end group, or pendant group structure will impart no more than 2 wt% C-H moiety, more preferably no greater than 1 wt% C-H moiety, with respect to the total weight of the perfluoropolymer. Preferably, the hydrogen content, if any, of the perfluoropolymer is no greater than 0.2 wt%, based on the total weight of the perfluoropolymer. A preferred group of perfluoropolymers are selected from polytetrafluoroethylene, modified polytetrafluoroethyene, and melt-fabricable copolymer comprising 40-99 mol% tetrafluoroethylene units and 1-60 mol% of at least one other perfluoromonomer.

The process is especially useful for making high molecular weight polytetrafluoroethylene (PTFE) (including modified PTFE) in the form of fine powders and dispersions. Polytetrafluoroethylene (PTFE) refers to (a) the polymerized tetrafluoroethylene by itself without any significant comonomer present, i.e. homopolymer and (b) modified PTFE, which is a copolymer of TFE having such small concentrations of comonomer that the melting point of the resultant polymer is not substantially reduced below that of PTFE. The modified PTFE contains a small amount of comonomer modifier which reduces crystallinity to improve film forming capability during baking (fusing). Examples of such monomers include perfluoroolefin, notably hexafluoropropylene (HFP) or perfluoro(alkyl vinyl ether) (PAVE), where the alkyl group contains 1

to 5 carbon atoms, with perfluoro(methyl vinyl ether) (PMVE), perfluoro(ethyl vinyl ether) (PEVE) and perfluoro(propyl vinyl ether) (PPVE) being preferred, chlorotrifluoroethylene (CTFE), perfluorobutyl ethylene (PFBE), or other monomer that introduces bulky side groups into the polymer molecule. The concentration of such comonomer is preferably less than 1 wt%, more preferably less than 0.5 wt%, based on the total weight of the TFE and comonomer present in the PTFE. A minimum amount of at least about 0.05 wt% is preferably used to have significant effect. High molecular weight PTFE (and modified PTFE) when isolated from dispersion as a powder, referred to as PTFE fine powder, typically have a melt creep viscosity of at least 1 x $10^6$ Pa•s and preferably at least 1 x $10^8$ Pa•s and, with such high melt viscosity, the polymer does not significantly flow in the molten state and therefore is not a melt-processible polymer. The measurement of melt creep viscosity of PTFE fine powder is disclosed in column 4 of U.S. Patent 7,763,680. The high melt viscosity of PTFE arises from is extremely high molecular weight (Mn), e.g. at least $10^6$. PTFE can also be characterized by its high melting temperature, of at least 330°C, upon first heating. The non-melt flowability of the PTFE, arising from its extremely high melt viscosity, results in a no melt flow condition when melt flow rate (MFR) is measured in accordance with ASTM D 1238 at 372°C and using a 5 kg weight, i.e., MFR is 0. The high molecular weight of PTFE is characterized by measuring its standard specific gravity (SSG). The SSG measurement procedure (ASTM D 4894, also described in U.S. Patent No. 4,036,802) includes sintering of the SSG sample free standing (without containment) above its melting temperature without change in dimension of the SSG sample. The SSG sample does not flow during the sintering.

The process of the present invention is also useful for producing low molecular weight PTFE by directly polymerizing to a low molecular weight. Low molecular weight PTFE is commonly known as PTFE micropowder to distinguish from high molecular weight PTFE fine powder described above which has significantly higher molecular weight. The molecular weight of PTFE micropowder is low relative to PTFE fine powder, i.e. the molecular weight (Mn) is generally in the range of $10^4$ to $10^5$. The result of this lower molecular weight of PTFE micropowder is fluidity in the molten state, in contrast to PTFE fine powder which is not melt flowable. PTFE micropowder has melt flowability, which can be characterized by a melt flow rate (MFR) of at least 0.01 g/10 min, preferably at least 0.1 g/10 min and more preferably at least 5 g/10 min, and still more preferably at least 10 g/10 min., as measured in accordance with ASTM D 1238, at 372°C using a 5 kg weight on the molten polymer.

The invention is also useful for making melt-processible fluoropolymers that are also melt-fabricable. Melt-processible means that the fluoropolymer can be processed in the molten state, i.e., fabricated from the melt using conventional processing equipment such as extruders and injection molding machines, into shaped articles such as films, fibers, and tubes. Melt-fabricable means that the resultant fabricated articles exhibit sufficient strength and toughness to be useful for their intended purpose. This sufficient strength may be characterized by the fluoropolymer by itself exhibiting an MIT Flex Life of at least 1000 cycles, preferably at least 2000 cycles, measured as disclosed in U.S. Patent No. ,703,185. The strength of the fluoropolymer is indicated by it not being brittle.

Examples of such melt-processible fluoropolymers include homopolymers such as polychlorotrifluoroethylene and polyvinylidene fluoride (PVDF) or copolymers of tetrafluoroethylene (TFE) and at least one fluorinated copolymerizable monomer (comonomer) present in the polymer usually in sufficient amount to reduce the melting point of the copolymer substantially below that of PTFE, e.g., to a melting temperature no greater than 315°C.

A melt-processible TFE copolymer typically incorporates an amount of comonomer into the copolymer in order to provide a copolymer which has a melt flow rate (MFR) of 0.1 to 200 g/10 min as measured according to ASTM D-1238 using a 5 kg weight on the molten polymer and the melt temperature which is standard for the specific copolymer. MFR will preferably range from 1 to 100 g/10 min, most preferably about 1 to about 50 g/10 min. Additional melt-processible fluoropolymers are the copolymers of ethylene (E) or propylene (P) with TFE or CTFE, notably ETFE and ECTFE.

A preferred melt-processible copolymer for the practice of the present invention comprises at least 40-99 mol% tetrafluoroethylene units and 1-60 mol% of at least one other monomer. Additional melt-processible copolymers are those containing 60-99 mol% TFE units and 1-40 mol% of at least one other monomer. Preferred comonomers with TFE to form perfluoropolymers are perfluoromonomers, preferably perfluoroolefin having 3 to 8 carbon atoms, such as hexafluoropropylene (HFP), and/or perfluoro(alkyl vinyl ether) (PAVE) in which the linear or branched alkyl group contains 1 to 5 carbon atoms. Preferred PAVE monomers are those in which the alkyl group contains 1, 2, 3 or 4 carbon atoms, and the copolymer can be made using several PAVE monomers. Preferred TFE copolymers include FEP (TFE/HFP copolymer), PFA (TFE/PAVE copolymer), TFE/HFP/PAVE wherein PAVE is PMVE, PEVE and/or PPVE, MFA (TFE/PMVE/PAVE wherein the alkyl group of PAVE has at least two carbon atoms) and THV (TFE/HFPNF2).

The melt-processible fluoropolymers described above can be characterized by MFR as recited above for the melt-processible TFE copolymers such as PFA and FEP, i.e. by the procedure of ASTM 1238 using standard conditions for the particular polymer, including a 5 kg weight on the molten polymer in the plastometer.

Further useful polymers are film forming polymers of polyvinylidene fluoride (PVDF) and copolymers of vinylidene fluoride as well as polyvinyl fluoride (PVF) and copolymers of vinyl fluoride.

The invention is also useful for making fluorocarbon elastomers (fluoroelastomers). Fluoroelastomers typically have a glass transition temperature below 25°C and exhibit little or no crystallinity at room temperature and little or no melting temperature. Fluoroelastomers that can be made by the process of this invention typically are copolymers

containing 25 to 75 wt%, based on total weight of the fluoroelastomer, of copolymerized units of a first fluoromonomer which may be vinylidene fluoride (VF2) or tetrafluoroethylene (TFE). The remaining units in the fluoroelastomers are comprised of one or more additional copolymerized monomers, different from the first monomer, selected from the group consisting of fluoromonomers, hydrocarbon olefins and mixtures thereof. Fluoroelastomers may also, optionally, comprise units of one or more cure site monomers. When present, copolymerized cure site monomers are typically at a level of 0.05 to 7 wt%, based on total weight of fluorocarbon elastomer. Examples of suitable cure site monomers include: i) bromine -, iodine -, or chlorine - containing fluorinated olefins or fluorinated vinyl ethers; ii) nitrile group-containing fluorinated olefins or fluorinated vinyl ethers; iii) perfluoro(2-phenoxypropyl vinyl ether); and iv) non-conjugated dienes. The process of the invention can also be used for fluoroelastomers that are polymerized in the presence of iodine-containing or bromine-containing chain transfer agents such as the diiodoperfluoroalkane compounds $I(CF_2)_nI$, wherein n is 3 to 7, that provide cure sites bonded to terminal carbons of the fluoroelastomer.

Preferred TFE based fluoroelastomer copolymers include TFE/PMVE, TFE/PMVE/E, TFE/P and TFE/PNF2. Preferred $VF_2$ based fluorocarbon elastomer copolymers include VF2/HFP, VF2/HFP/TFE, and VF2/PMVE/TFE. Any of these elastomer copolymers may further comprise units of cure site monomer and cure sites formed using iodine-containing or bromine-containing chain transfer agents.

**Hydrocarbon Dispersing Agents**

A polymerization process in accordance with the invention employs a hydrocarbon dispersing agent comprising a compound of formula I:

$$R\text{-}(XZ)_n \qquad \text{I}$$

wherein R is a hydrophobic hydrocarbon moiety that comprises one or more saturated or unsaturated, non-cyclic or cyclic aliphatic groups, the percentage of total $CH_3$ groups in relation to the total of $CH_3$, $CH_2$ and CH groups in the one or more aliphatic groups being at least 70%, the hydrophobic moiety being free of siloxane units;

wherein each X may be the same or different and represents an ionic hydrophilic moiety;

wherein each Z may be a same or different and represents one or more counter ions for the ionic hydrophilic moiety; and

wherein n is 1 to 3.

The dispersing agents employed in the polymerization process according to the present invention exhibit low reactivity with polymerization initiator and/or the propagating fluoropolymer radical in the emulsion polymerization of fluoromonomer.

In the hydrocarbon dispersing agents used in the process of the present invention, the percentage of total $CH_3$ groups in relation to the total of $CH_3$, $CH_2$ and CH groups in the one or more aliphatic groups in the hydrophobic hydrocarbon moiety is at least 70%. Preferably, the percentage of total $CH_3$ groups in relation to the total of $CH_3$, $CH_2$ and CH groups in the one or more aliphatic groups in the hydrophobic hydrocarbon moiety is at least 75%, still more preferably at least about 80%, still more preferably at least 85%, still more preferably at least about 90%, still more preferably at least 95%, and most preferably 100%.

Although there is no intent to be bound by any theory of operation, the hydrocarbon dispersing agents with above described percentage of total $CH_3$ groups in relation to the total of $CH_3$, $CH_2$ and CH groups in the one or more aliphatic groups in the hydrophobic hydrocarbon moiety are believed to confer resistance to the hydrophobic hydrocarbon moiety to radical attack by the initiator used in the polymerization and/or the propagating fluoropolymer radical. Surfactants with long chain hydrocarbon or polyalkylene oxide moieties are susceptible to attack by such radicals. Preferably in the process of the invention, the hydrophobic hydrocarbon moiety in the hydrocarbon dispersing agent comprises no more than 3 consecutive $CH_2$ groups, more preferably, no more than 2 consecutive $CH_2$ groups, and most preferably, no consecutive $CH_2$ groups. Preferably in the process of the invention, the hydrophobic hydrocarbon moiety of the hydrocarbon dispersing agent comprises at least 3 $CH_3$ groups, more preferably, at least 4 $CH_3$ groups. One preferred range for the number of $CH_3$ groups in the hydrophobic hydrocarbon moiety is 3 to 12. Another preferred range for the number of $CH_3$ groups in the hydrophobic hydrocarbon moiety is 3 to 10. Another preferred range for the number of $CH_3$ groups in the hydrophobic hydrocarbon moiety is 3 to 7. Another preferred range for the number of $CH_3$ groups in the hydrophobic hydrocarbon moiety is 4 to 7.

In another preferred embodiment of the process of the present invention, the hydrophobic hydrocarbon moiety in the hydrocarbon dispersing agent is free of carbon-hydrogen bonds with bond dissociation energies to yield a hydrocarbon radical of less than 100 kcal/mol.

The hydrocarbon dispersing agents for use in accordance with the present invention are free of siloxane units. Although there is no intent to be bound by any theory of operation, it is believed that a high percentage of $CH_3$ groups in

relation to the total of $CH_3$, $CH_2$ and CH groups that is present in some siloxane surfactants does not confer the same level of resistance to the hydrophobic hydrocarbon moiety to radical attack by the initiator used in the polymerization and/or the propagating fluoropolymer radical that is achieved with hydrocarbon dispersing agents in accordance with the invention with the above described percentage of total $CH_3$ groups in relation to the total of $CH_3$, $CH_2$ and CH groups. Additionally, siloxane surfactants are prone to hydrolysis and redistribution side-reactions under typical conditions of emulsion polymerization.

The low reactivity of the hydrocarbon dispersing agents used in a process of the invention can be demonstrated by their performance in a test in which the hydrocarbon dispersing agents are exposed to an oxidation agent. In a preferred embodiment of the present invention, hydrocarbon dispersing agent has a t-butyl hydroperoxide oxidative loss of less than 10 wt% in the test method described and illustrated in Reactivity Example 1. More preferably, the hydrocarbon dispersing agent has a t-butyl hydroperoxide oxidative loss of less than 5 wt%.

By "hydrocarbon" regarding the hydrophobic hydrocarbon moiety is meant that at least 85% of the monovalent substituents on the carbon atoms are hydrogen and substitutions by halogen, such as fluorine or chlorine, are possible provided that the low reactivity of the dispersing agent during polymerization is not adversely affected. In a preferred hydrophobic hydrocarbon moiety, at least 90%, more preferably at least 95% of the monovalent substituents are hydrogen. In a most preferred embodiment, 100% of the monovalent substituents on the carbon atoms are hydrogen. Thus, in a preferred embodiment of the invention, the hydrophobic hydrocarbon moiety is free of halogens. "Hydrocarbon" regarding hydrocarbon dispersing agent is intended to have the same meaning as described above for the hydrophobic hydrocarbon moiety.

In another preferred embodiment, the one or more aliphatic groups in the hydrophobic hydrocarbon moiety are non-cyclic or cyclic alkyl groups, most preferably non-cyclic, with percentage of total $CH_3$ groups in relation to the total of $CH_3$, $CH_2$ and CH groups in the one or more aliphatic groups in the hydrophobic hydrocarbon moiety being at least 70%. Preferred non-cyclic alkyl groups are t-butyl and 2,3,3-trimethyl-2-butyl groups that contain no $CH_2$ or CH groups.

The hydrophobic hydrocarbon moiety may contain heteroatom-containing bivalent or trivalent linking groups that do not significantly increase the reactivity of the dispersing agent during polymerization. Examples of suitable heteroatom-containing bivalent or trivalent linking groups are ether, ester, sulfide, sulfone, sulfoxide, sulfonamide, carbonate, carbonyl, phosphonate, and phosphate. The heteroatom-containing bivalent or trivalent linking groups may be present within the one or more saturated or unsaturated, non-cyclic or cyclic aliphatic groups or in other locations in the hydrophobic hydrocarbon moiety. For example, in one embodiment of the invention, heteroatom-containing bivalent or trivalent linking groups may be present within the one or more saturated or unsaturated, non-cyclic or cyclic aliphatic groups. In another embodiment of the invention in which the hydrophobic hydrocarbon group contains an aromatic group, the bivalent or trivalent linking groups may link the one or more saturated or unsaturated, non-cyclic or cyclic aliphatic groups to the aromatic group, e.g., an ether linkage such that the aliphatic group is an alkoxy group. In another embodiment of the invention with multiple aromatic groups, the heteroatom-containing bivalent or trivalent linking groups or may link aromatic groups together. Hydrocarbon dispersing agents for use in the process of the present invention may have heteroatom-containing bivalent or trivalent linking groups in multiple locations in the hydrophobic hydrocarbon moiety.

The ionic hydrophilic moiety X may be anionic or cationic. The ionic hydrophilic moiety X is preferably selected from the group consisting of acid groups and salts thereof, quaternary ammonium groups, amine-oxide groups, and tetrazole groups. The one or more counter ion Z for hydrophobic moiety has a charge opposite the ionic hydrophilic moiety will be present in a molar amount to balance the charge of the ionic hydrophilic moiety. The counter ion preferably provides adequate solubility for the hydrocarbon dispersing agent in the aqueous medium for the conditions used in the process. Preferably, the ionic hydrophilic moiety X is anionic. In one preferred embodiment, the hydrophilic moiety and counter ion XY is a group of the formula $-A^{-}-Y^{+}$ wherein $A^{-}$ is carboxylate, sulfonate, sulfate, phosphonate, or phosphate, and wherein $Y^{+}$ is hydrogen, ammonium, quaternary ammonium, nitrogen heterocycle, alkali metal, or alkaline earth. Most preferably, wherein $A^{-}$ is sulfonate. Up to 3 ionic hydrophilic moieties may be present although only one such moiety can confer sufficient hydrophilic character to the hydrocarbon dispersing agent to function in the process effectively.

The ionic hydrophilic moiety can be bonded to the hydrophobic hydrocarbon moiety at any location that makes the compound effective as a dispersing agent for use in the emulsion polymerization of fluoropolymer.

In one embodiment of the process of the invention, the hydrophobic hydrocarbon moiety is "entirely aliphatic". By "entirely aliphatic" is meant that the hydrophobic hydrocarbon moiety does not contain any aromatic groups and comprises only the one or more saturated or unsaturated, non-cyclic or cyclic aliphatic groups with the percentage total $CH_3$ groups in relation to the total of $CH_3$, $CH_2$ and CH groups in the one or more aliphatic groups being at least about 70%. The hydrophobic hydrocarbon moiety may comprise only one aliphatic group or may comprise multiple aliphatic groups linked by the linking groups such as those described above. In the embodiment of the invention in which the hydrophobic hydrocarbon moiety of hydrocarbon dispersing agent is entirely aliphatic, the hydrocarbon dispersing agent has a molecular weight without a counter ion Z of at least 170 g/mol, preferably at least 180 g/mol, more preferably at least 190 g/mol, and still more preferably at least 200 g/mol. A preferred upper limit of molecular weight for any of the stated lower limits for this embodiment of the invention without a counter ion Z is no more than 750 g/mol.

In another embodiment of the process of the invention, the hydrophobic hydrocarbon moiety comprises one or more aromatic groups. The one or more saturated or unsaturated, non-cyclic or cyclic aliphatic groups may be directly bonded to the aromatic groups or by the linking groups such as those described above. If there are multiple aromatic groups, they may be directly linked together, linked by the linking groups such as those described above, or linked together by aliphatic groups. In the embodiment of the invention in which the hydrophobic hydrocarbon moiety of hydrocarbon dispersing agent contains at least one aromatic group, the hydrocarbon dispersing agent has a molecular weight without a counter ion Z of at least 215 g/mol, preferably 220 g/mol, more preferably at least 225 g/mol, and still more preferably at least 230 g/mol. A preferred upper limit of molecular weight for any of the stated lower limits for this embodiment of the invention without a counter ion Z is no more than 800 g/mol.

Aromatic groups in the embodiment of the invention with one or more aromatic groups are typically phenyl groups but may also be polycyclic or heterocyclic aromatic groups.

In the embodiment of the invention that includes one or more aromatic groups, the one or more aromatic groups have a Hammett sigma plus value of greater than -1.0, more preferably a Hammett sigma plus value of greater than -0.8, and most preferably a Hammett sigma plus value of greater than 0. Hammett sigma plus value for an aromatic ring is calculated by summing the Hammett sigma plus values for each of the substituents on a ring. Hydrogens have a value of zero. Hammett sigma plus values are tabulated in Hansch et al. Chemical Reviews, 1991, volume 91, pages 165-195. Hammett sigma plus values can also be determined by the methods reviewed by Hansch, et al.

In a preferred embodiment of the process of the present invention, hydrocarbon dispersing agent comprises a substituted moiety of the formula:

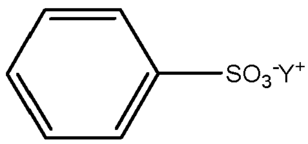

wherein $Y^+$ is hydrogen, ammonium, quaternary ammonium, nitrogen heterocycle, alkali metal, or alkaline earth.

In the embodiment that includes one or more aromatic groups, the hydrophobic hydrocarbon moiety is preferably free of methyl groups directed bonded to an aromatic group. It is also preferable in such embodiment for the hydrophobic hydrocarbon moiety to be free of benzylic hydrogen atoms. It is also preferable such embodiment for the hydrophobic hydrocarbon moiety to be free of phenolic hydroxy groups. Although there is no intent to be bound by any theory of operation, it is believed that dispersing agents with such groups or structures are susceptible to radical attack by the initiator used in the polymerization and/or the propagating fluoropolymer radical.

The hydrocarbon hydrophobic moiety may include any of a variety of substituents provided such groups do not significantly adversely affect the low reactivity of the hydrocarbon dispersing agent or impair its effectiveness as a dispersing agent.

In a preferred embodiment of the process of the present invention, the hydrophobic hydrocarbon moiety of the hydrocarbon dispersing agent comprises 8 to 50 carbon atoms, preferably 10 to 40 carbon atoms, more preferably 12 to 30 carbon atoms, most preferably, 12 to 25 carbon atoms.

Table A in the Examples which follow lists various embodiments of hydrocarbon dispersing agents suitable for use in a process in accordance with the invention. There is no intent to limit the invention to the disclosed embodiments and many other dispersing agents within the scope of he claims as described above can be used in a process in accordance with the invention. Table A also includes various comparative dispersing agents that are useful to compare with the low reactivity dispersing agents in polymerization processes for production of fluoropolymers.

In a preferred embodiment of the process of the present invention, the hydrocarbon dispersing agent is a compound of formula II:

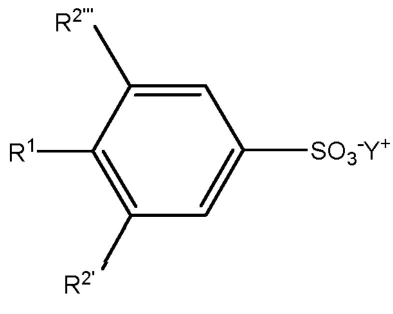

II

wherein $R^{2'}$ and $R^{2''}$ are the same or different and are saturated or unsaturated, non-cyclic or cyclic aliphatic groups having 4 to 16 carbon atoms with the percentage of total $CH_3$ groups in relation to the total of $CH_3$, $CH_2$ and CH groups in the $R^{2'}$ and $R^{2''}$ groups being at least 70%, or $R^{2'}$ and $R^{2''}$ may be joined together to form an saturated or unsaturated aliphatic ring that may contain ether or ester linkages, with the proviso that the percentage of total $CH_3$ groups in relation to the total of $CH_3$, $CH_2$ and CH groups in the ring is at least 70%;
wherein $R^1$ is hydrogen, methoxy, ethoxy or phenoxy; and
wherein $Y^+$ is hydrogen, ammonium, quaternary ammonium, nitrogen heterocycle, alkali metal, or alkaline earth.

Preferably, in the compound of formula II, $R^{2'}$, and $R^{2''}$ are the same or different and are t-butyl-, t-butoxy, 2,3,3-trimethyl-2-butyl, or 2,3,3-trimethyl-2-butoxy, or $-CO(O)C(CH_3)_3$, and wherein $R^1$ is hydrogen or methoxy, and wherein $Y^+$ is hydrogen, ammonium, or alkali metal.

In another preferred embodiment, in the compound of formula II, $R^{2}$ and $R^{2''}$ are the same or different and are t-butyl or 2,3,3-trimethyl-2-butyl, $R^1$ is hydrogen, and $Y^+$ is hydrogen, ammonium, or alkali metal.

In another preferred embodiment, in the compound of formula II, $R^{2'}$ and $R^{2''}$ are the same or different and are t-butyl or 2,3,3-trimethyl-2-butyl, $R^1$ is methoxy and $Y^+$ is hydrogen, ammonium, or alkali metal.

In another preferred embodiment, in the compound of formula II, $R^{2'}$ and $R^{2''}$ are both t-butyl, $R^1$ is hydrogen or methoxy, and $Y^+$ is hydrogen, ammonium, or alkali metal.

Preferred examples of the compound of formula II are shown below and their use in a process in accordance with a process of the invention is illustrated in the Examples:

Dispersing Agent I-8

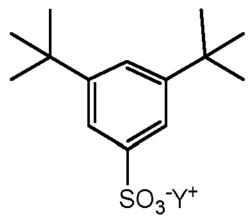

Dispersing Agent I-1

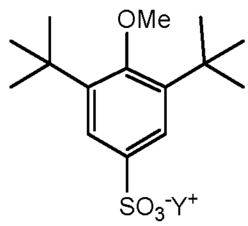

$Y^+$ in these preferred dispersing agents can be hydrogen, ammonium, or alkali metal.

In a preferred embodiment of the process of the present invention, the hydrocarbon dispersing agent is a compound of the formula III:

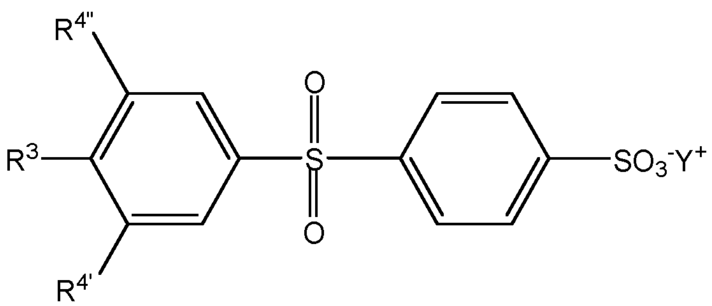

wherein $R^3$, $R^{4'}$, and $R^{4''}$ are the same or different and are hydrogen or saturated or unsaturated, non-cyclic or cyclic aliphatic groups having 4 to 16 carbon atoms with the percentage of total $CH_3$ groups in relation to the total of $CH_3$, $CH_2$ and CH groups the $R^3$, $R^{4'}$, and $R^{4''}$ groups being at least 70%, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and

$Y^+$ is hydrogen, ammonium, quaternary ammonium, nitrogen heterocycle, alkali metal, or alkaline earth.

Preferably, in the compound of formula III, $R^3$, $R^{4'}$, and $R^{4''}$ are the same or different and are hydrogen, t-butyl, t-butoxy, 2,3,3-trimethyl-2-butyl, or 2,3,3-trimethyl-2-butoxy, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth.

In a preferred embodiment, in the compound of formula III, $R^3$ is t-butyl or 2,3,3-trimethyl-2-butyl, $R^{4'}$ and $R^{4''}$ are hydrogen, and $Y^+$ is hydrogen, ammonium, or alkali metal.

In another preferred embodiment, in the compound of formula III, $R^3$ is t-butyl and $R^{4'}$ and $R^{4''}$ are hydrogen, and $Y^+$ is hydrogen, ammonium, or alkali metal.

In another preferred embodiment, in the compound of formula III, $R^{4'}$ and $R^{4''}$ are the same or different and are t-butyl or 2,3,3-trimethyl-2-butyl, $R^3$ is hydrogen and $Y^+$ hydrogen, ammonium, or alkali metal.

In another preferred embodiment, in the compound of formula III, $R^{4'}$ and $R^{4''}$ are t-butyl, $R^3$ is hydrogen and $Y^+$ is hydrogen, ammonium, and alkali metal.

A preferred example of compound of formula III is shown below and its use in a process in accordance with a process of the invention is illustrated in the Examples:

Dispersing Agent I-3

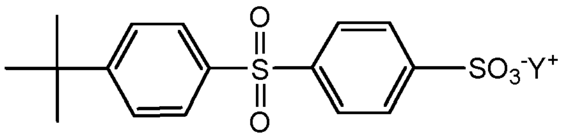

$Y^+$ in these dispersing agents can be hydrogen, ammonium, or alkali metal.

In accordance with another aspect disclosed here but not claimed, an aqueous composition comprises a hydrocarbon dispersing agent of the formula II:

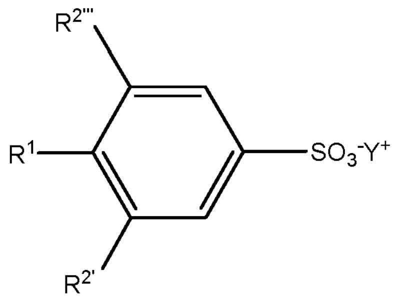

II

wherein $R^{2'}$ and $R^{2''}$ are the same or different and are saturated or unsaturated, non-cyclic or cyclic aliphatic groups having 4 to 16 carbon atoms with the percentage of total $CH_3$ groups in relation to the total of $CH_3$, $CH_2$ and CH groups in the $R^2$ and $R^3$ groups being at least 70%, or may be joined together to form an saturated or unsaturated aliphatic ring that may contain ether or ester linkages, with the proviso that the percentage of total $CH_3$ groups in relation to the total of $CH_3$, $CH_2$ and CH groups in the ring is at least 70%;

wherein $R^1$ is hydrogen, methoxy, ethoxy or phenoxy; and

wherein $Y^+$ is hydrogen, ammonium, quaternary ammonium, nitrogen heterocycle, alkali metal, or alkaline earth.

Preferably, in the aqueous composition of the compound of formula II, $R^{2'}$, and $R^{2''}$ are the same or different and are t-butyl-, t-butoxy, 2,3,3-trimethyl-2-butyl, 2,3,3-trimethyl-2-butoxy, or $-CO(O)C(CH_3)_3$, and wherein $R^1$ is hydrogen, methoxy, or phenoxy, and wherein $Y^+$ is a cation selected from the group consisting of hydrogen, ammonium, or alkali metal.

Preferably, in the aqueous composition of the compound of formula II, $R^{2'}$ and $R^{2''}$ are the same or different and are t-butyl or 2,3,3-trimethyl-2-butyl, $R^1$ is hydrogen, and $Y^+$ is hydrogen, ammonium, or alkali metal.

In the compound of formula II, it is preferred that $R^{2'}$ and $R^{2''}$ are the same or different and are t-butyl- or 2,3,3-trimethyl-2-butyl, and $R^1$ is methoxy.

In the compound of formula II, it is further preferred that $R^{2'}$ and $R^{2''}$ are both t-butyl, $R^1$ is hydrogen, or methoxy, and $Y^+$ is hydrogen, ammonium, or alkali metal.

Preferred examples of the compound of formula II for use in the aqueous composition are described above in the description of the process employing formula II dispersing agents.

The aqueous composition of the compound of formula II can be used in a variety of applications but is especially useful as an aqueous medium for the polymerization of fluoromonomer to form an aqueous dispersion of fluoropolymer particles as in a process in accordance with the present invention.

Disclosed but not claimed is also a compound of the formula III:

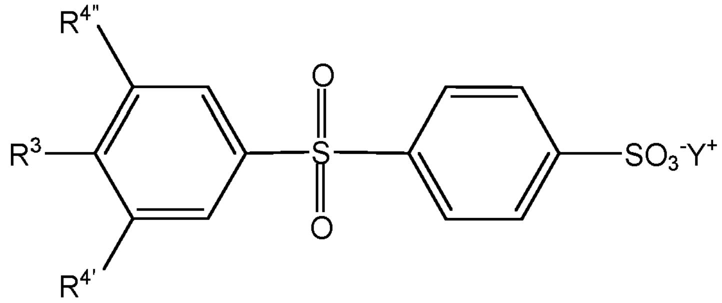

III

wherein $R^3$, $R^{4'}$, and $R^{4''}$ are the same or different and are hydrogen or saturated or unsaturated, non-cyclic or cyclic aliphatic groups having 4 to 16 carbon atoms with the percentage of total $CH_3$ groups in relation to the total of $CH_3$, $CH_2$ and CH groups the $R^3$, $R^{4'}$, and $R^{4''}$ groups being at least 70%, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and

$Y^+$ is hydrogen, ammonium, quaternary ammonium, nitrogen heterocycle, alkali metal, or alkaline earth.

Preferred are compounds wherein $R^3$, $R^{4'}$, and $R^{4''}$ are the same or different and are hydrogen, t-butyl, t-butoxy, 2,3,3-trimethyl-2-butyl, or 2,3,3-trimethyl-2-butoxy, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth.

Preferably $R^3$ is t-butyl or 2,3,3-trimethyl-2-butyl, $R^{4'}$ and $R^{4''}$ are hydrogen, and $Y^+$ is hydrogen, ammonium, or alkali metal.

Further preferably $R^3$ is t-butyl, $R^{4'}$ and $R^{4''}$ are hydrogen, and $Y^+$ is hydrogen, ammonium, or alkali metal.

Also preferably $R^{4'}$ and $R^{4''}$ are the same or different and are t-butyl or 2,3,3-trimethyl-2-butyl, $R^3$ is hydrogen and $Y^+$ is hydrogen, ammonium, and alkali metal.

Finally, it is also preferred that $R^{4'}$ and $R^{4''}$ are t-butyl, $R^3$ is hydrogen and $Y^+$ is hydrogen, ammonium, and alkali metal.

A preferred example of the compound of formula III is described above in the description of the process employing formula III dispersing agents.

**Production of Hydrocarbon Dispersing Agents**

Some embodiments of the hydrocarbon dispersing agents for use in the practice of the present invention are known compounds. For example, of the compounds illustrated in Table A, the following are known compounds: I-1, 3, 5-bis(1,1-dimethylethyl)-4-methoxybenzenesulfonic acid, I-5, 2-(4-(tert-butyl)phenoxy)-2-methylpropanoic acid; I-8, 3,5-di-tert-butylbenzenesulfonic acid; I-9, 4-(1,1,3,3-tetramethylbutyl)benzenesulfonic acid; I-10, N-[[4-(1,1-dimethylethyl)phenyl]sulfonyl]-2-methylalanine; and I-14, 2, 2, 3, 3, 4, 4- hexamethylpentanedioic acid.

Also, some of the comparative dispersing agents in Table A are known compounds: C-4, sodium 4-tert-butylbenzenesulfonate; C-8, sodium 4-(phenylsulfonyl)benzenesulfonate; C-9, sodium 3-(diphenylphosphoryl)benzenesulfonate; C-10, sodium 2-hydroxy-2,4,4-trimethylpentane-1-sulfonate; C-11, 3,5-Diisopropylbenzenesulfonic acid; and C-12, 3,5-di-tert-butyl-4-hydroxybenzenesulfonic acid.

Many embodiments of hydrocarbon dispersing agents for use in accordance with the present invention are novel compositions of matter or are not available commercially. These novel or non-commercial dispersing agents can be produced by application of synthetic transformations carried out on appropriately selected precursors that are available commercially or synthesizable. Synthetic transformations relevant to the production of various of the novel or non-commercial hydrocarbon dispersing agents are described below. The synthesis of the specific embodiments of novel hydrocarbon dispersing agents and certain comparative dispersing agents listed in Table A are illustrated in the Synthesis Examples which follow.

**Production Method: Aromatic Sulfonation**

In one embodiment the hydrocarbon dispersing agent has an aromatic sulfonic acid group that can be produced by a method including the step of allowing an appropriate precursor aromatic compound to react with chlorosulfonic acid. This method can be carried out in a solvent in which the precursor and chlorosulfonic acid has solubility. Examples of such solvent includes chloroalkanes, such as dichloromethane. This method can be carried out at low temperature, for example - 10°C, during combination of the precursor and chlorosulfonic acid. The resultant reaction mixture can then be stirred at ambient temperature until the reaction is complete, for example 2-18 hours. This method can be carried out at ambient pressure and the product isolated and purified by recrystallization, precipitation, or other techniques known in the art. (Sulfonic acids have too low vapor pressure for distillation)
Examples of hydrocarbon dispersing agents produced using this method include Synthesis Examples for C-4, C-8, C-11, C-12, I-1, I-3, I-7, I-9, I-8, I-11, I-12, and I-13.

**Production Method: Electrophilic Aromatic Alkylation**

In one embodiment the hydrocarbon dispersing agent has a hydrophobic hydrocarbon moiety that can be produced by a method including the step of allowing an appropriate aromatic compound to react with an appropriate halogenated alkane in the presence of a Lewis Acid catalyst. This method is typically carried out in a solvent in which the aromatic compound and halogenated alkane has solubility. Example such solvent includes aromatic hydrocarbons, such as chlorobenzene. This method can be carried out at low temperature, for example -20°C, during the contacting of the aromatic compound and halogenated alkane in the presence of the Lewis Acid. Following the addition of all reaction components the resultant reaction mixture is then typically stirred at low temperature until the reaction is complete, for example 10 minutes. This method is normally carried out at ambient pressure and the product isolated by aqueous washing followed by purification by vacuum distillation.
An example of a novel hydrocarbon dispersing agent produced using this method is the Synthesis Example for I-4.

**Production Method: Transformation of**

**Ketone to Alcohol, and then Alcohol to Halogen**

In one embodiment the hydrocarbon dispersing agent has a hydrophobic hydrocarbon-containing moiety, wherein the hydrophobic hydrocarbon-containing moiety can be produced by a two-step method including the steps of (i) allowing an appropriate ketone to react with a nucleophilic alkylating agent to produce an alcohol, followed by the step of (ii) allowing the resultant alcohol to be displaced by halide resulting in a halogenated alkane.
Step (i) can be carried out using a conventional Grignard reaction process, wherein an alkyl magnesium halide Grignard reagent is allowed to react with a ketone resulting in an alcohol. This step can be carried out in a solvent in which the Grignard reagent is prepared, and the ketone has solubility. Example such solvent includes hydrocarbon ethers, such as diethyl ether. This method can be carried out at low temperature, for example -20°C, during the contacting of the Grignard reagent and ketone. Following the addition of all reaction components the resultant reaction mixture is then typically stirred at low temperature until the reaction is complete, for example 30 minutes. This method is normally carried out at ambient pressure and the product alcohol isolated by aqueous washing followed by purification by vacuum distillation.
Step (ii) can be carried out by allowing the product alcohol to react with a halogen acid, preferably HCl, resulting in a halogenated alkane. This step can be carried out in water in which the alcohol, optionally with a cosolvent, in which the alcohol has suitable solubility. This method can be carried out at room temperature. Following the addition of the alcohol to the HCl, the resultant reaction mixture is then typically stirred at ambient temperature until the reaction is complete, for example 30 minutes. This method is normally carried out at ambient pressure and the product halogenated alkane isolated by aqueous washing followed by purification, for example by vacuum distillation or recrystallization as appropriate.
Steps (i) and (ii) described above result in the formation of a halogenated alkane, which can be used in the manufacture of hydrophobic hydrocarbon-containing moiety by the electrophilic aromatic alkylation method described earlier herein.
An example of a novel hydrocarbon dispersing agent produced using this method is the Synthesis Example for I-4.

**Production Method: Oxidation of Sulfide or Phosphine Moieties**

In one embodiment the hydrocarbon dispersing agent has a sulfonyl moiety that can be produced by a method

including the step of allowing an appropriate sulfide compound to react with an appropriate oxidizing agent, for example hydrogen peroxide. This method can be carried out in a solvent in which the aromatic sulfide has solubility. Example such solvent includes water. This method can be carried out at ambient temperature to about 95°C in some cases during the contacting of the sulfide and oxidizing agent. Following the combination of all reaction components the resultant reaction mixture is then typically stirred at ambient temperature until the reaction is complete, for example 60 minutes. This method is normally carried out at ambient pressure and the product isolated and purified, for example by vacuum distillation or recrystallization as appropriate.

Examples of novel hydrocarbon dispersing agents having a sulfonyl moiety produced using this method include Synthesis Examples for C-8, I-3, and I-13.

In one embodiment the hydrocarbon dispersing agent has a sulfinyl moiety that can be produced by a method including the step of allowing an appropriate sulfide compound to react with an appropriate oxidizing agent, for example hydrogen peroxide. The method for preparing the sulfinyl embodiment of the hydrocarbon dispersing agent can be essentially identical to the method for preparing the sulfonyl embodiment, except that the sulfonyl embodiment will require relatively more oxidizing agent than in the case of the sulfinyl embodiment. For example, the sulfonyl embodiment requires at least 2 equivalents of oxidizing agent per equivalent of sulfide compound, whereas the sulfinyl embodiment requires 1 or less equivalents of oxidizing agent per equivalent of sulfide compound.

Examples of novel hydrocarbon dispersing agents having a sulfinyl moiety produced using this method include Synthesis Examples for I-11 and I-12.

In one embodiment the hydrocarbon dispersing agent has a phosphoryl moiety that can be produced by a method including the step of allowing an appropriate phosphine compound to react with an appropriate oxidizing agent, for example hydrogen peroxide. The method for preparing the phosphoryl embodiment of the hydrocarbon dispersing agent can be essentially identical to the method for preparing the sulfonyl embodiment, except that the phosphoryl embodiment requires only about 1 or less equivalents of oxidizing agent per equivalent of phosphine compound.

An example of a novel hydrocarbon dispersing agent having a phosphoryl moiety produced using this method is the Synthesis Example for C-9.

**Production Method: Nucleophilic Displacement**

**of Halide by Aromatic Sulfide or Phenoxide**

In certain embodiments production of the hydrocarbon dispersing agent involves a process (i) of forming a sulfur-carbon bond resulting in formation of an alkyl phenyl sulfide, or a process (ii) of forming an oxygen-carbon bond resulting in formation of an alkyl phenyl ether. In these embodiments, processes (i) and (ii) involve nucleophilic displacement of a halogen from an alkyl halide by a (i) phenyl sulfide or (ii) phenoxide, respectively.

Process (i) can be carried out by allowing a suitable thiophenolate salt to react with a suitable alkyl halide. This process can be carried out in water as the solvent in the presence of phase transfer catalyst, such as alkyl phosphonium halide. This process can be carried out at elevated temperature, such as 70°C, for an appropriate period of time, such as 4 hours. This method is normally carried out at ambient pressure and the product alkyl phenyl sulfide isolated by extraction into organic solvent, aqueous washing, drying and purification, for example by vacuum distillation or recrystallization as appropriate.

Examples of novel hydrocarbon dispersing agents produced using this process include Synthesis Examples for I-13 and I-12.

Process (ii) can be carried out by allowing a suitable phenoxide salt to react with a suitable alkyl halide. This process can be carried out in polar aprotic solvent, such as ethanol or ethyl acetate, or mixtures of such. This process can be carried out at elevated temperature, such refluxing ethyl acetate/ethanol, for an appropriate period of time, such as 3 hours. This process is normally carried out at ambient pressure and the product alkyl phenyl ether isolated by extraction into organic solvent, aqueous brine washing, drying and purification, for example by vacuum distillation or recrystallization as appropriate.

An example of a novel hydrocarbon dispersing agent produced using this process is the Synthesis Example for I-5.

**Production Method: Epoxidation of Olefin**

**Followed by Epoxide Opening by Bisulfite**

In one embodiment a hydrocarbon dispersing agent can be produced by a two-step method including steps of (i) allowing an appropriate hydrophobic hydrocarbon-containing alkene to react with an epoxidizing agent to produce an epoxide of the alkene, followed by the step of (ii) ring opening of the epoxide with bisulfite resulting in a sulfonate-containing

hydrocarbon dispersing agent.

Step (i) can be carried out by allowing an appropriate hydrophobic hydrocarbon-containing alkene to react with a peroxycarboxylic acid, such as m-chloroperbenzoic acid, resulting in epoxidation of the alkene. This step can be carried out in a solvent, such as basic buffered aqueous solution or organic solvent, such as methylene chloride. This method can be carried out at ambient temperature and pressure. Following the addition of all reaction components the resultant reaction mixture is then typically stirred until the reaction is complete, for example 2 days. The product epoxide can be isolated from the reaction mixture by extraction into organic solvent and then aqueous washing (in the embodiment where the reaction solvent is aqueous), drying and purification, for example by vacuum distillation or recrystallization as appropriate.

Step (ii) can be carried out by allowing the product epoxide to react with sulfite, resulting in a sulfonate-containing hydrocarbon dispersing agent. This step can be carried out in water as the solvent in a pressure vessel at elevated temperature, such as 145°C, at the autogenous elevated pressure. Following the addition of all reaction components, the resultant reaction mixture is then typically stirred at elevated temperature and pressure until the reaction is complete, for example 6 hours. The product sulfonate-containing hydrocarbon dispersing agent can be isolated from the reaction mixture, normally by filtration followed by purification, for example by recrystallization from an alcohol, such as ethanol.

An example of a novel hydrocarbon dispersing agent produced using this process is the Synthesis Example for C-10.

**Production Method: Nucleophilic Displacement**

**of Halide from Aromatic Sulfonyl Chloride by Amine**

In one embodiment the hydrocarbon dispersing agent has a hydrophobic hydrocarbon-containing moiety that can be produced by a method including the step of allowing an appropriate aromatic sulfonyl chloride compound to react with an appropriate amine. This method is typically carried out in a solvent in which the reaction components have solubility. Example such solvent includes water containing a miscible polar aprotic cosolvent, such as acetone. This method can be carried out at low temperature during the combination of the sulfonyl chloride and amine, for example 0°C. Following the addition of all reaction components the resultant reaction mixture is then typically stirred at ambient temperature until the reaction is complete, for example 18 hours. This method is normally carried out at ambient pressure and the product isolated and purification by vacuum distillation. The product dispersing agent can be isolated from the reaction mixture, normally by filtration followed by purification, for example by recrystallization.

An example of a novel hydrocarbon dispersing agent produced using this process is the Synthesis Example for I-10.

**Production Method: Reduction of Phenol**

In one embodiment a hydrocarbon dispersing agent can be produced by a two-step method including the steps of (i) reduction of an appropriate hydrophobic hydrocarbon-containing phenol, followed by the step of (ii) aromatic sulfonation.

Step (i) can be carried out by allowing an appropriate hydrophobic hydrocarbon-containing phenol to react in two steps, (i-1) with sulfuryl fluoride in the presence of triethyl amine in DMSO as solvent, and then (i-2) with formic acid in the presence of triethyl amine and a catalyst of palladium(II) acetate and 1,3-bis(diphenylphosphino)propane. Step (i-1) can be carried out at ambient temperature and autogenous pressure. Following the addition of all reaction components the resultant reaction mixture is then typically stirred until the reaction is complete, for example 6 hours. The resultant solution is sparged with nitrogen and then step (i-2) is carried out by adding the triethyl amine, palladium(II) acetate and 1,3-bis(diphenylphosphino)propane to the reaction mixture. Then formic acid is added dropwise over a period of a few hours such that the reaction temperature does not exceed 60°C through use of a cold bath. The product reduced phenol can be isolated from the reaction mixture by extraction into organic solvent and then aqueous washing, drying and purification, for example by vacuum distillation or recrystallization as appropriate.

Step (ii) aromatic sulfonation of the product reduced phenol can be carried out as described earlier herein. The product sulfonate-containing hydrocarbon dispersing agent can be isolated from the sulfonation reaction mixture, followed by purification by vacuum distillation.

An example of a novel hydrocarbon dispersing agent produced using this process is the Synthesis Example for I-9.

**Production Method: Basic Hydrolysis of Ester**

In one embodiment the hydrocarbon dispersing agent has a carboxylic acid that is obtained by basic hydrolysis of

an ester. The carboxylic acid group can be produced from the ester by a method including the step of allowing an appropriate precursor aromatic compound ester to react with hydroxide. This method can be carried out in a solvent in which the precursor has solubility, for example a mixture of tetrahydrofuran and water. This method can be carried out at elevated temperature, for example 60°C, during combination of the precursor and hydroxide. The resultant reaction mixture can then be stirred at elevated temperature, for example 60°C, and ambient pressure until the reaction is complete, for example 16 hours. The product carboxylic acid can be isolated from the reaction mixture by extraction into organic solvent and then aqueous washing, drying and purification, for example by vacuum distillation or recrystallization as appropriate.

An example of a novel hydrocarbon dispersing agent having a carboxylic acid moiety produced using this ester hydrolysis method is the Synthesis Example for I-5.

**Production Method: Neutralization of Sulfonic**

**or Carboxylic Acids with Various Bases**

In one embodiment the hydrocarbon dispersing agent is a salt of a carboxylic or sulfonic acid, and the salt is produced by a method of basic neutralization of the acid. This method can be carried out in water, organic solvent, or water containing a suitable polar organic cosolvent miscible with water, such as ethanol or tetrahydrofuran. A slurry or solution of the acid is prepared in the solvent, and then the acidic solution is neutralized to a pH of about 8 with base containing the desired cation, such as aqueous hydroxides of sodium, potassium or ammonia. In one embodiment, the solvent is polar aprotic, for example diethyl ether, and the neutralizing base is anhydrous ammonia. The product carboxylic or sulfonic acid salt can be isolated from the reaction mixture, for example by filtration, and further purified as desired, for example by recrystallization.

Examples of novel hydrocarbon dispersing agents produced using this neutralization process include the Synthesis Examples for the sodium, potassium or ammonia salts of C-8, C-12, I-1, I-4, and I-8.

**Polymerization Process**

The process of the invention may be carried out in a pressurized polymerization reactor suitable for producing an aqueous dispersion of fluoropolymer particles by polymerization of fluoromonomer. Gaseous monomers such as TFE or VF2 are typically fed into the reactor to maintain the operating pressure, typically in the range of 30 to 1000 psig (0.3 to 7.0 MPa). Other or additional gaseous monomers can be fed into the reactor depending upon the fluoropolymer being manufactured. Liquid monomers can be pre-charged and/or pumped into the reactor as desired for the type of fluoropolymer being produced.

A batch or continuous process may be used although batch processes are typically used for commercial production of perfluoropolymers. Continuous processes are known for use for some grades of fluoroelastomers. In a batch process, the reactor is preferably equipped with a stirrer for the aqueous medium and the aqueous medium is preferably stirred throughout the polymerization. The reactor is also preferably equipped with a jacket surrounding the reactor so that the reaction temperature may be conveniently controlled by circulation of a controlled temperature heat exchange medium.

The aqueous medium provided in the polymerization reactor is preferably deionized and deaerated water. The temperature of the reactor and thus of the aqueous medium will preferably be from 25 to 120°C. For the production of PTFE homopolymers, paraffin wax is typically employed in the reactor as a stabilizer and the polymerization temperature employed will typically be above the melting point of the wax.

Polymerization initiator is added to the aqueous medium to cause the polymerization of fluoromonomer and form fluoropolymer particles in the aqueous medium. This is suitably accomplished using an aqueous solution of the polymerization initiator which is typically pumped into the reactor in sufficient amount to cause commencement of the polymerization reaction which is frequently referred to in the art as the "kick-off" of the polymerization reaction. Kick-off is typically determined by a reduction in reactor pressure from its initial pressurization, e.g. by a pressure drop of 10 psi (69 kPa), indicating the commencement of fluoromonomer consumption in the polymerization process and thereby commencement of the polymerization reaction. Kick-off can also be determined in a process in which reactor pressure is maintained constant by the increase in the feed rate of gaseous monomers into the reactor to maintain pressure.

The polymerization initiator employed is preferably a water-soluble free-radical polymerization initiator. For polymerization of TFE to produce PTFE, one preferred initiator is organic peracid such as disuccinic acid peroxide (DSP), which requires a large amount to cause kick-off, e.g. at least 600 ppm. Optionally, reducing agents such as sodium formaldehyde sulfoxylate, fluoroalkyl sulfinates, metabisulfite, or ascorbic acid can be used together with the organic peracid. A highly active initiator, such as inorganic persulfate salt such as ammonium persulfate can be used together with the organic peracid in a smaller amount. Organic peroxide initiators that are sufficiently soluble in aqueous media and

readily produce alkoxy radicals such as alkyl hydroperoxides, e.g., t-butyl hydroperoxide, optionally combined with reducing agents such as sodium formaldehyde sulfoxylate, fluoroalkyl sulfinates, metabisulfite, or ascorbic acid, are advantageously used as initiators together with the hydrocarbon dispersing agents for use in the present invention, especially for PTFE polymerization. For TFE copolymers such as FEP and PFA, and for fluoroelastomers, inorganic persulfate salt such as ammonium persulfate can be used as the initiator. Other initiators known in the art can also be used in a process in accordance with the invention.

As is known in the art, the polymerization initiator added to cause kick-off can be supplemented by pumping additional initiator solution into the reactor as the polymerization reaction proceeds. For higher solids batches, it is preferable to add supplemental initiator as the polymerization proceeds.

For the production of modified PTFE and for the production of TFE copolymers, relatively inactive fluoromonomer such as hexafluoropropylene (HFP) can already be present in the reactor prior to pressuring up with the more active TFE fluoromonomer. After kick-off, TFE is typically fed into the reactor to maintain the internal pressure of the reactor at the operating pressure. Additional comonomer such as HFP or perfluoro (alkyl vinyl ether) can be pumped into the reactor if desired. The aqueous medium should be adequately stirred to obtain a desired polymerization reaction rate and incorporation of comonomer, if present. For fluoroelastomer production, a mixture of the gaseous monomers in proportions needed to produce the final fluoroelastomer are typically fed into the reactor, e.g., VF2 and HFP for VF2/HFP dipolymers or VF2, HFP and TFE for VF2/HFP/TFE terpolymers. For peroxide curable fluoroelastomers, a cure site monomer that introduces an iodine or bromine cure site into fluoroelastomer, e.g., 4-iodo-3,3,4,4-tetrafluorobutene-1 (ITFB), is preferably added later in the batch.

Chain transfer agents can be introduced into the reactor when molecular weight control is desired, and they are sometimes employed in the production of melt-processable fluoropolymers such as PFA. Preferred chain transfer agents include hydrogen, aliphatic hydrocarbons which may be cyclic, halocarbons, hydrohalocarbons or alcohol having 1 to 20 carbon atoms, more preferably 1 to 8 carbon atoms. Representative preferred examples of such chain transfer agents are alkanes such as ethane, chloroform, 1,4-diiodoperfluorobutane and methanol. The amount of a chain transfer agent and the mode of addition depend on the activity of the particular chain transfer agent and on the desired molecular weight of the polymer product. The amount of chain transfer agent supplied to the polymerization reactor is preferably 0.005 to 5 wt%, more preferably from about 0.01 to about 2 wt% based upon the weight of the resulting fluoropolymer. For peroxide curable fluoroelastomers, iodine-containing or bromine-containing chain transfer agents such as the diiodoperfluoroalkyl compounds discussed above can be used as chain transfer agents that provide iodine or bromine cure sites bonded to terminal carbons of the fluoroelastomer.

In one preferred embodiment of the process in accordance with the invention, polymerization sites are provided in the aqueous medium. Polymerization sites in the aqueous medium increase the number of locations in the aqueous medium for the precipitation of fluoropolymer, thereby resulting in the smaller fluoropolymer particle size for a given percent solids. The hydrocarbon dispersing agents employed in accordance with the present invention vary in their effectiveness to nucleate in the polymerization process. Although not needed for some hydrocarbon dispersing agents used in accordance with the invention, providing polymerization sites is important for dispersing agents that are not effective for nucleation and especially for processes for achieving high solids levels with such dispersing agents. The performance of the polymerization sites employed in the fluoropolymerization can be judged primarily by the smaller particle size of the fluoropolymer compared to conducting the polymerization reaction without the polymerization sites being present.

The polymerization sites can be provided in the aqueous medium either before or simultaneously with commencement of polymerization. Preferably, the polymerization sites are present before commencement of polymerization. For polymerization sites that are formed by reaction *in situ* as are the oleophilic nucleation sites described below, it can be advantageous to form such polymerization sites simultaneously with commencement of polymerization to shorten batch times.

Providing polymerization sites in the aqueous medium is preferably accomplished by the addition of a nucleating additive to the aqueous medium. There are a variety of nucleating additives that can be used to provide polymerization sites.

One method of forming polymerization sites is to add a dispersion of small non-ionomeric fluoropolymer particles to the aqueous polymerization medium. These polymerized particles as a nucleating additive are often called polymer seeds. As is known in the art, the seeds may be formed by free-radical initiated aqueous dispersion polymerization of fluoromonomer that can include the use of a suitable surfactant or dispersing agent that stabilizes the fluoropolymer seeds in the aqueous medium. The polymer seeds may be the same or different from the fluoropolymer to be produced in the process but usually they are same as the polymer being produced. Typically, fluoropolymer dispersions for use in providing fluoropolymer seeds have very small particle size, e.g. 1 to 50 nm, and are made by running a polymerization process to only low solids levels when the size of the particles is only a fraction of the size typically desired for commercial use. In the event that fluorosurfactant is used as the stabilizing surfactant to make the polymer seeds, only a small amount of fluorosurfactant is typically necessary to maintain the polymer seeds in dispersion.

One preferred method of providing polymerization sites for use in carrying out a process in accordance with the present invention is disclosed in U.S. Patent 8,153,738 (Leffew et al.), wherein the polymerization sites are provided by adding a dispersed particulate of fluorinated ionomer as nucleating additive to the aqueous medium. Because the dispersed particulate of fluorinated ionomer contains a large number of ionic groups that are typically present along the polymer chain, the dispersed particulate of fluorinated ionomer is self-stabilizing as a dispersion in aqueous media and no surfactant is typically needed. Preferably, the dispersed particulate of fluorinated ionomer is "highly fluorinated" meaning that at least 90% of the univalent atoms bonded to carbon atoms in the ionomer are fluorine atoms. Most, preferably, the ionomer is perfluorinated. It is also preferred for the ionic groups in the ionomer to be sulfonic acid or sulfonate groups. Various types of fluorinated ionomer are suitable for the production dispersed particulate of fluorinated ionomer such as those disclosed in U.S. Patent Nos. 3,282,875, 4,358,545 and 4,940,525.

Although other processes can be used, it is preferred for the dispersed particulate of fluorinated ionomer to be formed by a process as disclosed in U.S. Patent 6,150,426 (Curtin et al.). The process of U.S. Patent 6,150,426 can be used to form a dispersed particulate of fluorinated ionomer in water that is free of organic compounds that could interfere with polymerization. The dispersed particulate in aqueous media as formed by the process of US Patent 6,150,426 also can be dried to form a powdered solid that can be readily re-dispersed in aqueous media. In addition, the particle size of the dispersed particulate of fluorinated ionomer formed by this method is very small, e.g., 2 to 30 nm, and is very effective in creating a large number of polymerization sites when used as a nucleating additive.

In another preferred method of providing polymerization sites, a hydrocarbon-containing compound is used as a nucleating additive to form oleophilic nucleation sites formed in the aqueous medium as disclosed in U.S. Patent Nos. 8,563,670 and 9,676,929 (Brothers et al.). These oleophilic nucleation sites are formed *in situ* and dispersed in the aqueous medium to provide the polymerization sites. The oleophilic nucleation sites are formed by the addition of small amounts of water-soluble hydrocarbon-containing compound and degradation agent, preferably an oxidizing agent, to the aqueous medium prior to or during commencement of polymerization. Preferably, the water-soluble hydrocarbon-containing compound is added in an amount of no greater than 50 ppm. The degradation agent subjects the hydrocarbon-containing compound to a reaction that degrades the compound thereby enabling the water-soluble hydrocarbon-containing compound to form the oleophilic nucleation sites.

In some embodiments, the water-soluble hydrocarbon-containing compound from which the oleophilic nucleation sites are derived is hydrocarbon-containing surfactant as disclosed in U.S. Patent No. 8,563,670 (Brothers et al.) A wide variety of hydrocarbon-containing surfactants are suitable for use in forming the oleophilic nucleation sites as disclosed in U.S. Patent No. 8,563,670. Preferably, nonionic hydrocarbon surfactants are employed such as polyoxyethylene alkyl ethers, polyoxyethylene alkyl phenyl ethers, polyoxyethylene alkyl esters, sorbitan alkyl esters, polyoxyethylene sorbitan alkyl esters, glycerol esters, their derivatives and the like. The preferred degradation of the surfactant causes the surfactant to lose hydrophilicity and its surfactant effect. However, no surfactant is necessary for the maintenance of the oleophilic nucleation sites as a dispersion to function as polymerization sites for the polymerization reaction.

In other embodiments, the water-soluble hydrocarbon-containing compound as a nucleating additive is polyalkylene oxide having a number average molecular weight of 50 to 2000 as disclosed in U.S. Patent No. 9,676,929 (Brothers et al.) By "polyalkylene oxide" is meant an oligomeric compound or mixtures of oligomeric compounds having oligomeric alkylene oxide segments such as polyethylene oxide, polyethylene oxide, polypropylene oxide, and polytetramethylene oxide. More than one type of segment can he present in a polyalkylene oxide useful for the practice of the present invention. Preferably, polyalkylene oxides are employed which have only one type of segment. Polyalkylene oxides may contain oligomeric compounds with differing molecular weights and most commercially available materials are sold as mixtures of compounds having a stated average molecular weight and thus contain compounds with a distribution of molecular weights around the average. Mixtures of compounds with greater differences in molecular weights or entirely different chemical compositions may be employed if desired by mixing different polyalkylene oxides. The polyalkylene oxides may be terminated with any of a wide variety of end groups and the end groups in a particular compound may be the same or different. An especially preferred alkylene oxide nucleating additive is polypropylene glycol.

Preferably, degradation of the water-soluble hydrocarbon-containing compound is carried out by adding degradation agent, preferably oxidizing agent, to the aqueous medium. Preferably, the degradation agent is a free radical polymerization initiator.

Preferably, the formation of the dispersion of oleophilic nucleation sites as described above is accompanied by the additional step of adding water-soluble inorganic salt to the aqueous medium prior to the exposure of the hydrocarbon-containing compound, to the degradation. Prior to or during exposure of the nucleating additive to oxidizing agent, water-soluble inorganic salt preferably is added to the aqueous medium. The addition of water-soluble inorganic salt is useful to increase the number of fluoropolymer particles formed during nucleation. Preferred amounts of water-soluble inorganic salt are 0.01 to 80 ppm, Suitable water-soluble inorganic salts include but are not limited to sodium sulfite, sodium bisulfite, sodium chloride, potassium sulfite, potassium bisulfite, potassium carbonate, ammonium oxalate, sodium tetraborate, sodium acetate, ammonium carbonate, ammonium phosphate monobasic, and ammonium phosphate dibasic.

Another preferred method for providing polymerization sites is to employ a fluoropolyether acid or salt nucleating additive of the type disclosed in U.S. Patent 7,897,682 (Brothers et al) for use in a polymerization agent combination with a hydrocarbon surfactant. Preferably, the fluoropolyether nucleating additive is a perfluoropolyether acid or salt thereof. The acid groups of the fluoropolyether acid or salt thereof preferably are acid groups selected from carboxylic acid, sulfonic acid, phosphonic acid. In preferred embodiments, the acid group of the fluoropolyether acid or salt there is carboxylic acid. Preferably, the fluoropolyether acid is employed as an alkali metal or ammonium salt, most preferably, a sodium or ammonium salt.

Preferred perfluoropolyether (PFPE) acids or salts thereof for use in accordance with the present invention can have any chain structure in which oxygen atoms in the backbone of the molecule are separated by saturated fluorocarbon groups having 1-3 carbon atoms. More than one type of fluorocarbon group may be present in the molecule. Representative structures have the repeat unit

$$(\text{-CFCF}_3\text{-CF}_2\text{-O-})_n \qquad (I)$$

$$(\text{-CF}_2\text{-CF}_2\text{-CF}_2\text{-O-})_n \qquad (II)$$

$$(\text{-CF}_2\text{-CF}_2\text{-O-})_n\text{-}(\text{-CF}_2\text{-O-})_m \qquad (III)$$

$$(\text{-CF}_2\text{-CFCF}_3\text{-O-})_n\text{-}(\text{-CF}_2\text{-O-})_m \qquad (IV)$$

These structures are discussed by Kasai in J. Appl. Polymer Sci. 57, 797 (1995). As disclosed therein, such PFPE can have a carboxylic acid group or salt thereof at one end or at both ends. Similarly, such PFPE may have a sulfonic acid or phosphonic acid group or salt thereof at one end or both ends. In addition, PFPE with acid functionality at both ends may have a different group at each end. For monofunctional PFPE, the other end of the molecule is usually perfluorinated but may contain a hydrogen or chlorine atom. Typically, unless extraordinary care is employed to manufacture a single specific PFPE compound, the PFPE contains multiple compounds in varying proportions within a molecular weight range about the average molecular weight.

The fluoropolyether acid or salt thereof has an average molecular weight which enables it to function effectively as a nucleating agent. The number average molecular weight of the fluoropolyether acid or salt preferably is greater than 500 g/mol but less than 6000 g/mol because fluoropolyether acids or salt with very high molecular weights generally are difficult to dissolve/disperse in the aqueous polymerization medium. It has been found to be advantageous to mix the fluoropolyether acid or salt with low telegenic or non-telogenic dispersing aid such as a polar solvent that does not significantly interfere with the fluoropolymerization. One preferred class of dispersing aids includes C3 to C8 branched alcohols with a particularly suitable dispersing aid being t-butanol. Preferably, the t-butanol is employed with the perfluoropolyether acid or salt at a weight ratio of 1:2 to 2:1 t-butanol to perfluoropolyether acid, more preferably with t-butanol being in an amount less than the amount of the perfluoropolyether acid.

A particularly preferred fluoropolyether acid or salt for use as a nucleating additive that disperses effectively and provides effective nucleation is disclosed in U.S. Patent 9,732,212. This perfluoropolyether acid or salt is a mixture of fluoropolyether acids or salts having a number average molecular weight of 800 to 2500 g/mol. In the mixture, the amount of fluoropolyether acids or salt having a molecular weight of not more than 500 g/mol is not more than 50 ppm by weight of the total amount of fluoropolyether acids or salts, the amount of fluoropolyether acids or salts in the mixture having a molecular weight of 2500 g/mol or greater is not more than 40% by weight of the total amount of fluoropolyether acids or salts, and the amount of fluoropolyether acids or salts in the mixture having a molecular weight of 3000 g/mol or greater is not more than 10% by weight of the total amount of fluoropolyether acids or salts.

To carry out the polymerization process in accordance with the invention, hydrocarbon dispersing agent in accordance with the invention is added to the aqueous medium so that the aqueous medium contains the hydrocarbon dispersing agent. The amount of hydrocarbon dispersing agent in the aqueous medium will vary with the type of dispersing agent, the fluoropolymer being produced, and the desired solids level. A preferred range is 50 ppm to 10,000 ppm based on the weight of fluoropolymer solids, more preferably 100 ppm to 5000 ppm based on the weight of fluoropolymer solids, more preferably 1000 ppm to 5000 ppm based on the weight of fluoropolymer solids, and most preferably 1000 ppm to 4000 ppm based on the weight of fluoropolymer solids.

Because the hydrocarbon dispersing agents for use in accordance with the invention have low reactivity with the polymerization initiator and/or the propagating fluoropolymer radical, all of the hydrocarbon dispersing agent for the polymerization can be added by pre-charging into the reactor prior to or simultaneously with commencement of polymerization. Alternatively, any of a variety of other methods can be used for providing the hydrocarbon dispersing agent in the aqueous medium. For example, addition of the hydrocarbon dispersing agent can be delayed until after commencement of polymerization as in the process disclosed in WO2012/064841A1. Another method is to pre-charge a portion of the hydrocarbon dispersing agent and add one or more portions later. Hydrocarbon dispersing agent can be

added continuously into the aqueous medium during the entire batch or continuously after commencement of polymerization as disclosed in WO2012/064841A1. Continuous addition of hydrocarbon dispersing agent into the batch can be combined with a portion of the hydrocarbon dispersing agent being pre-charged before or simultaneously with commencement of polymerization. Combinations of these methods, as well as other methods, can be used as desired to add the hydrocarbon dispersing agent to the aqueous medium.

For achieving high solids batches and/or reducing batch time, especially for producing perfluoropolymers, the addition of hydrocarbon dispersing agent can be delayed until after commencement of polymerization and to add the hydrocarbon dispersing agent added over time as polymerizing is carried out. The hydrocarbon dispersing agent can be fed into the reactor in a continuous manner as the polymerization proceeds, i.e., to be metered into the reactor. The techniques as disclosed in WO2012/064841A1 can be employed for the delayed addition and continuous feeding of hydrocarbon dispersing agents to carry out a process in accordance with the present invention.

It is preferred to provide polymerization sites in the aqueous medium by one of the methods discussed above if the hydrocarbon dispersing agent does not provide effective nucleation, especially to achieve high solids. It can be advantageous in a process in accordance with the present invention for polymerization sites to be provided in combination with the delayed addition of hydrocarbon dispersing agent and continuous feeding of hydrocarbon dispersing agents into the reactor.

After completion of the polymerization when the desired amount of dispersed fluoropolymer solids content has been achieved (typically several hours in a batch process), the feeds are stopped, the reactor is vented, and the raw dispersion of fluoropolymer particles in the reactor is transferred to a cooling or holding vessel.

The process of the present invention is capable of producing fluoropolymer in a wide range solids contents and the solids content of the aqueous fluoropolymer dispersion as polymerized can range from 10% by weight to up to 65 wt%. The process preferably produces an as polymerized dispersion with a solids content of at least 30 wt%, more preferably, at least 40%. Particle size (Dv(50)) of the fluoropolymer particles in the aqueous fluoropolymer dispersion can range from 10 nm to 500 nm, preferably Dv(50) 100 to 400 nm.

In a preferred process of the invention, polymerizing produces less that 10 wt%, more preferably less than 3 wt% undispersed fluoropolymer based on the total weight of fluoropolymer produced. Undispersed fluoropolymer (often referred to as coagulum) is fluoropolymer which does not remain in the aqueous medium as dispersed fluoropolymer particles and usually will remain inside the reactor or will be present in large particles which settle or are filtered out of the dispersion. Undispersed polymer typically must be discarded as waste.

For use in fluoropolymer coatings on materials such as metals, glass and fabric, PTFE dispersion is typically transferred to a dispersion concentration operation which produces stabilized concentrated dispersions useful as coatings or for addition to coating formulations. Typically, concentrated dispersions are stabilized with nonionic surfactants and concentrated by known methods. The solids content of concentrated dispersion is typically 35 to 70 wt%.

Certain grades of PTFE dispersion are made for the production of fine powder. For this use as is known in the art, the dispersion is coagulated, the aqueous medium is removed, and the PTFE is dried to produce fine powder.

For melt processible fluoropolymers such as FEP and PFA, methods well known in the art can be used to coagulate the dispersion and dry the fluoropolymer resin which is then typically processed into a convenient form such as chip or pellet for use in subsequent melt-processing operations.

For fluoroelastomers, the dispersion is also coagulated. As is known in the art for use in the manufacture of fluoroelastomers parts, the coagulated fluoroelastomer is typically formed into slabs that are convenient for subsequent compounding with curing additives, fillers, pigments, etc., prior to being molded into parts and cured by heating.

## TEST METHODS

Raw Dispersion Particle Size (RDPS) is measured using laser light scattering with a Zetasizer Nano-ZS manufactured by Malvern Instruments. Samples for analysis are prepared in 10x10x45 mm polystyrene cuvettes, capped and placed in the device for analysis. Preparation of the sample is as follows. Water used to flush the cuvette and used to dilute the dispersion sample is rendered substantially free of particles by drawing deionized, deaerated water into a 10 cc glass hypodermic syringe with locking tip. A Whatman 0.02 micron filter (Cat. No. 6809-2002) is fitted to the locking tip of the syringe and pressure is applied force water through the filter and into the cuvette. Approximately 1.5 ml of water is placed in the cuvette, the cuvette is capped, shaken and uncapped. Water is poured out of the cuvette thus assuring the cuvette is free of particles. Approximately 2.5 gm of filtered water is placed in the cuvette. One drop of the fluoropolymer dispersion to be analyzed is added to the cuvette. The cuvette is capped and shaken to completely mix the fluoropolymer particles in the water. The sample is placed in the Nano-ZS for determination of Dv(50). Dv(50) is the median particle size based on volumetric particle size distribution, i.e. the particle size below which 50% of the volume of the population resides.

The melting point ($T_m$)

Weight % fluoropolymer solids in aqueous dispersions is determined by moisture analyzer as follows: Wt% fluoropolymer solids of the fluoropolymer dispersion is measured using MB45 Moisture Analyzer manufactured by Ohaus.

The measurement is carried out as follows. A clean glass fiber pad is placed on the instrument balance, and tared. A two-gram dispersion sample is pipetted on the glass fiber pad. The drying process is started by pushing the start button. The integral halogen dryer is programed to reach 175°C. During the drying process, water will vaporize and upon completion, the results are displayed as wt% solids. Typical drying time is about 5 minutes.

*Number of particles per cm$^3$ (Particle # per cm$^3$)* is calculated from the Weight % fluoropolymer solids using the following equation:

$$Particle\ Number = \frac{(\frac{wt\%\ Solids}{100} * Dispersion\ Weight)}{\text{Polymer Density}} * 6 * \frac{1{,}000{,}000^3}{\pi / DV(50)^3}$$

*Kick-off Time* is considered to be commencement of polymerization and calculated as time required for 10 psi pressure drop from the maximum pressure observed during the injection of initiator solution or the pressure drop observed after a set time if 10 psi is not reached. Kick-off Rate (psi/min) is calculated from the kick-off time, i.e., 10 psi divided by the Kick-off Time if kick-off occurs or, if kick-off does not occur, the pressure drop observed divided by the set time.

*Inherent Viscosity* of fluoroelastomer is determined in accordance with ASTM D5225-92 using a Viscotek Y501 model viscometer sold by Malvern Panalytical Ltd. The fluoroelastomer is dissolved in in a methyl ethyl ketone solvent.

*Mooney Viscosity* is measured under conditions ML 1 + 10 (121°C) according to ASTM D1646.

*Compression Set Resistance* is the percentage change after 70 hr at 200°C as determined by following the ISO 815-1:2008 testing protocol.

*Tensile Strength* and Elongation at Break are determined by the ISO 37:2005 C or 1 2008 testing protocol.

*Standard Specific Gravity (SSG)* is measured by ASTM D792-08. *T-butylhydroperoxide Oxidative Loss (wt%)* is determined by the test method described in Reactivity Example 1.

| Table A - Hydrocarbon Dispersing Agents | | | | | | | |
|---|---|---|---|---|---|---|---|
| Dispersing Agent No. | Name | Structure | %$CH_3$ Based on total $CH_3$, $CH_2$, and CH groups | Number of $CH_3$ Groups | MW (Without counter ion) | Hammett Sigma Plus Value of Aromatic Groups | Carbon Atoms |
| C-1 | Dodecyl sulfate | | 8.3 | 1 | 265.4 | N/A | 12 |
| C-2 | Linear dodecyl benzene sulfonate (has 1 branch) | | 16.7 | 2 | 325.5 | 0.06 | 18 |
| C-3 | Branched dodecyl benzene sulfonate | | 55* | 6.6* | 325.5 | 0.06 | 18 |
| C-4 | Tert-butylbenzene sulfonate | | 100 | 3 | 213.3 | 0.09 | 10 |
| C-5 | Octyl sulfonate | | 13 | 1 | 193.3 | N/A | 8 |
| C-6 | Naphthalene sulfonic Acid-formaldehyde condensate | | 0 | 0 | | N/A | |
| C-7 | Octyl sulfate | | 13 | 1 | 209.3 | N/A | 8 |
| C-8 | Sodium 4-(phenylsulfonyl)-benzenesulfonate | 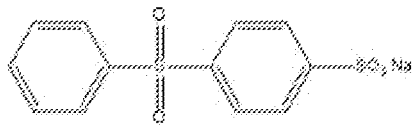 | 0 | 0 | 297.3 | A:0.65<br>B:0.98 | 12 |
| C-9 | 3-(Diphenylphosphoryl)-benzenesulfonate | 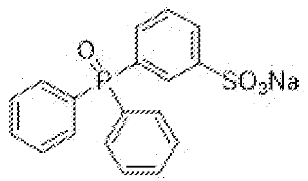 | 0 | 0 | 357.3 | A: 0.52<br>B: 0.87 | 18 |
| C-10 | 2-Hydroxy-2,4,4-trimethylpentane-1-sulfonate | | 66.7 | 4 | 209.3 | N/A | 8 |
| C-11 | 3,5-Diisopropyl-benzenesulfonate | | 66.7 | 4 | 241.3 | -0.21 | 12 |
| C-12 | 3,5-Di-tert-butyl, 4-hydroxybenzenesulfon ate | | 100 | 6 | 285.4 | -1.09 | 14 |

(continued)

**Table A - Hydrocarbon Dispersing Agents**

| Dispersing Agent No. | Name | Structure | $\%CH_3$ Based on total $CH_3$, $CH_2$, and CH groups | Number of $CH_3$ Groups | MW (Without counter ion) | Hammett Sigma Plus Value of Aromatic Groups | Carbon Atoms |
|---|---|---|---|---|---|---|---|
| I-1 | 3,5-Di-tert-butyl, 4-methoxybenzenesulfon ate | | 100 | 7 | 299.4 | -0.95 | 15 |
| I-2 | 4-(Tert-butylcarbonyloxy) benz enesulfonate | | 100 | 3 | 257.3 | 0.16 | 11 |
| I-3 | 4-((4-(Tert-butyl) phenyl) sulfonyl)-benzenesulfonate) | | 100 | 3 | 353.4 | A:0.39<br>B:1.00 | 16 |
| I-4 | 4-(2,3,3-Trimethylbutan-2-yl)benzenesulfonate | | 100 | 5 | 255.4 | 0.09 | 13 |
| I-5 | 2-(4-(Tert-butyl)phenoxy)-2-methylpropanate | | 100 | 5 | 235.3 | -1.04 | 14 |
| I-6 | 4-((4-(tert-butyl)phenyl thio)benzenesulfonate | | 100 | 3 | 321.4 | A:-0.81<br>B:-0.20 | 16 |
| I-7 | 4,4'-(2,3-Dimethylbutane-2,3-diyl)dibenzene sulfonate | | 100 | 4 | 396.5 | A:0.09<br>B:0.09 | 18 |
| I-8 | 3,5-Di-tert-butylbenzenesulfonate | | 100 | 6 | 269.4 | -0.17 | 14 |
| I-9 | 4-(1,1,3,3-Tetramethylbutyl)-benzenesulfonate | | 83.3 | 5 | 269.4 | 0.09 | 14 |
| I-10 | N-[[4-(1,1-Dimethylethyl) phenyl] sulfonyl]-2-methylalanine | | 100 | 5 | 298.4 | 0.31 | 14 |
| I-11 | 4-((4-(Tert-butyl)phenyl) sulfinyl) benzenesulfonate) | | 100 | 3 | 337.4 | A:0.21<br>B:0.82 | 16 |
| I-12 | 4-(Neopentylsulfinyl) Benzenesulfonate | | 75 | 3 | 275.4 | 0.86 | 11 |

(continued)

**Table A - Hydrocarbon Dispersing Agents**

| Dispersing Agent No. | Name | Structure | $\%CH_3$ Based on total $CH_3$, $CH_2$, and CH groups | Number of $CH_3$ Groups | MW (Without counter ion) | Hammett Sigma Plus Value of Aromatic Groups | Carbon Atoms |
|---|---|---|---|---|---|---|---|
| I-13 | 4-(Neopentylsulfonyl) benzenesulfonate | | 75 | 3 | 291.4 | 1.07 | 11 |
| I-14 | Hexamethylglutarate | | 100 | 6 | 214.3 | N/A | 11 |
| I-15 | Tetramethylsuccinate | | 100 | 4 | 172.2 | N/A | 8 |

**As determined by NMR*

## SYNTHESIS EXAMPLES

The synthesis examples which follow include examples of dispersing agents for use in accordance with the process of the present invention and certain comparative dispersing agents useful for comparisons in the polymerization examples that follow.

### Synthesis Example 1

### I-3 (Acid) - 4-((4-(*tert*-butyl)phenyl)sulfonyl)benzenesulfonic acid

4-t-Butyldiphenyl sulfide (TCI) (815 g, 3.36 mol, 1 equiv) is dissolved in DCM (dichloromethane, 1.6 L) under inert atmosphere. The solution is cooled to - 10°C, and chlorosulfonic acid (422 g, 3.62 mol, 1.08 equiv) is added dropwise. The reaction is stirred at ambient temperature for 18 hours. The solution is then sparged with $N_2$ for 30 minutes and transferred to glass bottles.

A reactor is charged with water (1.75 L) which is then heated to 75°C. A short-path distillation head is attached, and a portion of the sparged DCM solution of sulfonic acid intermediate (4-(4-(tert-butyl)phenylthio)-benzenesulfonic acid) (2.5 kg solution, 750 g sulfonic acid intermediate, 2.33 mol) is added slowly to control foaming as the DCM is distilled off. After complete addition of the DCM solution, the reactor is heated to 92°C for 15 minutes to ensure complete removal of DCM. The short-path distillation head is removed, and the reactor is cooled to ambient temperature.

A reflux condenser is attached along with an addition funnel charged with 30 wt% $H_2O_2$ aqueous solution (633 g, 5.58 mol, 2.4 equiv). The H2O2 solution is then added slowly, keeping the reaction temperature between 25-30°C. The reaction mixture is then stirred at ambient temperature until NMR analysis confirms reaction completion.

The reactor is then emptied to a large flask and recharged with platinum black (0.175 g, 0.9 mmol) and water (2 L). The suspension is heated to 75°C. The reactor contents from the previous step are charged to an addition funnel and added slowly to control foaming. Upon complete addition, the heat is slowly increased to 92°C, and the reaction stirred until no peroxide is detected by peroxide test strip. Additional water (4 L) is added, and the reaction is cooled to ambient temperature. The reaction mixture is filtered through a pad of diatomaceous earth to give 9.7 kg clear product solution. An aliquot is analyzed by quantitative NMR (DMSO internal standard, D2O solvent) which showed the solution to be 6.89 wt% product (81% yield) of 4-((4-(tert-butyl)phenyl)sulfonyl)benzenesulfonic acid. 1H NMR (D2O, 400 MHz): $\delta$ 1.12 (s, 9H), 7.51 (d, 2H, J = 8.5 Hz), 7.74-7.92 (m, 6H).

### Synthesis Example 2

### I-11 (Acid) - 4-((4-(tert-butyl)phenyl)sulfinyl)benzenesulfonic acid

4-t-Butyldiphenyl sulfide (TCI) (815 g, 3.36 mol, 1 equiv) is dissolved in DCM (1.6 L) under inert atmosphere. The solution is cooled to -10°C, and chlorosulfonic acid (422 g, 3.62 mol, 1.08 equiv) is added dropwise. The reaction mixture is stirred at ambient temperature for 18 hours. The solution is then sparged with N2 for 30 minutes and transferred to glass bottles.

A reactor is charged with water (2.25 L) which is then heated to 75°C. short-path distillation head is attached, and a portion of the sparged DCM solution of sulfonic acid intermediate (4-(4-(tert-butyl)phenylthio)-benzenesulfonic acid) (2.5 kg solution, 750 g sulfonic acid intermediate, 2.33 mol) is added slowly to control foaming as the DCM is distilled off. After complete addition of the DCM solution, the reactor is heated to 92°C for 15 minutes to ensure complete removal of DCM. The short-path distillation head is then removed, and the reactor is cooled to ambient temperature.

A reflux condenser is then attached along with an addition funnel charged with 30 wt% H2O2 aqueous solution (317 g, 2.79 mol, 1.2 equiv). The reactor and contents are cooled to 15°C. The H2O2 solution is added slowly, keeping the reaction temperature between 15-20°C. The reaction mixture is then stirred at 25°C until NMR analysis confirms reaction completion.

The reactor is then emptied to a large flask and recharged with platinum black (0.1 g, 0.5 mmol) and water (2 L). The reactor contents from the previous step are charged to an addition funnel and added slowly at ambient temperature to control foaming. Upon complete addition, the reaction mixture is then stirred at ambient temperature until no peroxide is detected by peroxide test strip. Additional water (3 L) is added, and the reaction mixture is filtered through a pad of diatomaceous earth to give 8.8 kg clear product solution. An aliquot is analyzed by quantitative NMR (DMSO internal standard, D2O solvent) which showed the solution to be 6.95 wt% product (77% yield) of 4-((4-(tert-butyl)phenyl)sulfinyl) benzenesulfonic acid. 1H NMR (D2O, 400 MHz): $\delta$ 0.99 (s, 9H), 7.30-7.75 (m, 8H).

### Synthesis Example 3

**I-13 (Acid) - 4-(neopentylsulfonyl)benzenesulfonic acid**

Neopentyl phenyl sulfide is prepared according to a method adopted from Org. Synth. 1978, 58, 143-146. Sodium thiophenolate (Sigma Aldrich) (47.8 g, 90% purity, 326 mmol, 1 equiv) and tributylhexadecylphosphonium bromide (Aldrich) (5.68 g, 11 mmol, 0.034 equiv) are dissolved in water (102 mL). Neopentyl bromide (Beantown Chemical) (54.9 g, 98% purity, 356 mmol, 1.1 equiv) is added, and the mixture is heated to 70°C for 4 hours and then cooled to ambient temperature overnight. The layers are separated, and the aqueous layer is extracted with diethyl ether. The organic layers are combined and washed consecutively with water and then with brine. The organic layers are dried over magnesium sulfate, concentrated under reduced pressure, and vacuum distilled at 0.5 torr and 55°C to give 64 g oil (99% yield) neopentyl phenyl sulfide.

Neopentyl phenyl sulfide (64 g, 355 mmol, 1 equiv) is dissolved in DCM (dichloromethane, 240 mL) and cooled to -20°C. Chlorosulfonic acid (43.4 g, 372 mmol, 1.05 equiv) is added dropwise. After addition, the reaction mixture is then stirred at -20°C for an additional 30 minutes and then heated to ambient temperature over 3 hours. The volatiles are removed under reduced pressure, and water (188 mL) is added to give 280 g of 33 wt% aqueous solution of 4-(neopentylthio)benzenesulfonic acid (assumed quantitative yield).

4-(Neopentylthio)benzenesulfonic acid 33 wt% aqueous solution (50 g, 63.4 mmol, 1 equiv) is diluted with water (100 mL) and cooled to 10°C. 30 wt% H2O2 aqueous solution (36 g, 317 mmol, 5 equiv) is added slowly over 1 hour, keeping the temperature between 10-12°C. The reaction mixture is then stirred at 10°C for 5 days. Additional H2O2 solution (36 g, 317 mmol, 5 equiv) is added, and the reaction mixture is heated to 50°C for 3 days at which point NMR analysis shows complete conversion. Platinum black (50 mg, 0.25 mmol) is mixed with water (200 g), and the reaction mixture is added to the platinum dispersion. The reaction mixture is then stirred at 50°C for 1 day and then at ambient temperature for 2 more days. The reaction mixture is then filtered through a pad of diatomaceous earth to give 440 g clear product solution. An aliquot is analyzed by quantitative NMR (DMSO internal standard, D2O solvent) which shows the solution to be 3.17 wt% product (75% yield) 4-(neopentylsulfonyl)benzenesulfonic acid. 1H NMR (D2O, 400 MHz): δ 1.05 (s, 9H), 3.33 (s, 2H), 7.96-8.01 (m, 4H).

**Synthesis Example 4**

**I-12 (Acid) - 4-(neopentylsulfinyl)benzenesulfonic acid**

4-(Neopentylthio)benzenesulfonic acid 33 wt% aqueous solution is prepared according to the method of Example 3. An amount of this solution (50 g, 63.4 mmol, 1 equiv) is diluted with water (50 mL) and cooled to 10°C. 30 wt% H2O2 aqueous solution (8.6 g, 76 mmol, 1.2 equiv) is diluted with water (15 mL) and added slowly, keeping the temperature between 10-15°C. The reaction mixture is then stirred at 10°C for 18 hours at which point NMR analysis shows complete conversion. Platinum black (0.1 g, 0.5 mmol) is mixed with water (100 g), and the reaction mixture is added to the platinum dispersion. The reaction mixture is stirred at ambient temperature for 1 day, and then is filtered through a pad of diatomaceous each to give 300 g of clear product solution. An aliquot is analyzed by quantitative NMR (DMSO internal standard, D2O solvent) which shows the solution to be 4.56 wt% product (78% yield) 4-(neopentylsulfinyl)benzenesulfonic acid. 1H NMR (D2O, 400 MHz): δ 1.09 (s, 9H), 2.86 (dd, 2H, J = 14.2 Hz, 82.2 Hz), 7.73 (d, 2H, J = 7.8 Hz), 7.92 (d, 2H, J = 7.8 Hz).

**Synthesis Example 5**

**I-5 (Acid) - 2-(4-(tert-butyl)phenoxy)-2-methylpropanoic acid**

Ethyl 2-(4-(tert-butyl)phenoxy)-2-methylpropanoate is prepared according to a method adopted from Org. Process Res. Dev. 2007, 11, 431-440. Sodium ethoxide solution (21 wt% in EtOH, 59 g, 182 mmol, 1.1 equiv) is dissolved in ethyl acetate (30 mL) and heated to reflux for 2 hours. 4-Tert-butylphenol (Aldrich, 25.3 g, 168 mmol, 1 equiv) is added, and the reaction mixture is heated at reflux for 30 minutes. Ethyl 2-bromoisobutyrate (Sigma Aldrich) (97 g, 498 mmol, 3 equiv) is then added, and the reaction mixture is heated at reflux for 2 hours. GC analysis shows incomplete conversion of the starting phenol, so additional 21 wt% sodium ethoxide solution (35 g, 108 mmol, 0.65 equiv) is added, and heating at reflux is continued an additional 30 minutes at which time GC analysis shows complete conversion of the starting phenol. Heating is then stopped, and the reaction mixture is diluted with water. The layers are separated, and the aqueous layer is extracted with ethyl acetate. The combined organic layers are washed with brine, dried over magnesium sulfate, and concentrated under reduced pressure. Product is then isolated by vacuum distillation at 5 torr and 40-45°C to give 41 g brown oil (92% yield) ethyl 2-(4-(tert-butyl)phenoxy)-2-methylpropanoate.

Ethyl 2-(4-(tert-butyl)phenoxy)-2-methylpropanoate (41 g, 156 mmol, 1 equiv) is dissolved in a mixture of THF (500 mL) and water (60 mL). Sodium hydroxide powder (12.9 g, 322 mmol, 2 equiv) is added, and the reaction mixture is

heated to 60°C for 16 hours. The reaction mixture is cooled to ambient temperature and acidified to pH of about 2 with concentrated hydrochloric acid solution. The reaction mixture is diluted with water, and the layers are separated. The aqueous layer is extracted with diethyl ether, and the combined organic layers are washed with brine, dried over magnesium sulfate, and concentrated under reduced pressure. The resulting brown solids show trace methacrylic acid which is removed azeotropically with a mixture of water, THF, and heptane. Drying under high vacuum provides the product as 33 g of brown solid (91% yield) 2-(4-(tert-butyl)phenoxy)-2-methylpropanoic acid. 1H NMR (D2O, 400 MHz): δ 1.25 (s, 9H), 1.49 (s, 6H), 6.75 (d, 2H, J = 8.8 Hz), 7.27 (d, 2H, J = 8.8 Hz), 12.95 (s, 1H).

**Synthesis Example 6**

**C-9 ($Na^+$ Salt) - Sodium 3-(diphenylphosphoryl)benzenesulfonate**

Sodium 3-(diphenylphosphoryl)benzenesulfonate is prepared according to a method adopted from New J. Chem. 2003, 27, 1603-1608. Sodium 3-(diphenylphosphaneyl)benzenesulfonate (Sigma-Aldrich) (14.9 g, 41 mmol, 1 equiv) is dissolved in water (600 mL). 30 wt% $H_2O_2$ aqueous solution (4.65 g, 41 mmol, 1 equiv) is added, and the reaction stirred at ambient temperature for 18 hours. The reaction mixture is concentrated under reduced pressure to 99 g. An aliquot is analyzed by quantitative NMR (DMAC internal standard, D2O solvent) which shows the solution to be 15 wt% product (95% yield) sodium 3-(diphenylphosphoryl)benzenesulfonate. 1H NMR (D2O, 400 MHz): δ 7.5-7.8 (m, 12H), 8.0-8.1 (m, 2H).

**Synthesis Example 7**

**C-10 ($Na^+$ Salt) - Sodium**

**2-hydroxy-2,4,4-trimethylpentane-1-sulfonate**

2-Methyl-2-neopentyloxirane is prepared according to a method adopted from Tet. Lett. 1981, 22, 5023-5026. Diisobutylene (Sigma Aldrich) (34.3 g, 306 mmol, 1 equiv) is mixed with water (1.3 L) and sodium bicarbonate (46.3 g, 551 mmol, 1.8 equiv). m-Chloroperbenzoic acid (77% purity, 82.3 g, 367 mmol, 1.2 equiv) is added, and the mixture stirred vigorously for 2 days. The reaction mixture is extracted with diethyl ether which is then washed with cold 10 wt% sodium hydroxide solution. The organic phase is dried over magnesium sulfate and concentrated under reduced pressure to 25 g (64% yield) 2-methyl-2-neopentyloxirane.

Sodium 2-hydroxy-2,4,4-trimethylpentane-1-sulfonate is prepared according to a method adopted from J. Chem. Soc. 1954, 2180-2200. 2-Methyl-2-neopentyloxirane (25 g, 195 mmol, 1 equiv) is mixed with water (195 mL) and sodium sulfite (25 g, 195 mmol, 1 equiv) and heated at 145°C for 6 hours in a pressure vessel. The reaction mixture is filtered, and the solids are recrystallized from ethanol and dried to give 5.6 g white solid (12% yield) Sodium 2-hydroxy-2,4,4-trimethylpentane-1-sulfonate. 1H NMR (D2O, 400 MHz): δ 0.95 (s, 9H), 1.40 (s, 3H), 1.60-1.68 (m, 2H), 3.11 (s, 2H).

**Synthesis Example 8**

**1-10 (Acid) - N-[[4-(1,1-dimethylethyl)phenyl]sulfonyl]-2-methylalanine**

Alanine, N-[[4-(1,1-dimethylethyl)phenyl]sulfonyl]-2-methyl-, is synthesized by a method adapted from Tetrahedron (1960), 11, 39-51. A 500 ml flask with 3 necks, a thermowell, and a PTFE-coated magnetic stir bar is charged with 2-aminoisobutyric acid (TCI-US, 9.4 g, 91 mmol), 2.5 N sodium hydroxide (36 mL, 90 mmol), and acetone (36 mL). Buret A is charged with 50 mL of a solution comprising 4-tert-butylbenzenesulfonyl chloride (Oakwood Chemical, 23.623 g, 98.8 mmol) dissolved in 24.3 grams of acetone. Buret B is charged with 2.5 N sodium hydroxide (50 mL, 125 mmol). The flask is cooled to 0°C, then the solutions in Burets A and B are added simultaneously in 1 mL increments over a period of 2 hours so as to maintain the internal temperature of the flask between 0 and 5°C. The solution is then warmed to room temperature and stirred 18 hours. The contents of the flask are transferred to a 1-neck flask and 53 g of acetone is removed on a rotary evaporator. The flask is cooled to 15°C and 10% HCl solution is added dropwise with stirring until the pH of the solution is 2.16. The slurry is filtered through Whatman #1 filter paper. The solid contains tBuC6H4SO3Na side product, which is removed by washing twice with 250 mL of H2O. The white solid is dried 18 hours in a 50°C vacuum oven to afford 10.0 g (33.4 mmol, 37% yield) of alanine, N-[[4-(1,1-dimethylethyl)phenyl]sulfonyl]-2-methyl-, product in acid form, mp 181°C. 1H NMR (DMSO-d6): δ 12.5 (br s, 1H), 7.89 (s, 1H), 7.73 (d, 8.5 Hz, 2H), 7.57 (d, 8.5 Hz, 2H), 1.30 (s, 9H), 1.26 (s, 6H).

**Synthesis Example 9**

**1-9 (Acid) - 4-(1,1,3,3-tetramethylbutyl)benzenesulfonic acid**

Benzene, (1,1,3,3-tetramethylbutyl)- is synthesized by a method adapted from Tetrahedron Letters (2017), 58(24), 2340-2343. A 1 L pressure vessel is charged with 4-(1,1,3,3-tetramethylbutyl) phenol (TCI, 69.2 g, 335 mmol), 500 mL anhydrous DMSO, and triethylamine (40 g, 395 mmol). The vessel is cooled and the air is evacuated, then sulfuryl fluoride (Synquest, 38 g, 372 mmol) is condensed into the vessel. The vessel is sealed and agitated at 25°C for 6 hours. The reaction is repeated at identical scale, then the two reaction masses are transferred to a 2L 3-neck flask equipped with a dropping funnel, nitrogen inlet, and PTFE-coated magnetic stir bar. A gas-dispersing sparging tube is used to sparge the solution with nitrogen for 15 minutes at ambient temperature. Palladium(II) Acetate (Alfa-Aesar, 0.5905g, 2.630 mmol), 1,3-Bis(diphenylphosphino)propane (TCI, 6.78 g, 16.4 mmol), then triethylamine (275 g, 2.72 mol) are charged to the flask. Next formic acid (TCI, 141 g of 88% solution, 2.70 mol) is added dropwise through the dropping funnel over a period of 2.25 hours and the exothermic reaction reaches 53°C. An ice bath is used to control the exotherm 30 minutes after the formic acid addition is started, then removed. The reaction mixture is next stirred 18 hours at room temperature. The reaction mass is extracted with diethyl ether 2 x 250 mL, then the ether extracts are combined and washed with 1L H2O, 600 mL 0.5 N NaOH, 500 mL H2O, 600 mL 0.5 N HCl, 500 mL H2O, then 250 mL brine. The ether solution is dried over magnesium sulfate, filtered, and concentrated with a rotary evaporator. The crude product is distilled through a short path distillation head, bp 107-112°C / 15 mm Hg, to afford 110.1 g (0.578 mol, 86% yield) clear liquid benzene, (1,1,3,3-tetramethylbutyl)-. 1H NMR (400 MHz, CDCl3) δ 7.42 (m, 2H), 7.31 (m, 2H), 7.19 (m, 1H), 1.79 (s, 2H), 1.41 (s, 6H), 0.76 (s, 9H).

Benzene, (1,1,3,3-tetramethylbutyl)- (8.375 g, 0.044 mol) is dissolved in 40 mL dichloromethane under an inert atmosphere. The solution is cooled to -20°C, and chlorosulfonic acid (5.412 g, 0.0464 mol) is added dropwise over a 15 -minute time period then the solution is allowed to warm to room temperature and stirred for 4 hours. Dichloromethane is removed in vacuo, yielding 11.8 g (99%) of benzenesulfonic acid, 4-(1,1,3,3-tetramethylbutyl)- as a viscous, yellow oil. 1H NMR (400 MHz, D2O, reference to DHO/sulfonic acid signal) δ 7.64 (d, 8.4 Hz, 2Hz), 7.39 (d, J=8 Hz, 2H), 1.62 (s, 2H), 1.22 (s, 6H), 0.61 (s, 9H).

**Synthesis Example 10**

**I-14 (Acid) - Hexamethylglurtaric Acid**

**(2,2,3,3,4,4-Hexamethyl-pentanedioic acid)**

Cumyl chloride is prepared by adapting the method reported in Journal of Organic Chemistry (1966), 31(4), 1090-3. A jacketed 500 mL flask is equipped with a stir bar, sub-surface gas inlet tube, thermocouple, and a dry ice condenser vented to a caustic scrubber. The flask is charged with alpha-methyl styrene (61g, 0.51 mol) and 300 mL dry dichloromethane. The flask is cooled to 0°C then anhydrous HCl (22.5 g, 0.62 mol) is bubbled through the reaction mass for a period of 90 minutes. Next nitrogen is bubbled through the flask for 30 minutes to remove unreacted HCl. The complete conversion of alpha-methyl styrene to cumyl chloride is confirmed by GCMS. The cumyl chloride solution in dichloromethane is used in the next step without purification.

1,1,2,2,3,3-Hexamethylindane is prepared according to the method reported in Journal of Organic Chemistry (1988), 53(19), 4626-8. A 2L reactor attached to a nitrogen tee and equipped with a dropping funnel is charged with cumyl chloride (0.55 mol) in dichloromethane and diluted with dichloromethane to a total volume of 1.25 L. 2,3-Dimethyl-2-butene (58.4 g, 0.69 mol) is added and the mixture is cooled to -78°C. Titanium tetrachloride (17.4 g, 0.092 mol) is added over a 3-hour period and then the mixture is stirred an additional 30 minutes. The cold mixture is quenched by adding to a stirred mixture of 250 mL concentrated HCl and 500 mL H2O. The organic layer is separated and dried over MgSO4, then the dichloromethane is removed on a rotary evaporator and the crude oil is distilled to afford 34.8 g (0.172 mol, 31% Yield) 1,1,2,2,3,3-hexamethylindane, bp 52-55°C / 0.4 mm Hg in 96.4% GC purity.

Hexamethylglutaric acid is prepared by adapting the procedures reported in Journal of Organic Chemistry (1988), 53(19), 4626-8 and Journal of Organic Chemistry (1990), 55(6), 1928-32. A 2L reactor is charged with 1,1,2,2,3,3-Hexamethylindane (22.1 g, 0.109 mol), 221 mL carbon tetrachloride, 221 mL acetonitrile, 332 mL H2O, and periodic acid (354 g, 1.55 mol). The biphasic mixture is set to stir then ruthenium trichloride hydrate (0.5475 g, 2.19 mmol) is added and the mixture is stirred 4 hours at 30-40°C. The reaction mass is cooled to 0°C then 266 mL diethyl ether is added with mixing. The organic layer is separated, then the aqueous layer is washed with ether (3 x 100 mL). The organic layers are combined and washed with 180 mL 5% NaHCO3 then 180 mL brine, then dried over MgSO4. The solvents are removed by rotary evaporator to afford 16.03 g crude hexamethylglutaric anhydride which is charged to a 500 mL flask with 130 mL 40% aqueous sodium hydroxide solution and 130 mL 2-propanol, then stirred 14 hours at room temperature. 145 mL H2O is added and mixed for 10 minutes, then the mixture is extracted with diethyl ether 3 x 145 mL. The water layer is mixed with charcoal and filtered. The filter cake is washed with 30 mL of 1% sodium hydroxide. The aqueous filtrate is treated with 170

mL con. HCl, cooled in an ice bath, then filtered to afford hexamethylglutaric acid (4.57, 0.0211 mol, 19% Yield). 1H NMR (400 MHz, DMSO-d6) δ 1.14 (s, 12 H), 1.10 (s, 6H).

**Synthesis Example 11**

**I-14 ($NH_4^+$ Salt) - Hexamethylglurtaric Acid, ammonium salt**

**(2,2,3,3,4,4-hexamethyl-pentanedioic acid, ammonium salt (1:2)**

A jacketed 500 mL flask is equipped with a stir bar, sub-surface gas inlet tube, thermocouple, and a dry ice condenser vented to an acid scrubber. The flask is charged with hexamethylglutaric acid (2.008 g, 9.28 mmol) and 120 mL anhydrous diethyl ether and stirred 72 h at room temperature. An additional 10 mL of diethyl ether is added, the reactor is chilled to 0°C, then anhydrous $NH_3$ gas (1.2 g, 67 mmol) is bubbled through the reaction mass over a 30-minute time period. The diethyl ether and excess NH3 are removed in vacuo at room temperature to afford hexamethylglutaric acid diammonium salt (1.958 g, 7.82 mmol, 84% Yield). 1H NMR (400 MHz, DMSO-d6) δ 1.06 (s, 12H), 0.93 (s, 6H).

**Synthesis Example 12**

**I-4 ($Na^+$ Salt) - Sodium 4-(2,3,3-Trimethylbutan-2-yl)benzenesulfonate**

2,3,3-Trimethylbutan-2-ol is prepared by the method described in Journal of Organic Chemistry (1966), 31(1), 137-42. 2,3,3-Trimethylbutan-2-ol (50.2 g, 0.432 mol) is added dropwise to 370 mL con. hydrochloric acid and stirred for 30 minutes at ambient temperature. The solution is filtered and washed with cold water, yielding 58.7 g (0.438 mol, >100% Yield) of 2-chloro-2,3,3-trimethylbutane as a slightly wet, fluffy, white solid.

2-Chloro-2,3,3-trimethylbutane (57.6 g, 0.430 mol) is dissolved in benzene (219 g, 2.80 mol) and dried over CaCl2 for 24 hours. The solution is filtered into a round bottom flask and 250 mL chlorobenzene is added. The solution is cooled to -20°C, then aluminum chloride (5.81 g, 0.0436 mol) is added slowly and the solution is stirred for 10 minutes. Cold brine (50 mL) is added to the solution and stirred for 15 minutes. The solution is transferred to a separatory funnel and washed with water (3 x 50 mL), saturated sodium bicarbonate solution (50 mL), water (3 x 50 mL) and brine (50 mL). The organic layer is dried over CaCl2 and then the excess solvent is removed on a rotary evaporator, yielding 55.1 g (0.313 mol, 73% Yield) of (2,3,3-trimethylbutan-2-yl)benzene as a pale, yellow oil.

(2,3,3-Trimethylbutan-2-yl)benzene (54.9 g, 0.311 mol) is dissolved in 275 mL dichloromethane. The solution is cooled to -20°C, then chlorosulfonic acid (38.4 g, 0.330 mol) is added dropwise over a 15-minute time period. The solution is stirred at -20°C for 4 hours. Dichloromethane is removed in vacuo. The resulting solids are slurried in water and neutralized to a pH of 8 with aqueous NaOH. The solution is chilled to 0°C, then the resultant crystals are filtered and washed with cold water. The solids are dried in a vacuum oven at 50°C for 24-hr to afford 57.2 g (0.206 mol, 66% yield) of sodium 4-(2,3,3-trimethylbutan-2-yl)benzenesulfonate as a white solid. 1H NMR (400 MHz, DMSO-d6): δ 7.46 (d, J=8.4 Hz, 2H), 7.23 (d, J=8.4 Hz, 2H), 1.22 (s, 6H), 0.72 (s, 9H).

**Synthesis Example 13**

**C-4 ($Na^+$ Salt) - Sodium 4-tert-butylbenzenesulfonate**

Tert-Butylbenzene (50 g, 0.373 mole) is chilled to 0°C then sulfuric acid (91.5 g, 0.91 mol) is added dropwise. The solution is then heated to 80°C and stirred for 1 hour. The solution is poured into 375 mL water, then sodium bicarbonate (30 g, 0.357 mol) and sodium chloride (37.5 g, 0.64 mol) are added sequentially. The solution is cooled to 0°C for 1 hour, filtered and the solids are washed with cold water. The solids are dried in a vacuum oven 100°C for 18 hours, yielding 68 g (0.288 mol, 77% yield) of sodium 4-tert-butylbenzenesulfonate as a white, flaky solid. 1H NMR (400 MHz, DMSO-d6): δ 7.56 (d, J=8.5 Hz, 2H), 7.36 (d, J=8.5 Hz, 2H), 1.27 (s, 9H).

**Synthesis Example 14**

**C-8 ($Na^+$ Salt) - Sodium 4-(Phenylsulfonyl)benzenesulfonate**

Diphenyl sulfide (101 g, 0.542 mol) is dissolved in 250 mL dichloromethane. The solution is cooled to 0°C and chlorosulfonic acid (71.1 g, 0.610 mol, 1.13 eqv) is added dropwise via addition funnel over a 2-hour time period. The reaction mixture is allowed to warm to room temperature then stirred for 18 hours. Water (250 mL) is added to the reactor. A distillation head is affixed to the reactor, and the solution is heated to 40°C for 1 hour, and then 90°C for 30 minutes to

remove all traces of dichloromethane. The solution is cooled to ambient temperature. The distillation head is replaced with a reflux condenser and P25 titanium dioxide (4.32 g, 0.054 mol) is added to the reactor. The solution is cooled to 0°C, and 30% hydrogen peroxide (148 g, 1.3 mol) is added dropwise via addition funnel at a rate to keep the reaction temperature below 20°C. Next, the solution is heated to 80°C and stirred for 18 hours. The solution is cooled to ambient temperature then filtered over a pad of diatomaceous earth. The filtrate is cooled to 0°C and neutralized to a pH of 8 with aqueous 30% NaOH. The solution is filtered and the solids are washed with cold water. The solids are dried in a vacuum oven 100°C for 24 hours to yield 130 g (0.406 mol, 75% yield) of sodium 4-(phenylsulfonyl)benzenesulfonate as a fine, off-white powder. 1H NMR (400 MHz, DMSO-d6): δ 7.96 (d, 8.2 Hz, 4H), 7.84 (d, 8.2 Hz, 2H), 7.67-7.74 (m, 1H), 7.60-7.67 (m, 2H).

**Synthesis Example 15**

**I-7 (Acid) - 4,4'-(2,3-Dimethylbutane-2,3-diyl)dibenzenesulfonic acid**

Dicumene (20.2 g, 0.0847 mol) is dissolved in 100 mL dichloromethane under an inert atmosphere. The solution is cooled to -20°C, and chlorosulfonic acid (20.2 g, 0.173 mol) is added dropwise over a 1-hour time period. The reaction is allowed to warm to room temperature and stirred for 2 hours. The solution is filtered and the solids washed with cold dichloromethane. The solids are dried at ambient temperature under a stream of nitrogen for 24 hours, yielding 29 g (0.073 mol, 86% yield) of 4,4'-(2,3-dimethylbutane-2,3-diyl)dibenzenesulfonic acid as a fine, white powder. 1H NMR (400 MHz, DMSO-d6): δ 7.42 (d, 8.1 Hz, 4H) 7.14 (d, , 8.1 Hz, 4H), 1.21 (s, 12H).

**Synthesis Example 16**

**I-1 (Acid) - 3,5-bis(1,1-dimethylethyl)-4-methoxybenzenesulfonic Acid**

3,5-di-tert-Butyl-4-methoxybenzene (50.2 g, 0.228 mol) is dissolved in 150 mL dichloromethane under an inert atmosphere. The solution is cooled to - 20°C, and chlorosulfonic acid (27.4 g, 0.235 mol) is added dropwise over a 1-hour time period. The reaction is warmed to room temperature and stirred 2 hours. Water (6.3 mL) is added to the solution. The solution is filtered and the solids washed with cold dichloromethane. The solids are dried at ambient temperature under a stream of nitrogen for 24 hours, yielding 64.4 g (0.214 mol, 94% Yield) of benzenesulfonic acid, 3,5-bis(1,1-dimethylethyl)-4-methoxy-, as a fine, white powder. 1H NMR (400 MHz, DMSO-d6): δ 7.53 (s, 2H), 3.63 (s, 3H), 1.37 (s, 18H).

**Synthesis Example 17**

**I-1 ($NH4^+$ Salt) - 3,5-bis(1,1-dimethylethyl)-4-methoxybenzenesulfonic acid, ammonium salt (1:1)**

A 250 mL flask with a magnetic stir bar is charged with benzenesulfonic acid, 3,5-bis(1,1-dimethylethyl)-4-methoxy-, (19.7 g, 65.6 mmol) and 67 g H2O. A pH probe is placed in the solution and concentrated ammonium hydroxide solution is added dropwise until the pH is stable at a pH between 8 and 9. The H2O is removed by freeze-drying under a vacuum of 0.2 mm Hg to afford a white solid and the active content is determined to be 97.14% by an Ohaus MB45 moisture analyzer at 175°C. The sample analyzed at 175°C is dissolved in H2O and shows neutral pH, indicating the ammonium salt is formed. 19.1 g (58.4 mmol, 89% yield) benzenesulfonic acid, 3,5-bis(1,1-dimethylethyl)-4-methoxy-, ammonium salt (1:1) is isolated. 1H NMR (400 MHz, DMSO-d6): δ 7.50 (s, 2H), 7.13 (br S, 4H), 3.63 (s, 3H), 1.37 (s, 18H).

**Synthesis Example 18**

**C-11 (Acid) - 3,5-Diisopropylbenzenesulfonic acid**

1,3-Diisopropylbenzene (50.0 g, 0.308 g) is dissolved in 150 mL dichloromethane. The solution is cooled to -20°C, and chlorosulfonic acid (38.3 g, 0.329 mol) is added dropwise over a hour time period. The reaction is warmed to room temperature and stirred 4 hours. Water (9 g) is added to the solution. The solution is filtered and the resulting solids washed with cold dichloromethane. The solids are dried at ambient temperature under a stream of nitrogen 24 hours, yielding 69 g (0.285 mol, 93% yield) of 3,5-diisopropylbenzenesulfonic acid as a fine, white powder. 1H NMR (400 MHz, DMSO-d6): δ 7.67 (d, 8.1 Hz, 1H), 7.19 (s, 1H), 6.92 (d, 8.1 Hz, 1H), 4.01-4.09 (m, 1H), 2.81-2.89 (m, 1H),1.13-1.19 (m, 12H).

**Synthesis Example 19**

**1-8 (Acid) - 3,5-Di-tert-butylbenzenesulfonic acid**

A jacketed 500 mL flask is equipped with a stir bar, sub-surface nitrogen inlet, thermocouple, dropping funnel, and a dry ice condenser vented to a caustic scrubber. Check valves are installed before the gas inlet tube and scrubber. The flask is charged with 1,3,5-tri-tert-butyl benzene (TCI, 25.09 g, 0.102 mol) and 125 mL dry dichloromethane. The solution is chilled to -10°C, then chlorosulfonic acid (12.3g, 0.105 mol) is added dropwise over a 15-minute period. The mixture is stirred 4 hr while bubbling nitrogen through the reaction mass, then water (0.15g, 8.3 mmol) is added and nitrogen is bubbled through the mixture for an additional 30 minutes. The mixture is filtered while cold through a polypropylene filter funnel with a 10 micron polyethylene fritted disc, and washed with 150 mL chilled (-40°C) dichloromethane. The solids are dried at 40°C and vacuum of 40 mm Hg to afford 15.2 g (0.056 mol, 55% yield) off-white crude solid 3,5-di-tert-butylbenzenesulfonic acid. The 3,5-di-tert-butylbenzenesulfonic acid is purified by recrystallization from dichloromethane. 1H NMR of 3,5-Di-tert-butylbenzenesulfonic acid (600 MHz, DMSO-d6): δ 7.47 (d, 2 Hz, 2H), 7.39 (t, 2Hz, 1H), 1.26 (s, 18H).

**Synthesis Example 20**

**I-8 ($NH_4^+$ Salt) - Ammonium 3,5-di-tert-butylbenzenesulfonate**

3,5-Di-tert-butylbenzenesulfonic acid (30.2 g, 111.9 mmol) is placed in a glass beaker and 30 mL of deionized water is added to make a slurry. The mixture is stirred until the slurry is homogeneous. Then, 28-30% ammonium hydroxide solution is added dropwise until pH = 8-9. The reaction mixture is stirred for 30 minutes and pH is re-checked. Then, white slurry is filtered using a glass filter. The solid on the filter is washed 4x25 mL of ice-cold deionized water and then dried overnight at 70-75°C under vacuum of 15 in Hg. Dried ammonium salt of 3,5-di-tert-butylbenzenesulfonic acid was washed with diethyl ether (3x30 mL) and obtained material was re-dried at 70-75°C under vacuum of 10-15 inHg for 2 hours. Ammonium 3,5-di-tert-butylbenzenesulfonate product obtained is 26.5 g (82.6% yield). The moisture content of the product is determined to be 0.13% utilizing Mettler Toledo HR 73 moisture analyzer. Water is evaporated, and the dry solid is washed with diethyl ether 3x10mL to give additional 3.8 g of product but of lower purity. 1H NMR (Methanol-d4, 500 MHz): δ 1.37 (s, 18H), 7.56 (t, 1H, J = 1.8 Hz), 7.76 (d, 2H, J = 1.8Hz).

**Synthesis Example 21**

**I-8 ($K^+$ Salt) - Potassium 3,5-di-tert-butylbenzenesulfonate**

3,5-Di-tert-butylbenzenesulfonic acid (36.6 g, 135.6 mmol) is placed in a glass beaker and 40 mL of deionized water is added to make a slurry. The mixture is stirred until the slurry is homogeneous. Then, 30% aqueous solution of potassium hydroxide (KOH content is minimum 85%) is added dropwise until pH=8-9. The reaction mixture is stirred for 30 minutes and pH is re-checked and found to be around 9-10. Then, the white slurry is filtered using a glass filter. The solid remaining on the filter is washed with ice-cold deionized water until the filtrate has pH of 6-7. The solid material obtained is dried for 3 days at 70-75°C under vacuum of 15 inHg. The obtained dry potassium salt of 3,5-di-tert-butylbenzenesulfonic acid is washed with diethyl ether (3x30 mL) and the obtained material is re-dried at 70-75°C under vacuum of 15 inHg for 3 days. Potassium 3,5-di-tert-butylbenzenesulfonate product obtained is 35.7 g (83.1% yield). The moisture content of the product was determined to be 0% utilizing a Mettler Toledo HR 73 moisture analyzer. The water fraction is evaporated, and the remaining dry solid is washed with diethyl ether 3x10mL to give another 3.6 g of crude product. 1H NMR (Methanol-d4, 500 MHz): δ 1.37 (s, 18H), 7.56 (brs, 1H), 7.76 (d, 2H, J = 1.8Hz).

**Synthesis Example 22**

**I-8 ($Na^+$ Salt) - Sodium 3,5-di-tert-butylbenzenesulfonate**

3,5-Di-tert-butylbenzenesulfonic acid (17.6 g, 65.2 mmol) is placed in a glass beaker and 20 mL of deionized water is added to make a slurry. The mixture is stirred until the slurry is homogeneous. Then, 18 mL of 30% aqueous solution of sodium hydroxide is added dropwise (pH is 9-10). Reaction mixture is stirred for 30 minutes and pH is re-checked. Then, the white slurry is filtered using a glass filter. The solid remaining on the filter is washed with ice-cold deionized water until the filtrate has pH of 6-7. The obtained solid material is dried overnight at 70-75°C under vacuum of 15 inHg. The obtained dry sodium salt of 3,5-di-tert-butylbenzenesulfonic acid is washed with diethyl ether (3x30 mL) and then re-dried at 70-75°C under vacuum of 15 inHg for 2 days. Sodium 3,5-di-tert-butylbenzenesulfonate product obtained is 35.7 g (90.2% yield). The moisture content is determined to be 2.3% utilizing a Mettler Toledo HR 73 moisture analyzer. The water fraction is evaporated, and the remaining dry solid is washed with diethyl ether 3x10mL to give 7.0 g of additional

crude product (moisture content was not determined). 1H NMR (Methanol-d4, 500 MHz): δ 1.37 (s, 18H), 7.56 (brs, 1H), 7.76 (brs, 2H).

**Synthesis Example 23**

**C-12 (Acid) - 3,5-Di-tert-butyl-4-hydroxybenzenesulfonic acid**

2,6-Di-tert-butylphenol (TCI) (50 g, 242.4 mmol) is placed in 3-necked round bottom flask equipped with addition funnel with gas outlet, thermometer, gas inlet and mechanical stirrer. The gas outlet is connected to a caustic scrubber. Then, 100mL of dichloromethane is added to the reactor and the agitator is set to 400 rpm. A nitrogen line is connected to the gas inlet and a small nitrogen stream is started. The reactor is cooled to -25 to -20°C and a mixture of 18mL of chlorosulfonic acid and 24 mL of anhydrous dioxane is added to the addition funnel. The dioxane/HSO3Cl solution is added to the stirred reaction mixture dropwise within 25 minutes. Then the reaction temperature is allowed to increase to -15 to -10°C and the reaction mixture is stirred for another 2 hours. Then, the reaction mixture is warmed to 0°C and 100mL of hexane is added to precipitate the 3,5-di-tert-butyl-4-hydroxybenzenesulfonic acid product. The isolated solid product is filtered from the reaction mixture and washed with hexane (3x50mL). The organic solution is subjected to evaporation on a rotary evaporator. Precipitation of additional product is observed after ~15-20% of solution is removed. The precipitated material is filtered and washed with hexane (3x40mL). All amounts of solid material is combined and dried at 70-75°C under vacuum of 10-15 inHg for 2 hours. 3,5-Di-tert-butyl-4-hydroxybenzenesulfonic acid product obtained is 60.8 g (88.0% yield). Formation of a second layer is observed in the organic solution. All solvent is then removed to give and additional 11.0.g of crude product. 1H NMR (Methanol-d4, 500 MHz): δ 1.46 (s, 18H), 7.71 (s, 2H).

**Synthesis Example 24**

**C-12 ($NH_4^+$ Salt) - Ammonium**

**3,5-di-tert-butyl-4-hydroxybenzenesulfonate**

3,5-Di-tert-butyl-4-hydroxybenzenesulfonic acid (20.3 g, 71 mmol) is placed in a glass beaker and 30 mL of deionized water is added to make a slurry. The mixture is stirred until the slurry is homogeneous. Then, 8 mL of 28-30% ammonium hydroxide solution is added dropwise (pH is 8-9). The reaction mixture is stirred for 30 minutes and the pH is re-checked. Then, the resultant white slurry is filtered using a glass filter. The solid remaining on the filter is washed with ice-cold deionized water until the filtrate pH is 6-7. The isolated solid product is dried for 2 hours at 70-75°C under vacuum of 15 inHg and is then left overnight at ambient pressure and room temperature. The dry ammonium salt of 3,5-di-tert-butyl-4-hydroxybenzenesulfonic acid is washed with diethyl ether (3x25 mL) and then re-dried at 70-75°C under vacuum of 15 inHg for 2 hours. Ammonium 3,5-di-tert-butyl-4-hydroxybenzenesulfonate product obtained is 18.4 g (85.6% yield). The moisture content of the product is determined to be ~0.2% utilizing a Mettler Toledo HR 73 moisture analyzer. The water filtrate is evaporated, and the remaining dry solid is washed with diethyl ether 2x10mL to give an additional 2.2 g of crude product (moisture content was not determined). 1H NMR (Methanol-d4, 500 MHz): δ 1.46 (s, 18H), 7.72 (s, 2H).

**POLYMERIZATION EXAMPLES**

**Comparative Examples 1-12 and Polymerization**

**Examples 1-13 Fluoroelastomer - Five Hour**

**Reaction Time with Low Dispersing Agent Concentration**

The following examples illustrate semi-batch emulsion polymerization processes for making VF2/HFP/TFE fluoroelastomer by pre-charging a low concentration of dispersing agent and feeding of monomer at a constant pressure over a five-hour reaction time. No nucleating agent is employed. Comparative Examples 1-12 illustrate processes employing comparative dispersing agents. Examples 1-13 illustrate processes in accordance with the invention.

VF2/HFP/TFE fluoroelastomer is prepared by a semi-batch emulsion polymerization process as follows. A 4.0-liter reactor is charged with a solution containing 2.0 grams of disodium phosphate, dispersing agent described in Table 1 (1-3 grams dependent on molecular weight) to provide the molar amounts indicated in Table 1 and deionized, deoxygenated water for a total of 2,500 grams of solution. The reactor is heated to 80°C, agitated at 700 rpm, and pressurized with a mixture of 25.0 % vinylidene fluoride, 73% hexafluoropropylene, and 2% tetrafluoroethylene to a pressure of 320 psi. To commence polymerization, 5.0 milliliters of 2.5% ammonium persulfate and 0.5% disodium phosphate heptahydrate is

added to the reactor. A mixture of 50% vinylidene fluoride, 30% hexafluoropropylene, and 20% tetrafluoroethylene is then set to feed the reactor in order to maintain a pressure of 320 psi. Additional initiator is fed to the reactor to continue the polymerization in increments of 0.0-0.4 milliliters every 30 minutes to attempt to achieve or maintain a monomer flow rate of 80 grams/hour. Total initiator is listed in Table 1. At the completion of the five-hour batch time all feeds to the reactor are halted. Results are reported in Table 1 including the kick-off time, total monomer fed during the 5-hour batch time as well as the particle concentration and coagulum.

Table 1

| Example | Dispersing Agent No. | Cation | Surfactant Amount (mmols) | APS (mmols) | Kick-off Time (hr) | Monomer Feed (g) | Particle # per $cm^3$ | Solids Wt% | Part. Size Dv(50) nm | Coagulum (g) |
|---|---|---|---|---|---|---|---|---|---|---|
| Comp. 1 | C-1 | $Na^+$ | 5.548 | 0.942 | 1.5 | 14.3 | 4.12E+12 | 0.66 | 120 | 0 |
| Comp. 2 | C-2 | $Na^+$ | 5.596 | 0.942 | 0 | 0 | - | - | - | - |
| Comp. 3 | C-3 | $Na^+$ | 5.56 | 0.942 | 2 | 13.4 | 1.05E+13 | 0.51 | 79.2 | 0 |
| Comp. 4 | C-4 | $Na^+$ | 5.59 | 0.767 | 0.25 | 380.9 | 1.41E+12 | 9.23 | 415 | 181.9 g Dry |
| Comp. 5 | C-5 | $Na^+$ | 5.55 | 0.942 | 0.9 | 278.4 | 3.03E+12 | 11.68 | 359 | 0 |
| Comp. 6 | C-6 | $Na^+$ | 5.6 | 0.942 | 0 | 0 | - | - | - | - |
| Comp. 7 | C-7 | $Na^+$ | 5.6 | 0.942 | 1 | 158.5 | 5.95E+12 | 7.05 | 237 | 0 |
| Comp. 8 | C-8 | $Na^+$ | 5.619 | 0.942 | 0.5 | 46.4 | 1.48E+13 | 2.7 | 121 | 0 |
| Comp. 9 | C-10 | $Na^+$ | 5.623 | 0.877 | 0.25 | 338.1 | 9.96E+11 | 11.25 | 485 | 82.7g Dry |
| Comp.10 | C-11 | $NH_4^+$ | 5.56 | 0.942 | 0.45 | 149.1 | 2.79E+12 | 6.76 | 298 | 0 |
| Comp. 11 | C-12 | $NH_4^+$ | 5.6 | 0.942 | 0.5 | 79.4 | 1.33E+13 | 3.93 | 148 | 0 |
| Comp. 12 | C-9 | $Na^+$ | 5.60 | 0.942 | 0.5 | 149.1 | 2.79E+12 | 8.51 | 206 | 0 |
| Ex. 1 | I-3 | $NH_4^+$ | 5.303 | 0.942 | 0.25 | 286.7 | 1.45E+13 | 11.81 | 210 | 0 |
| Ex. 2 | I-4 | $Na^+$ | 4.351 | 0.909 | 1 | 250.8 | 7.84E+12 | 10.79 | 249 | 0 |
| Ex. 3 | I-7 | $Na^+$ | 5.56 | 0.942 | 0.5 | 213 | 8.43E+12 | 9.72 | 234 | 0 |
| Ex. 4 | I-8 | $NH_4^+$ | 5.542 | 0.833 | 0.5 | 362 | 4.25E+12 | 14.50 | 340 | 0 |
| Ex. 5 | I-9 | $NH_4^+$ | 5.568 | 0.942 | 0.32 | 219.7 | 4.96E+12 | 9.71 | 279 | <0.01g |
| Ex. 6 | I-10 | $NH_4^+$ | 5.37 | 0.942 | 0.5 | 200.2 | 6.35E+12 | 9.13 | 252 | 0 |
| Ex. 7 | I-11 | $NH_4^+$ | 5.57 | 0.942 | 0.9 | 255.1 | 1.03E+13 | 10.9 | 228 | 0 |
| Ex. 8 | I-12 | $NH_4^+$ | 5.62 | 0.942 | 0.5 | 292.2 | 1.38E+12 | 11.61 | 451 | 16.6 g Dry |
| Ex. 9 | I-13 | $NH_4^+$ | 5.568 | 0.942 | 0.083 | 298.9 | 7.85E+12 | 12.14 | 258 | 10.5g Dry |
| Ex 10 | I-1 | $NH_4^+$ | 5.64 | 0.789 | 0.25 | 382.5 | 1.98E+12 | 15.36 | 447 | 0 |
| Ex 11 | I-14 | $NH_4^+$ | 5.56 | 0.942 | 0.5 | 236.2 | 7.45E+12 | 10.1 | 247 | 0 |
| Ex. 12 | I-15 | $H^+$ | 5.6 | 0.942 | 0.5 | 295.4 | 8.14E+11 | 11.73 | 535 | 14.7g dry |
| Ex. 13 | I-2 | $Na^+$ | 5.6 | 0.942 | 0.5 | 198.4 | 6.08E+12 | 8.95 | 254 | 0 |

The results of Examples 1-13 show that processes to produce approximately 10 wt% solids batches of fluoroelastomer in accordance with the invention using low reactivity hydrocarbon dispersing agents enable the polymerization of fluoroelastomer to kick-off in the presence of pre-charged dispersing agent. As illustrated by Comparative Examples 2 and 6, some of the comparative hydrocarbon dispersing agents, linear sodium dodecyl benzene sulfonate (C-2) ($Na^+$ salt) and naphthalene sulfonic acid-formaldehyde condensate (C-6), respectively, inhibit the reaction to the extent that no kick-off occurs during the five-hour reaction time. In general, kick-off times are longer for the comparative hydrocarbon surfactants than for processes in accordance with the invention. In addition, processes in accordance with the invention provide higher amounts of monomer feed with no coagulum or low levels of coagulum.

Except for Comparative Examples 4, 5, and 9, the Comparative Examples exhibit low monomer feed levels. Comparative Example 4 demonstrates the use of sodium tert-butylbenzene sulfonate (C-4) ($Na^+$ salt) that does not show inhibition of polymerization and exhibits a high monomer feed but the coagulum level is nearly equal to half of the monomer feed indicating that that this comparative dispersing agent is ineffective for stabilization of the fluoroelastomer dispersion. Comparative Example 9 illustrates the use of sodium 2-hydroxy-2,4,4-trimethylpentane-1-sulfonate (C-10) ($Na^+$ salt) that as a dispersing agent produce significant coagulum. Comparative Example 5 illustrates the use of dispersing agent sodium octyl sulfonate (C-5) ($Na^+$ salt) that does not inhibit monomer feed or produce high coagulum in the lower solids batches in this series of examples. However, the following Example 14 with its data plotted in the graph of FIG. 1 shows the effect of increased dispersing agent concentration. In Example 14, dispersing agent sodium octyl sulfonate (C-5) ($Na^+$ salt) shows a sharp decrease in monomer feed with increased concentration of dispersing agent whereas dispersing agents used in a process of the present invention do not cause a significant decrease in monomer uptake.

**Polymerization Example 14**

**Fluoroelastomer - Increased Dispersing Agent Concentration for Selected Comparative and Inventive Dispersing Agents**

The same procedure as in Comparative Examples 1-12 and Examples 1-13 is used except that selected dispersing agents are used at the increased concentrations as described in Table 2. Results are reported in Table 2 including the kick-off time, total monomer fed during the 5-hour batch time as well as the particle number, particle size, solids concentration and coagulum. FIG. 1 is graphical representation of the data showing the effect of increasing dispersing agent concentration on monomer uptake for the selected comparative and inventive dispersing agents.

Table 2

| Run. No. | Dispersing Agent No. | Cation | Surfactant Amount | | APS | Kick-off Time | Reaction Time | Monomer Feed | Particle # | Particle Size Dv(50) | Solids | Coagulum |
|---|---|---|---|---|---|---|---|---|---|---|---|---|
| | | | Mmol | Ppm | Mmol | hr | hr | 9 | $/cm^3$ | nm | Wt% | 9 |
| **1** | C-1 | $Na^+$ | 1.85 | 160 | 0.942 | 0.5 | 5 | 79.8 | 4.77E+12 | 207 | 3.88 | 0 |
| **2** | C-1 | $Na^+$ | 2.77 | 320 | 0.942 | 1.5 | 5 | 34.8 | 3.77E+12 | 175 | 1.87 | 0 |
| **3** | C-1 | $Na^+$ | 5.55 | 640 | 0.942 | 1.5 | 5 | 14.3 | 4.12E+12 | 120 | 0.66 | 0 |
| **4** | C-5 | $Na^+$ | 5.55 | 480 | 0.942 | 1 | 5 | 254.8 | 6.16E+12 | 273 | 11.14 | 0 |
| **5** | C-5 | $Na^+$ | 7.40 | 640 | 0.942 | 1 | 5 | 244.6 | 6.79E+12 | 260 | 10.69 | 0 |
| **6** | C-5 | $Na^+$ | 11.56 | 1000 | 0.920 | 1 | 5 | 218.7 | 9.77E+12 | 211 | 8.25 | 0 |
| **7** | C-5 | $Na^+$ | 23.12 | 2000 | 0.942 | 2.5 | 5 | 18.4 | 1.38E+13 | 89.6 | 0.92 | 0 |
| **8** | I-8 | $NH_4^+$ | 5.54 | 637 | 0.833 | 0.25 | 5 | 362 | 4.48E+15 | 340 | 14.5 | 0 |
| **9** | I-8 | $NH_4^+$ | 11.08 | 1274 | 0.833 | 0.25 | 5 | 367.6 | 4.80E+15 | 335 | 14.77 | 0 |
| **10** | I-8 | $NH_4^+$ | 22.17 | 2202 | 0.942 | 0.5 | 5 | 306.4 | 8.20E+12 | 261 | 1273 | 0 |
| **11** | I-1 | $NH_4^+$ | 5.64 | 718 | 0.789 | 0.25 | 5 | 382.5 | 1.98E+12 | 447 | 15.36 | 0 |
| **12** | I-1 | $NH_4^+$ | 11.28 | 1437 | 0.866 | 0.5 | 5 | 347.1 | 2.03E+13 | 200 | 14.15 | 0 |
| **13** | I-1 | $NH_4^+$ | 22.60 | 2874 | 0.942 | 0.5 | 5 | 258.4 | 3.42E+12 | 331 | 11.97 | 0 |

With reference Table 2 and FIG. 1, Polymerization Example 14 shows that the use of dispersing agents ammonium 3,5-di-tert-butylbenzenesulfonate (I-8) ($NH_4^+$ salt) and 3,5-di-tert-butyl, ammonium 4-methoxybenzenesulfonate (I-1) ($NH_4^+$ salt) do not cause a significant decrease in monomer feed rate (indicating little decrease in reaction rate) with increasing concentration of dispersing agents in a process employing pre-charged dispersing agent. As a result, final fluoroelastomer solids concentrations are high at all dispersing agent levels. In addition, kick-off times for these dispersing agents are short. The data show that levels of dispersing agent in the range of 1000-4000 ppm often used to achieve solids levels for commercial production can successfully be used in a process employing the low reactivity dispersing agents in accordance with present invention and that the dispersing agent can be pre-charged if desired. The comparative dispersing agents, on the other hand, show sharp decreases in monomer uptake with increasing concentrations of dispersing agent. Moreover, the dispersing agent sodium dodecyl sulfate (C-1) ($Na^+$ salt) shows poor monomer uptake even at low concentration. At somewhat higher concentrations, there is almost no monomer uptake with dispersing agent C1. With dispersing agent sodium octyl sulfonate (C-5) ($Na^+$ salt), monomer uptake is good at low concentration as also seen in Comparative Example 5 but use of dispersing agent C-5 shows a sharp decrease with increasing concentrations. Monomer uptake with dispersing agent C-5 is very low at 2000 ppm. In addition, kick-off times for dispersing agent C-5 are longer at all concentrations compared to dispersing agents, I-8 and I-1.

**Polymerization Example 15**

**High Solids Fluoroelastomer I-3 (Acid) Dispersing Agent**

The following example illustrates a high solids, semi-batch emulsion polymerization process for making VF2/HFP/TFE fluoroelastomer by pre-charging the dispersing agent 4-((4-(tert-butyl)phenyl)sulfonyl)benzenesulfonic acid) (I-3) (employed as the acid). The process employs the use of perfluorodiiodoalkane compounds to provide iodine cure sites as end groups as well employing an iodine-containing cure site monomer.

The fluoroelastomer is prepared by a semi-batch emulsion polymerization process, carried out at 80°C in a 40-liter, well-stirred reaction vessel. A solution of 23.7 g disodium phosphate heptahydrate in 24 liters deionized, deoxygenated water is charged to the reactor. A solution of 393 g of 6.89 % I-3 dispersing agent (as the acid) in water is pumped into the reactor, followed by pumping 1 liter of deionized, deoxygenated water to provide 0.11 wt% of the I-3 dispersing agent in the water in the reactor. The reactor is heated to 80°C. After removal of trace oxygen, the reactor is pressurized with a mixture of 4 wt% vinylidene fluoride (VF2), 86 wt% hexafluoropropylene (HFP), and 10 wt% tetrafluoroethylene (TFE). At the end of pressurization, the reactor pressure is 2.2 MPa. The reactor is charged with 174 ml of an initiator solution of 1% ammonium persulfate and 7.5% disodium phosphate heptahydrate to start polymerization. As the reactor pressure drops, a mixture of 35 wt% VF2, 37 wt% HFP, and 28 wt% TFE is fed to the reactor to maintain a 2.2 MPa pressure. After 45 g of this monomer mixture is fed, 24.2 g of a mixture of 72.0 mol % 1,4-diiodoperfluorobutane, 22.7 mol % 1,6-diiodoperfluorohexane, 4.0 mol % 1,8-diiodoperfluorooctane, and 1.2 mol % 1,10-diiodoperfluorodecane is charged to the reactor. Additional initiator solution is added to maintain polymerization rate. After 2922 g of the monomer mixture is added, 4-iodo-3,3,4,4-tetrafluorobutene-1 (ITFB) is introduced to the reactor at a feed rate of 4.83 g ITFB per 1000 g monomer. After a total of 8333 g incremental major monomer is fed, corresponding to a total of 319 ml initiator solution, 20.4 g ITFB and 13.2 hours, monomer and initiator fed is discontinued. The reactor is cooled and the pressure in the reactor reduced to atmospheric. The resulting fluoroelastomer latex has a solids content of 24.6 wt% solids, and a pH of 3.3. The latex is coagulated with aluminum sulfate solution, washed with deionized water, and dried. The fluoroelastomer has an inherent viscosity of 0.53 dl/g, a Mooney viscosity at 121°C, ML (1 + 10), of 68 and contains 37.3 wt% VF2, 36.6 wt% HFP, 25.9 wt% TFE and 0.23 wt%. The polymer is compounded on a roll mill with the formulation by parts by weight in Table 3 below:

**Table 3**

| | **Parts by weight** |
|---|---|
| Fluoroelastomer | 100 |
| Medium Thermal Carbon Black | 30 |
| Zinc Oxide | 3 |
| 2,5-Bis(tert-butylperoxy)-2,5-dimethylhexane, blend with calcium carbonate and silica sold as Luperox® 101 XL 45 crosslinking peroxide by Arkema | 1.5 |
| Triallyl isocyanurate containing a small amount of a hydroquinone inhibitor for storage stability sold by The Chemours Company as Diak™ No. 7 coagent for peroxide curing | 2.2 |

Test articles are compression molded from the fluoroelastomer compound at 180°C/10 minutes and post-cured 230°C for 4 hours. A 25% compression set resistance is measured for ageing condition of 70 hours at 200°C. The tensile strength is 22 MPa and elongation at break is 318%.

Example 15 shows that a VF2/HFP/TFE fluoroelastomer can productively be produced by a process in accordance with the present invention employing a low reactivity hydrocarbon dispersing agent and that the fluoroelastomer has properties equivalent to fluoroelastomer produced using fluorosurfactant.

**Comparative Examples 13-19 and Polymerization Examples 16-22**

**PTFE Dispersion - 10 wt% Solids Batches**

**Polypropylene Glycol Nucleating Additive**

The following examples illustrate a semi-batch process for making aqueous PTFE dispersion by pre-charging the dispersing agent and feeding TFE in an amount to produce 10 weight % solids batches. Polypropylene glycol is employed as a nucleating additive. Comparative Examples 13-19 illustrate processes employing comparative dispersing agents. Examples 16-22 illustrate processes in accordance with the invention.

For the dispersing agents listed in Table 4, the following polymerization procedure is used unless otherwise indicated in Table 4.

To a 12 liter, horizontally disposed, jacketed, stainless steel autoclave with a two-blade agitator is added 4170 gm of deionized, deaerated water and 250 gm of paraffin wax. To the autoclave is added an additional 500 gm of deionized, deaerated water which contains 0.0297 gm of polypropylene glycol (PPG) having a molecular weight of approximate 450 g/mol. The autoclave is sealed and placed under vacuum. The autoclave pressure is raised to 30 psig (310kPa) with nitrogen and vented to atmospheric pressure. The autoclave is pressured with nitrogen and vented 2 more times. This pressure/vent cycle is repeated three times using tetrafluoroethylene (TFE). The reactor is heated to 60°C with no agitation. The agitator speed is set to 75 RPM after reactor temperature is at 60°C and continued heating the reactor to 90°C. To the autoclave is added 500 gm of deionized, deaerated water which contains 1.176 mMoles of the dispersing agents (DA) listed in Table 3 at 78 ml/min and followed with flush of 200 ml deionized, deaerated water.

TFE is next charged to the reactor to bring the reactor pressure to 400 psig (2.86MPa). 200 ml of an initiator solution composed of 10.0 gm of (70% active) disuccinic acid peroxide and 990.0 gm of deionized water is charged to the reactor at 80 ml/min. Kick-off Time is recorded and is shown in Table 4. After kick-off, autoclave pressure is brought back to 400 psig (2.86 MPa) with makeup TFE and maintained at that pressure for the duration of the polymerization by continuous addition of makeup TFE. At kick-off, 30 ml of solution composed of 0.5 gm of iron (II) sulfate heptahydrate in 499.5 gm of deionized water is charged to the reactor at 100 ml/min. After the prescribed amount of makeup TFE to produce 10 weight % solids, 700 gm, has been added since kick-off to the reactor, the agitator is stopped, this establishing the completion of the polymerization reaction. After the agitator is stopped, the reactor is vented to atmospheric pressure, is cooled to 80°C and then dispersion is discharged. Upon cooling of the dispersion, the solid wax is separated from the dispersion and the dispersion is filtered to remove undispersed solids. The reactor is opened and all adhered polymer removed from the reactor. Reactor cleanout is combined with the filtered solids and recorded as total wet coagulum. This total wet coagulum includes polymer, paraffin wax and water.

The polymer dispersion is coagulated by adding aqueous ammonium carbonate solution followed by vigorous agitation until the polymer fully separates from the water. The resultant polymer is dried in a vacuum oven at 110°C for 12 hours. Results are reported in Table 4 including Kick-off Time, Kick-off Rate, batch time, wt% solids, particle size, wet coagulum and melting point.

Table 4

| Example # | Dispersing Agent | | | | Kick-off Time | Kick-off Rate | Batch Time | Solids | Dispersion Amount | Particle Size | Wet Coagulum | Wet Coagulum* | DSC 1st Melt |
|---|---|---|---|---|---|---|---|---|---|---|---|---|---|
| | DA No. | Cation | Amount, gm | mMoles | Min | PSI/ min | min | % | gm | Dv(50) nm | gm | % | $^0C$ |
| Comp. 13 | C-1 | Na+ | 0.339 | 1.176 | No Kick-off (6 psi drop in 62.1 min) | 0.097 | | | | | | | |
| Comp. 14 | C-2 | Na+ | 0.410 | 1.177 | No Kick-off (4 psi drop in 62.1 min) | 0.067 | | | | | | | |
| Comp. 15 | C-3 | Na+ | 0.410 | 1.177 | No Kick-off (4 psi drop in 60.7 min) | 0.066 | | | | | | | |
| Comp. 16 | C-6 | Na+ | 1.401 | 1.177 | No Kick-off (4 psi drop in 60.7 min) | 0.066 | | | | | | | |
| Comp. 17 | C-4 | Na+ | 0.278 | 1.177 | 10.7 | 0.935 | 65.4 | 1.84 | 4808 | 209 | 911 | 18.95 | 334.6 |
| Comp. 18 | C-9 | Na+ | 0.448 | 1.177 | 46.6 | 0.215 | Aborted the batch @121 gm TFE fed since Kick-off as TFE feed rate dropped to 0 gm/hr | | | | | | |
| Comp. 19 | C-8 | Na+ | 0.377 | 1.177 | 13.6 | 0.735 | 136.7 | 0.73 | 4920 | 230 | 897 | 18.23 | 335.4 |
| Ex. 16 | I-4 | Na+ | 0.327 | 1.175 | 13.7 | 0.730 | 46.8 | 10.83 | 6205 | 183 | 32 | 0.52 | 335.5 |
| Ex. 17 | I-3 | $NH_4$+ | 0.437 | 1.176 | 15.0 | 0.667 | 95.9 | 11.00 | 6211 | 188 | 29 | 0.47 | 335.8 |
| Ex. 18 | I-8 | $NH_4$+ | 0.338 | 1.176 | 16.6 | 0.602 | 72.9 | 10.52 | 6291 | 181 | 32 | 0.51 | 336.3 |
| Ex. 19 | I-9 | $NH_4$+ | 0.338 | 1.176 | 16.2 | 0.679 | 67.0 | 10.93 | 6287 | 168 | 42 | 0.67 | 336.0 |
| Ex. 20 | I-11 | $NH_4$+ | 0.418 | 1.176 | 13.1 | 0.763 | 96.8 | 10.53 | 6301 | 177 | 55 | 0.87 | 334.9 |
| Ex. 21 | I-8 | Na+ | 0.344 | 1.177 | 16.4 | 0.610 | 82.9 | 11.11 | 6298 | 184 | 24 | 0.38 | 336.3 |
| Ex. 22 | I-1 | $NH_4$+ | 0.374 | 1.178 | 7.2 | 1.389 | 25.8 | 11.34 | 6271 | 184 | 50 | 0.8 | 335.6 |

**Based on dispersed polymer weight*

The results of Examples 16-22 show that processes in accordance with the invention using low reactivity hydrocarbon dispersing agents enable the polymerization of PTFE to kick-off in the presence of pre-charged dispersing agent and polymerize PTFE to 10% solids with low levels of coagulum. On the other hand, Comparative Examples 13-16 and Comparative Example 18 either did not kick-off after 60 minutes or stopped polymerizing shortly after kick-off. Comparative Example 17 employing sodium tert-butylbenzene sulfonate (C-4) ($Na^+$ salt) as dispersing agent did not stop polymerization from kicking off but the coagulum level is extremely high indicating that that this comparative dispersing agent is ineffective for stabilization of the PTFE dispersion, even at a low solids level. Comparative Example 19 employing as dispersing agent sodium 4-(phenylsulfonyl)-benzenesulfonate (C-8) ($Na^+$ salt) also enabled kick-off but again produced extremely high levels of coagulum indicating ineffective stabilization.

**Polymerization Example 23**

**PTFE Dispersion - 25-30 wt% Solids Batches with Increased Dispersing Agent Concentration for Selected Comparative and Inventive Dispersing Agents Polypropylene Glycol Nucleating Agent**

The following example illustrate a semi-batch process for making aqueous PTFE dispersion with selected comparative and inventive dispersing agents by pre-charging part of dispersing agent and feeding TFE in an amount to produce 25-30 wt% solids batches. The remaining dispersing agent is fed into the reactor with a delay after polymerization is started. Pre-charging part of the dispersing agent and adding the remainder of the dispersing agent later in the batch is referred to herein as "split addition of dispersing agent." Polypropylene glycol is employed as a nucleating additive.

For the selected dispersing agents listed in Table 5, the following polymerization procedure is used with unless otherwise indicated in Table 5. The polymerization of Run Nos. 1-3 (inventive) and Run Nos. 4-5 (comparative) is run using equal mass of dispersing agents. Polymerization of Run Nos. 6-7 (inventive) and Run Nos.8-9 (comparative) is run using equal molar amounts of dispersing agent. Concentration (ppm) amounts of the dispersing agents listed in Table 5 are with respect to the amount of water present at the end of batch.

To a 12 liter, horizontally disposed, jacketed, stainless steel autoclave with a two-blade agitator is added 4000 gm of deionized, deaerated water and 250 gm of paraffin wax. To the autoclave is added 500 gm of deionized, deaerated water which contains 0.09 gm of polypropylene glycol (PPG) having a molecular weight of approximately 450 g/mol. The autoclave is sealed and placed under vacuum. The autoclave pressure is raised to 30 psig (310 kPa) with nitrogen and vented to atmospheric pressure. The autoclave is pressured with nitrogen and vented 2 more times. This pressure/vent cycle is repeated three times using tetrafluoroethylene (TFE). The reactor is heated to 60°C with no agitation. The agitator speed is set to 75 RPM after reactor temperature is at 60°C and continued heating the reactor to 90°C. For polymerization of Run Nos. 1-3 (inventive) and Run Nos. 4-5 (comparative) using equal mass of dispersing agents, to the autoclave is added at 78ml/min an aqueous solution containing 8.1 gm of the dispersing agents listed in Table 5 made using deionized, deaerated water. For polymerization of Run Nos. 6-7 (inventive) and Run Nos.8-9 (comparative) using equal molar amounts of dispersing agent (27.7 millimoles), to the autoclave is added at 78ml/min an aqueous solution containing the dispersing agents listed in Table 5 made using deionized, deaerated water (equal to one-half of the total amount in Table 5). After addition of the dispersing agent, 87 ml of ammonium per sulfate (APS) solution composed of 0.4 gm of APS in 500 gm of deionized, deaerated water is added at 76ml/min.

TFE is charged to the reactor to bring the reactor pressure to 400 psig (2.86MPa). The agitator speed is changed to 90 RPM. 300 ml of an initiator solution composed of 21.0 gm (70% active) of disuccinic acid peroxide and 879.0 gm of deionized, deaerate water is added to the reactor at 80ml/min feed rate. Kick-off of the polymerization reaction is considered to have occurred after a drop of 10 psi (69kPa) from the maximum pressure observed during injection of the initiator solution. Autoclave pressure is brought back to 400 psig (2.86MPa) with TFE and maintained at that pressure for the duration of the polymerization by continuous addition of makeup TFE. At kick-off, commenced the simultaneous addition of maintenance initiator solution containing 21.0 gm (70% active) of disuccinic acid peroxide and 879.0 gm of deionized, deaerated water at a rate of 3 ml/min feed rate until the end of batch and 100 ml of solution composed of 0.5 gm of Iron (II) Sulfate heptahydrate in 499.5 gm of deionized water is charged to the reactor at 100 ml/min. After 250 gm of TFE has been fed since kick-off, an aqueous solution made using deionized, deaerated water containing 8.1 gm of the dispersing agents listed in Table 5 is fed to the reactor at a rate of 10 ml/min feed rate for Run Nos. 1-3 (inventive) and Run Nos.4-5 (comparative) using equal mass of dispersing agents. However, as noted in Table 5, Run No. 4 results in no kick-off and Run No. 5 results in kick-off and stopping of the batch after feed rate dropped to zero and thus the additional dispersing agent is not added to the reactor. For Run Nos. 6-7 (inventive) and Run Nos. 8-9 (comparative), equal molar amounts of dispersing agent, is added at the same rate but the solution contains 27.7 millimoles of each dispersing agent listed in Table 5 (equal to one-half of the total amount listed in Table 5). After a total of 2100 gm of TFE has been added since the kick-off to the reactor, the agitator is stopped, establishing the completion of the polymerization reaction. After the agitator is stopped, the reactor is vented to atmospheric pressure, is cooled to 80°C and then dispersion is discharged. Upon cooling, the solid wax is separated from the dispersion and the dispersion is filtered to remove undispersed solids.

The reactor is opened, and all adhered polymer removed from the reactor. Reactor cleanout is combined with the filtered solids and recorded as total wet coagulum. The total wet coagulum is reported as % wet coagulum formed based on dispersed polymer weight.

Results are reported in Table 5 including dispersing agent concentration, kick-off time, kick-off rate, batch time, wt% solids, particle size, wet coagulum, melting point, and space time yield (STY).

For calculation of space time yield (STY), space is the volume of the reactor, time is the time from kick-off of the polymerization reaction until its completion, and yield is the weight of dispersed polymer formed. STY is expressed herein as gm (of dispersed polymer)/l-hr.

**Table 5**

| Run # | Precharge Water | Dispersing Agent | | | DispersingAgent Precharge | | DispersingAgent Added after Kick-off | | Total Dispersing Agent | | |
|---|---|---|---|---|---|---|---|---|---|---|---|
| | gm | DA No. | Cation | Amount gm | Aqueous solution ml | Amount gm | Aqueous solution ml | Addition Rate ml/min | Amount, gm | mMoles | ppm based on water @ end of batch |
| 1 | 4000 | I-3 | $NH_4$+ | 8.1 | 270 | 8.1 | 270 | 10 | 16.2 | 43.6 | 2725 |
| 2 | 3730 | I-8 | Na+ | 8.1 | 405 | 8.1 | 405 | 15 | 16.2 | 55.4 | 2846 |
| 3 | 4000 | I-1 | $NH_4$+ | 8.1 | 270 | 8.1 | 270 | 25 | 16.2 | 51.0 | 2875 |
| 4 | 4000 | C-5 | Na+ | 8.1 | 270 | | | | 8.1 | 37.5 | 1571 |
| 5 | 4000 | C-7 | Na+ | 8.1 | 270 | | | | 8.1 | 34.9 | 1529 |
| 6 | 4000 | I-3 | $NH_4$+ | 10.3 | 343 | 10.3 | 343 | 17 | 20.6 | 55.4 | 3383 |
| 7 | 4000 | I-1 | $NH_4$+ | 8.3 | 293 | 8.3 | 293 | 15 | 17.6 | 55.4 | 3095 |
| 8 | 4000 | C-5 | Na+ | 6.0 | 200 | 6.0 | 200 | 10 | 12.0 | 55.5 | 2068 |
| 9 | 4000 | C-7 | Na+ | 6.45 | 215 | 6.45 | 215 | 11 | 12.9 | 55.4 | 2214 |

**Table 5 (Continued)**

| Run # | Kick-off Time | Kick-off Rate | Batch Size | Batch Time | Solids | Dispersion Amount | Particle Size | Coagulum Wet | Wet Coagulum* | DSC 1st Melt | STY |
|---|---|---|---|---|---|---|---|---|---|---|---|
| | min | PSI/min | gm of TFE | min | % | Gm | Dv(50) nm | gm | % | $^0$C | gm/ (L-hr) |
| 1 | 29.9 | 0.334 | 2100 | 139.8 | 26.7 | 7928 | 218 | 100 | 1.26 | 335.2 | 75.6 |
| 2 | 33.7 | 0.297 | 2100 | 54.9 | 28.4 | 7929 | 216 | 20 | 0.25 | 335.8 | 205.2 |
| 3 | 14.8 | 0.676 | 2101 | 35.5 | 29.9 | 7889 | 260 | 14 | 0.18 | 334.9 | 331.9 |
| 4 | No Kick-off (4 psi drop in 60.4 min) | 0.066 | | | | | | | | | |
| 5 | 33.7 | 0.297 | 5.8 | 10.0 | Batch was aborted after 10 min wait time after TFE feed rate dropped to zero and did not recover (total 5.8 gm TFE fed in 10 min) | | | | | | 0.0 |
| 6 | 27.0 | 0.370 | 2100 | 190.6 | 25.8 | 8246 | 213 | 73 | 0.89 | 335.0 | 55.9 |
| 7 | 12.6 | 0.794 | 2101 | 35.4 | 29.7 | 8041 | 261 | 14 | 0.17 | 335.2 | 337.3 |

(continued)

**Table 5 (Continued)**

| Run # | Kick-off Time | Kick-off Rate | Batch Size | Batch Time | Solids | Dispersion Amount | Particle Size | Coagulum Wet | Wet Coagulum* | DSC 1st Melt | STY |
|---|---|---|---|---|---|---|---|---|---|---|---|
| | min | PSI/min | gm of TFE | min | % | Gm | Dv(50) nm | gm | % | $^0C$ | gm/ (L-hr) |
| **8** | 42.6 | 0.235 | 2100 | 225.1 | 26.3 | 7411 | 248 | 429 | 5.79 | 336.0 | 43.2 |
| **9** | 20.0 | 0.500 | 1850.0 | 234.2 | 22.5 | 5952 | 262 | 1096 | 17.99 | 334.9 | 28.6 |

**Based on dispersed polymer weight*

With reference Table 5, Example 23 shows in Run Nos. 1-3 that the use of dispersing agents ammonium 4-((4-(tert-butyl)phenyl)sulfonyl)benzenesulfonate) (I-3) ($NH_4^+$ salt), ammonium 3,5-di-tert-butylbenzenesulfonate (I-8) ($NH_4^+$ salt) and ammonium 3,5-di-tert-butyl, 4-methoxybenzenesulfonate (I-1) ($NH_4^+$ salt) produce high solids PTFE batches in the presence of high equal amounts dispersing agents in a process employing split addition of dispersing agent. The high PTFE solids concentrations are achieved with low amounts of coagulum. The data show that total levels of dispersing agent in the range of 1000-4000 ppm often used to achieve solids levels for commercial production can successfully be used in a process employing the low reactivity dispersing agents in accordance with present invention and that split addition of the dispersing agent can be used if desired.

The comparative dispersing agent, sodium octyl sulfonate (C-5) ($Na^+$ salt) using the same amount by weight in Run No. 4 as the I-3, I-8, and I-1 dispersing agents results in no kick-off in over an hour in the presence of the pre-charged dispersing agent. The comparative dispersing agent sodium octyl sulfate (C-7) ($Na^+$ salt) used in the same amount by weight in Run No. 5 as the I-3, I-8, and I-1 dispersing agents results in stopping of the batch because the TFE feed rate dropped to zero in the presence of the pre-charged dispersing agent. Similar good results, i.e., high solids levels with low coagulum, are again achieved in Run Nos. 6 and 7 with the I-3 and I-1 dispersing agents used in equal molar amount in a process with split addition of the dispersing agent. The comparative dispersing agent, sodium octyl sulfonate (C-5) ($Na^+$ salt) using the same molar amount of dispersing agent in Run No. 8 as the I-3 and I-1 dispersing agents results in high solids but with a longer batch time with significant coagulum. The comparative dispersing agent, sodium octyl sulfate (C-7) ($Na^+$ salt) using the same molar amount of dispersing agent in Run No. 9 as the I-3 and I-1 dispersing agents results in somewhat lower solids but with a longer batch time than Run No. 8 with very high coagulum. The space time yield (STY), both in the processes using the I-3, I-8, and I-1 dispersing agents in the equal weight runs, and using the using the I-3 and I-1 dispersing agents in the equal molar runs, is higher than the STY of the comparative dispersing agents C-5 and C-7 in the equal molar runs (STY of the comparative dispersing agents C-5 and C-7 in the equal weight runs is 0 since no PTFE is produced).

### Comparative Example 20 and Polymerization Example 24

### PTFE Dispersion - 10 wt% Solids Batches

### Perfluoropolyether Acid Nucleating Additive

The following examples illustrate a semi-batch process for making aqueous PTFE dispersion by pre-charging the dispersing agent and feeding TFE in an amount to produce 10 weight % solids batches. Perfluoropolyether acid is employed as a nucleating additive. Comparative Example 20 illustrates a process employing a comparative dispersing agent. Polymerization Example 24 illustrates a process accordance with the invention.

### Comparative Example 20

To a nominally 10-gallon jacketed, cylindrically shaped stainless-steel reactor with an aspect ratio of 1.5 equipped with a paddle stirrer is charged 600 grams of natural paraffin wax, 1.6 g of succinic acid and 19.5 liters of demineralized water. An aqueous solution (160 mL) containing a 4.0 g carboxylic acid functional perfluoropolyether acid polymerized from hexafluoropropylene oxide with a number average molecular weight of approximately 1500 and 2.4 g t-butanol is added, and the reactor is sealed. The reactor is purged with nitrogen to greater than 20 PSIG and vented to 5 PSIG three times. After heating to 65°C, the reactor is agitated at 70 RPM then pressured to 400 PSIG with nitrogen and checked for leaks. After venting and reducing the agitation rate to 20 RPM, the reactor is purged with TFE to greater than 25 PSIG and vented to 5 PSIG three times. The agitator is set to 70 RPM and the contents are heated to 90°C. The reactor is charged with an aqueous solution containing 1.2 g of linear sodium dodecyl benzene sulfonate (C-2) ($Na^+$ salt) at a rate of 100 mL/min, then TFE is charged until a pressure of 400 PSIG is reached. To start the polymerization, 60 mL of a 1.0% (m/v) aqueous solution of disuccinyl peroxide (DSP) are added at a rate of 80 mL/min. The required 10 PSIG pressure drop does not occur after 45 minutes (only a decrease of 1.5 PSIG occurred), so the agitator is stopped, and the reactor is vented. No polymer is recovered due to no reaction.

### Polymerization Example 24

The procedure of Comparative Example 20 is repeated except that the reactor is charged with an aqueous solution containing 1.0 g of sodium 3,5-di-tert-butylbenzenesulfonate acid (I-8) ($Na^+$ salt) dispersing agent at a rate of 100 mL/min. Also, polymerization began (kick-off) as indicated by a decrease in pressure of 10 PSIG, which occurred at a Kick-off Time of 45 minutes (Kick-off Rate of 0.22 PSIG/min). Then, additional TFE is fed to the reactor at a rate sufficient to maintain a constant pressure of 400 PSIG. Also, after kick-off, 100 mL of an aqueous solution containing 0.1 g ferrous

sulfate heptahydrate is added at a rate of 100 mL/min. After a total of 2268g of TFE are added since kick-off, the TFE addition valve is closed, the agitator is stopped, and the reactor is vented. The reaction time is 36 minutes. The resulting dispersion, containing 11.30% polymer, is discharged from the reactor and allowed to cool. The dispersion is found to have a raw dispersion particle size of 195 nm. After draining the dispersion, 192 g of coagulum, containing water, paraffin wax and polymer, are left behind in the reactor.

The results of Comparative Example 20 and Polymerization Example 24 are summarized in Table 6 including Results are reported in Table 6 including Kick-off Time, Kick-off Rate, batch time, wt% solids, particle size, and wet coagulum.

**Table 6**

| Example | Dispersing Agent | | | Kick-off Time (time to drop 10 PSI) | Kick-off Rate | Batch Time | Dispersion solids | Particle Size | Coagulum Wet |
|---|---|---|---|---|---|---|---|---|---|
| | DA No. | Cation | Amount, gm | Min | PSI/ min | Min | wt% | Dv(50) nm | gm |
| **Comp. 20** | C-2 | $Na^+$ | 1.2 | No Kick-off (1.5 psi drop in 45 min) | 0.03 | | | | |
| **Ex. 24** | I-8 | $Na^+$ | 1 | 45 | 0.22 | 36 | 11.3 | 195 | 192 |

The results of Example 24 again show that a process in accordance with the invention using a low reactivity hydrocarbon dispersing agent enables the polymerization of PTFE to kick-off in the presence of pre-charged dispersing agent and to polymerize to 10% solids. On the other hand, Comparative Example 16 employing a comparative dispersing agent did not kick-off after 45 minutes.

**Polymerization Example 25**

**PTFE Dispersion - 10 wt% Solids Batches**

**(1-8) ($NH_4^+$ Salt) Dispersing Agent**

**Perfluoroionomer Particulate Nucleating Additive**

The following example illustrates a semi-batch process in accordance with the invention for making aqueous PTFE dispersion by delayed addition of the ammonium 3,5-di-tert-butylbenzene sulfonate (I-8) ($NH_4^+$ salt) dispersing agent and feeding TFE in an amount to produce 10 weight % solids. Perfluoroionomer particulate as disclosed in US Patent 6,916,853 is employed as a nucleating additive.

To a nominally 1-gallon jacketed, cylindrically-shaped stainless-steel horizontal reactor with a paddle stirrer, 40 grams of natural paraffin wax and 1.5 liters of demineralized water are added.

An aqueous solution (500 mL) containing 0.4 g ascorbic acid, 0.27 g ammonium pentaborate octahydrate, 3 g tert-butanol, 0.15 g perfluoroionomer particulate as disclosed in US Patent 6,916,853, 0.015 g ferrous sulfate heptahydrate, 60 μL 25-30% ammonium hydroxide, and 2 g potassium di-tert-butyl phosphate is added to the reactor.

The reactor is then sealed and heated up to 40 °C. The reactor is purged with nitrogen to greater than 20 PSIG and vented to 5 PSIG three times. The reactor is pressured to 400 PSIG with nitrogen and checked for leaks. After venting, the reactor is purged with TFE to greater than 25 PSIG and vented to 5 PSIG three times. After heating up the reactor to 65 °C,

setting the agitator at 40 RPM, and pressurizing the reactor to 350 PSIG, 40 mL of initiator solution is pumped into the reactor at a rate of 10 mL/min. The initiator solution contains 0.075 % tert-butyl hydroperoxide (TBHP). After the 40 mL initiator solution is completed, the agitator is set to 100 RPM and begin pumping the initiator solution at 2 ml/min. After polymerization has begun (kick-off) as indicated by a decrease in pressure of 10 PSIG, additional TFE is fed to the reactor at a rate sufficient to maintain a constant pressure of 350 PSIG. The temperature is maintained at 65 °C. After 45 g of TFE is added since kick-off, begin pumping in the aqueous solution containing 1.16% ammonium 3,5-di-tert-butylbenzene sulfonate (I-8) ($NH_4^+$ salt) at a rate of 10 ml/min till the end of the reaction. After a total of 227 g of TFE are added since kick-off, the TFE addition valve is closed, the agitator is stopped, and the reactor is vented. The reaction time is 36 minutes. The resulting dispersion, containing 10.04 wt% polymer, is discharged from the reactor and allowed to cool. After removing the paraffin wax, the dispersion is found to have a raw dispersion particle size of 144.5 nm. After draining the dispersion, 27 g of coagulum, containing a mix of water, paraffin wax and polymer, are left behind in the reactor.

**Polymerization Example 26**

**High Solids PTFE Dispersion**

**(1-3) ($NH_4^+$ Salt) Dispersing Agent**

**No Nucleating Agent**

The following example illustrate a semi-batch process for making aqueous PTFE dispersion by pre-charging ammonium 4-((4-(tert-butyl)phenyl)sulfonyl)benzenesulfonate) (I-3) ($NH_4^+$ salt) dispersing agent and feeding TFE in an amount to produce an approximately 30 weight % solids batch. No nucleating agent is used.

To a 12 liter, horizontally disposed, jacketed, stainless steel autoclave with a two-blade agitator is added 4340 gm of deionized, deaerated water and 250 gm of paraffin wax. To the autoclave is added 500 gm of deionized, deaerated water which contains 15.657 gm of I-3 dispersing agent ($NH_4^+$ salt) and 0.058 gm of L-ascorbic acid and 500 gm of deionized, deaerated water which contains 0.092 gm of iron (II) sulfate heptahydrate. The autoclave is sealed and placed under vacuum. The autoclave pressure is raised to 30 psig (310kPa) with nitrogen and vented to atmospheric pressure. The autoclave is pressured with nitrogen and vented 2 more times. This pressure/vent cycle is repeated three times using tetrafluoroethylene (TFE). The reactor is heated to 60°C with no agitation. The agitator speed is set to 75 RPM after reactor temperature is at 60°C and continued heating the reactor to 90°C. 683.3 gm of TFE is charged to the reactor to bring the reactor pressure to 400 psig (2.86MPa). The agitator speed is changed to 90 RPM. 370 ml of an initiator solution composed of 45.0 gm (70% active) of disuccinic acid peroxide and 855.0 gm of deionized, deaerated water is added to the reactor at 100ml/min feed rate. After 7.6 minutes from the start of initiator injection the reactor pressure drops 10 psi (69kPa) from the maximum pressure observed during injection of the initiator solution. Autoclave pressure is brought back to 400 psig (2.86MPa) with TFE and maintained at that pressure for the duration of the polymerization. After 250 gm of TFE has been fed since kick-off, the simultaneous addition of a maintenance initiator solution is commenced containing 45.0 gm (70% active) of disuccinic acid peroxide and 855.0 gm of deionized, deaerated water at a rate of 2 ml/min feed rate until the end of batch. After 97.7 minutes since kick-off, 2300 gm of TFE and 144 ml of maintenance initiator solution has been added to the reactor. The agitator is stopped, the reactor is vented to atmospheric pressure, the reactor is cooled to 80 $^0$C and then dispersion is discharged. Upon cooling, 160 gm of the solid wax is separated from the dispersion and the dispersion is filtered to remove undispersed solids. The reactor is opened and all adhered polymer removed from the reactor. Reactor cleanout is combined with the filtered solids and recorded as total wet coagulum of 115 gm. The total wet coagulum formed contains water, paraffin wax and polymer. 8346 gm of aqueous dispersion is produced with 29.7% solids and an average particle size by volume, Dv(50), of 220 nm. Polymer is coagulated by diluting the dispersion to about 10 wt% solids and by adding 10% by volume of a 20 wt% aqueous ammonium carbonate solution followed by vigorous agitation until the polymer fully separates from the water. The polymer is dried in a vacuum oven at 110°C for 12 hours. Melting point of this polymer as measured by DSC on first heat is 334.44°C.

**Polymerization Example 27**

**High Solids PTFE Dispersion and Production of Fine Powder**

**(1-8) ($Na^+$ salt) Dispersing Agent**

**Polypropylene Glycol Nucleating Additive**

The following example illustrates a semi-batch process for making aqueous PTFE dispersion by adding sodium

3,5-di-tert-butylbenzene sulfonate (I-8) ($Na^+$ salt) dispersing agent immediately after kick-off and feeding TFE in an amount to produce an approximately 35 weight % solids batch. Polypropylene glycol is employed as a nucleating additive. PTFE fine powder is produced from the dispersion.

To a nominally 10-gallon jacketed, cylindrically-shaped stainless steel reactor with an aspect ratio of 1.5 equipped with a paddle stirrer is charged 600 grams of natural paraffin wax, 1.6 g of succinic acid and 18.1 liters of demineralized water. An aqueous solution (100 mL) containing 0.075 g polypropylene glycol having number average molecular weight of approximately 450 is added, and the reactor is sealed. The reactor is purged with nitrogen to greater than 20 PSIG and vented to 5 PSIG three times. After heating to 65°C, the reactor is agitated at 70 RPM then pressured to 400 PSIG with nitrogen and checked for leaks. After venting and reducing the agitation rate to 20 RPM, the reactor is purged with TFE to greater than 25 PSIG and vented to 5 PSIG three times before adding 40 mL of an aqueous solution containing 0.08 g of ammonium persulfate. The agitator is set to 70 RPM and the contents are heated to 90°C. The reactor is charged with TFE until a pressure of 400 PSIG is reached. To start the polymerization, 140 mL of a 1.0% (m/v) aqueous solution of disuccinyl peroxide (DSP) are added at a rate of 80 mL/min. After polymerization has begun (kick-off) as indicated by a decrease in pressure of 10 PSIG, additional TFE is fed to the reactor at a rate sufficient to maintain a constant pressure of 400 PSIG. Also, immediately following kick-off, 2940 mL of an aqueous solution containing 50 g of sodium 3,5-di-tert-butylbenzene sulfonate (I-8) ($Na^+$ salt), 80 mL of an aqueous solution containing 0.08 g of I-ascorbic acid, and 100 mL of an aqueous solution containing 0.1 g of ferrous sulfate heptahydrate are all added at a rate of 80 mL/min. After a total of 10.9 kg of TFE are added since kick-off, the TFE addition valve is closed, the agitator is stopped, and the reactor is vented. The reaction time is 164 minutes. The resulting dispersion, containing 35.34% polymer, is discharged from the reactor and allowed to cool. The dispersion is found to have a raw dispersion particle size of 230 nm. After draining the dispersion, 1132 g of coagulum, containing water, paraffin wax and polymer, are left behind in the reactor.

The dispersion is coagulated at 12% solids in a 3L vessel and dried in a static oven at a temperature of 150°C for 24 hours. The PTFE fine powder produced has an SSG of 2.1666 and thus has properties equivalent to PTFE fine powder made using fluorosurfactant.

**Polymerization Example 28**

**High Solids PTFE Dispersion**

**(1-3) ($NH_4^+$ Salt) Dispersing Agent**

**Perfluoropolyether Acid Nucleating Additive**

The following example illustrates a semi-batch process for making aqueous PTFE dispersion using ammonium 4-((4-(tert-butyl)phenyl)sulfonyl)benzenesulfonate) (I-3) (NH4+ salt) dispersing agent with the addition of the dispersing agent being delayed until after kick-off and feeding TFE in an amount to produce an approximately 32 weight % solids batch. Perfluoropolyether acid is employed as a nucleating additive.

To a nominally 10-gallon jacketed, cylindrically-shaped stainless steel reactor with an aspect ratio of 1.5 equipped with a paddle stirrer is charged 600 grams of natural paraffin wax, 2.0 g of succinic acid, 0.05 g of Tomadol 23-1 nonionic surfactant (Evonik Industries AG, Essen, Germany) and 19.5 liters of demineralized water. An aqueous solution (160 mL) containing 8.0 g perfluoropolyether acid polymerized from hexafluoropropylene oxide with a number average molecular weight of approximately 1500 and 8 g t-butanol is added, and the reactor is sealed. The reactor is purged with nitrogen to greater than 20 PSIG and vented to 5 PSIG three times. After heating to 65°C, the reactor is agitated at 70 RPM then pressured to 400 PSIG with nitrogen and checked for leaks. After venting and reducing the agitation rate to 20 RPM, the reactor is purged with TFE to greater than 25 PSIG and vented to 5 PSIG three times. The agitator is set to 70 RPM and the contents are heated to 90°C. The reactor is charged with TFE until a pressure of 400 PSIG is reached. To start the polymerization, 300 mL of an aqueous solution containing 4.5 g of disuccinyl peroxide (DSP) are added at a rate of 80 mL/min. After polymerization has begun (kick-off) as indicated by a decrease in pressure of 10 PSIG, additional TFE is fed to the reactor at a rate sufficient to maintain a constant pressure of 400 PSIG. After 45 g of TFE are added since kick-off, 300 mL of an aqueous solution containing 0.24 g ferrous sulfate heptahydrate and 0.3 g of I-ascorbic acid is added at a rate of 45 mL/min. After 181 g of TFE are added since kick-off, 2200 mL of an aqueous solution containing 44 g of ammonium 4-((4-(tert-butyl)phenyl)sulfonyl)benzenesulfonate) (I-3) ($NH_4^+$ salt) dispersing agent is added at a rate of 80 mL/min. After a total of 9979 g of TFE are added since kick-off, the TFE addition valve is closed, the agitator is stopped, and the reactor is vented. The reaction time is 197 minutes. The resulting dispersion, containing 32.62% polymer, is discharged from the reactor and allowed to cool. The dispersion is found to have a raw dispersion particle size of 247 nm. After draining the dispersion, 930 g of coagulum, containing water, paraffin wax and polymer, are left behind in the reactor.

**Polymerization Example 29**

**High Solids PTFE Dispersion**

**(1-8) ($Na^+$ Salt) Dispersing Agent**

**Dispersed Particulate of Fluorinated Ionomer Nucleating Additive T-butylhydroperoxide Initiator**

The following example illustrates a semi-batch process for making aqueous PTFE dispersion using sodium 3,5-di-tert-butylbenzene sulfonate (I-8) ($Na^+$ salt) dispersing agent with some the of dispersing agent being pre-charged and some being added starting immediately after kick-off, i.e., "split addition". TFE is fed in an amount to produce an approximately 41 weight % solids batch. T-butylhydroperoxide is used as the initiator. Dispersed particulate of fluorinated ionomer is employed as a nucleating additive as disclosed in U.S. Patent 8,153,738.

To a nominally 10-gallon jacketed, cylindrically-shaped stainless steel reactor with an aspect ratio of 1.5 equipped with a paddle stirrer is charged 600 grams of natural paraffin wax and 19.5 liters of demineralized water. An aqueous solution (100 mL) containing 3.0 g dispersed particulate of fluorinated ionomer made by a process as disclosed in U.S. Patent 6,150,426, and 5.0 ml t-butanol is added, and the reactor is sealed. The reactor is purged with nitrogen to greater than 20 PSIG and vented to 5 PSIG three times. After heating to 65°C, the reactor is agitated at 70 RPM then pressured to 400 PSIG with nitrogen and checked for leaks. After venting and reducing the agitation rate to 20 RPM, the reactor is purged with TFE to greater than 25 PSIG and vented to 5 PSIG three times. The agitator is set to 70 RPM then 600 mL of a 0.5% (m/v) aqueous solution of L-ascorbic acid and 1250 mL of a 2% (m/v) aqueous solution of sodium 3,5-di-tert-butylbenzene sulfonate (I-8) ($Na^+$ salt) are added both at a rate of 80 mL/min. TFE is charged until a pressure of 390 PSIG is reached. To start the polymerization, 80 mL of a 1.0% (m/v) aqueous solution of t-butyl hydroperoxide (TBHP) is added at a rate of 80 mL/min, kick-off is observed, then TFE is fed to maintain a constant pressure of 400 PSIG. After kick-off, additional 1.0% (m/v) aqueous TBHP solution is added at a rate of 2.0 mL/min for the remainder of the batch. Also, immediately after kick-off, 1250 mL of a 2% (m/v) aqueous solution of sodium 3,5-di-tert-butylbenzene sulfonate is added at a rate of 80 mL/min. After a total of 13.15 kg of TFE are added since kick-off, the TFE addition valve is closed, the agitator is stopped, and the reactor is vented. The reaction time is 68 minutes. The resulting dispersion, containing 40.66% polymer, is discharged from the reactor and allowed to cool. The dispersion is found to have a raw dispersion particle size of 236 nm. After draining the dispersion, 253 g of coagulum, containing approximately 6 g water, 241 g of paraffin wax and 6 g PTFE (representing a 0.05% mass loss), is left behind in the reactor. Isolated PTFE polymer after coagulation and drying has an SSG of 2.1843.

**Polymerization Example 30**

**High Solids PTFE Dispersion**

**(1-1) ($NH_4^+$ salt) Dispersing Agent**

**Dispersed Particulate of Fluorinated Ionomer Nucleating Additive T-butylhydroperoxide Initiator**

The following example illustrates a semi-batch process for making aqueous PTFE dispersion using ammonium 3,5-di-tert-butyl, 4-methoxybenzene sulfonate (I-1) (NH4+ salt) dispersing agent with some the of dispersing agent being pre-charged and some being added starting immediately after kick-off, i.e., "split addition". TFE is fed in an amount to produce an approximately 41 weight % solids batch. T-butylydroperoxide (TBHP) is used as the initiator. Dispersed particulate of fluorinated ionomer is employed as a nucleating additive as disclosed in U.S. Patent 8,153,738.

The procedure of Example 29 is repeated except that 2% (m/v) aqueous solution of ammonium 3,5-di-tert-butyl, 4-methoxybenzene sulfonate (I-1) ($NH_4^+$ salt) as dispersing agent is added at a rate of 80 mL/min prior to kick-off. Also immediately after kick-off, 1289 mL of a 2% (m/v) aqueous solution of ammonium 3,5-di-tert-butyl, 4-methoxybenzene sulfonate (I-1) ($NH_4^+$ salt) as dispersing agent is added at a rate of 80 mL/min.

The reaction time is 56 minutes. The resulting dispersion, containing 40.55% polymer, is discharged from the reactor and allowed to cool. The dispersion is found to have a raw dispersion particle size of 227 nm. After draining the dispersion, 630 g of coagulum, containing approximately 161 g of water, 322g of paraffin wax and 161 g of PTFE (representing a 1.2% mass loss), is left behind in the reactor. Isolated PTFE polymer after coagulation and drying has an SSG of 2.1920.

**Example Illustrating Reactivity of Hydrocarbon**

**Dispersing Agents To T-butylhydroperoxide Initiator**

**Reactivity Example 1**

**T-butylhydroperoxide Oxidative Loss**

This example illustrates the T-butylhydroperoxide Oxidative Loss of the dispersing agents used in processes in accordance with the present invention in comparison to results observed with comparative dispersing agents in the same test.

A 100 mM buffer solution of monobasic/dibasic sodium phosphate is prepared to pH = 7.00 ± 0.02 at ambient temperature. This solution is deoxygenated via a sparge of nitrogen gas. Under a nitrogen atmosphere (< 10 ppm/v $O_2$), a solution 10 ± 1 mM of a dispersing agent listed in Table 5 and 70 ± 1 mM *t*-butylhydroperoxide in the previously-prepared buffer is prepared. A control solution of 10 ± 1 mM dispersing agent in pH 7 buffer, but without t-butylhydroperoxide, is also prepared. 0.55 ± 0.1 mL of each solution is transferred to two separate borosilicate glass tubes (5.0 mm o.d., 0.77 mm wall thickness). These tubes are connected to a vacuum manifold on which ca. 1/3 of the nitrogen gas headspace is removed, and then the tubes are flame sealed. The sealed tubes are heated in an oven at 95°C for 72 h. After heating, the tubes are cooled to ambient temperature, scored with a file, and cracked open. The solutions are withdrawn.

Liquid chromatography [LC] is used to assay the quantities of dispersing agent in heated control solution and in heated solution with t-butylhydroperoxide. Solutions are diluted 100X in LC-grade water. A quadratic calibration curve of at least five points is prepared with the dispersing agent in question. A reverse-phase LC method with a gradient of 95:5 2 mM aqueous ammonium acetate:acetonitrile and acetonitrile is employed, using a $C_{18}$ column. Quantitative LC is performed to determine the decreased percentage of dispersing agent, i.e., wt% T- butylhydroperoxide Oxidative Loss, performed with a UV detector if possible (if the dispersing agent in question reported in the UV spectrum above 200 nm), otherwise with a mass spectral detector.

Results of testing of the dispersing agents are listed in Table 7.

| Table 7 | |
|---|---|
| **Dispersing Agent** | **Wt% Loss** |
| Sodium dodecyl sulfate C1 - $Na^+$ salt | 26 |
| Linear sodium dodecyl benzene sulfonate C-2 - $Na^+$ salt | 29 |
| 3,5-Diisopropylbenzenesulfonic acid C-11 - used as the acid | 47 |
| Sodium *Tert*-butylbenzene sulfonate C-4 - $Na^+$ salt | 3.7 |
| Sodium 4-(phenylsulfonyl)-benzenesulfonate C-8 - $Na^+$ salt | 4.4 |
| 4-((4-(tert-butyl)phenyl)sulfonyl)benzenesulfonic acid) I-3 - used as the acid | 2.9 |
| 3,5-Di-tert-butylbenzenesulfonic acid I-8 - used as the acid | 3.3 |
| 3,5-Di-tert-butyl, 4-hydroxybenzenesulfonic acid C-12 - used as the acid | ~100 |

The results show very significantly lower wt% T-butylhydroperoxide Oxidative Loss and thus lower reactivity to the initiator t-butylhydroperoxide for the I-3 and I-8 dispersing agents than for the comparative dispersing agents C-1, C-2, C11, and C-12. While the C-4 and C-8 dispersing agents show low wt% T-butylhydroperoxide Oxidative Loss, the polymerization results in the Polymerization Examples above show that C-4 and C-8 dispersing agent provides ineffective stabilization of fluoropolymer dispersions. C-12 shows extreme loss that is believed to be due to the presence of a phenolic hydroxy group in this compound. Phenolic hydroxy groups are believed to be susceptible to radical attack by initiators such as tert-butylhydroperoxide.

## Claims

1. A process for polymerizing at least one fluoromonomer in an aqueous medium containing initiator and hydrocarbon dispersing agent to form an aqueous dispersion of particles of fluoropolymer, said hydrocarbon dispersing agent comprising a compound of formula I:

$$R\text{-}(XZ)_n \qquad I$$

wherein R is a hydrophobic hydrocarbon moiety that comprises one or more saturated or unsaturated, non-cyclic or cyclic aliphatic groups, the percentage of total $CH_3$ groups in relation to the total of $CH_3$, $CH_2$ and CH groups in said one or more aliphatic groups being at least 70%, said hydrophobic moiety being free of siloxane units;
wherein each X may be the same or different and represents an ionic hydrophilic moiety;
wherein each Z may be a same or different and represents one or more counter ions for said ionic hydrophilic moiety; and
wherein n is 1 to 3.

**2.** The process of claim 1 wherein said hydrophobic hydrocarbon moiety of hydrocarbon dispersing agent is entirely aliphatic and said hydrocarbon dispersing agent has a molecular weight without a counter ion Z of at least 170 g/mol.

**3.** The process of claim 1 wherein said hydrophobic hydrocarbon moiety of hydrocarbon dispersing agent contains at least one aromatic group and said hydrocarbon dispersing agent has a molecular weight without a counter ion Z of at least 215 g/mol.

**4.** The process of claims 1, 2, or 3 wherein

(i) said hydrophobic hydrocarbon moiety comprises no more than 3 consecutive $CH_2$ groups, preferably no more than 2 consecutive $CH_2$ groups, more preferably no consecutive $CH_2$ groups; and/or
(ii) said hydrophobic hydrocarbon moiety comprises at least 3 $CH_3$ groups, preferably at least 4 $CH_3$ groups, or 3 to 12 $CH_3$ groups.

**5.** The process of any of the preceding claims wherein said ionic hydrophilic moiety X is selected from the group consisting of acid groups and salts thereof, quaternary ammonium groups, amine-oxide groups, and tetrazole groups.

**6.** The process of any of the preceding claims wherein said hydrophilic moiety and counter ion XY is a group of the formula $-A^{-}-Y^{+}$, wherein $A^{-}$ is carboxylate, sulfonate, sulfate, phosphonate, or phosphate, preferably sulfonate; and wherein $Y^{+}$ is a hydrogen, ammonium, quaternary ammonium, nitrogen heterocycle, alkali metal, or alkaline earth.

**7.** The process of any of the preceding claims wherein said hydrophilic hydrocarbon moiety is free of halogens, and/or wherein said one or more aliphatic groups in said hydrophobic hydrocarbon moiety are non-cyclic or cyclic alkyl groups.

**8.** The process of any of claims 1 or 3-7 wherein said hydrophobic hydrocarbon moiety comprises one or more aromatic groups,
preferably wherein:

(i) said one or more aromatic groups are substituted and have a Hammett sigma plus value of greater than -1.0, preferably greater than -0.8, more preferably greater than 0, wherein the Hammett sigma plus value of the substituted aromatic group is calculated by summing the Hammett sigma plus values for each of the substituents on the ring of the aromatic group,
(ii) said hydrophobic hydrocarbon moiety is free of methyl groups directed bonded to an aromatic group,
(iii) said hydrophobic hydrocarbon moiety is free of benzylic hydrogen atoms, and/or
(iv) said hydrophobic hydrocarbon moiety is free of phenolic hydroxy groups.

**9.** The process of any of claims 1-8 wherein said hydrophobic hydrocarbon moiety comprises 8 to 50 carbon atoms, preferably 10 to 40 carbon atoms, more preferably 12 to 30 carbon atoms, most preferably 12 to 25 carbon atoms.

**10.** The process of any of the preceding claims wherein said hydrophobic hydrocarbon moiety is free of carbon-hydrogen bonds with bond dissociation energies to yield a hydrocarbon radical of less than 100 kcal/mol, and/or wherein said hydrocarbon dispersing agent has a t-butylhydroperoxide oxidative loss of less than 10 wt%, as measured in accordance with the method described in Reactivity Example 1.

**11.** The process of any of claims 1 or 3-10 wherein said hydrocarbon dispersing agent comprises a substituted moiety of the formula:

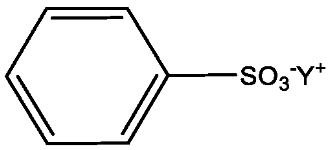

wherein $Y^+$ is hydrogen, ammonium, quaternary ammonium, nitrogen heterocycle, alkali metal, or alkaline earth.

**12.** The process of claim 1 wherein said hydrocarbon dispersing agent is a compound of formula II:

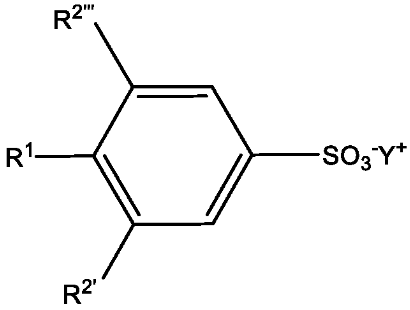

II

wherein $R^{2'}$ and $R^{2''}$ are the same or different and are saturated or unsaturated, non-cyclic or cyclic aliphatic groups having 4 to 16 carbon atoms with the percentage of total $CH_3$ groups in relation to the total of $CH_3$, $CH_2$ and CH groups in the $R^{2'}$ and $R^{2''}$ groups being at least about 70%, or $R^{2'}$ and $R^{2''}$ may be joined together to form an saturated or unsaturated aliphatic ring that may contain ether or ester linkages, with the proviso that the percentage of total $CH_3$ groups in relation to the total of $CH_3$, $CH_2$ and CH groups in said ring is at least about 70%;
wherein $R^1$ is hydrogen, methoxy, ethoxy, or phenoxy; and
wherein $Y^+$ is hydrogen, ammonium, quaternary ammonium, nitrogen heterocycle, alkali metal, or alkaline earth, preferably wherein:

(i) in the compound of formula II, $R^{2'}$, and $R^{2''}$ are the same or different and are t-butyl, t-butoxy, 2,3,3-trimethyl-2-butyl, or 2,3,3-trimethyl-2-butoxy, or - $CO(O)C(CH_3)_3$, and wherein $R^1$ is hydrogen, or methoxy, and wherein $Y^+$ is hydrogen, ammonium, or alkali metal,
(ii) in the compound of formula II, $R^{2'}$ and $R^{2''}$ are the same or different and are t-butyl or 2,3,3-trimethyl-2-butyl, $R^1$ is hydrogen, and $Y^+$ is hydrogen, ammonium, or alkali metal,
(iii) in the compound of formula II, $R^{2'}$ and $R^{2''}$ are the same or different and are t-butyl or 2,3,3-trimethyl-2-butyl, $R^1$ is methoxy and $Y^+$ is hydrogen, ammonium, or alkali metal,
or
(iv) in the compound of formula II, $R^{2'}$ and $R^{2''}$ are both t-butyl, $R^1$ is hydrogen or methoxy, and $Y^+$ is hydrogen, ammonium, or alkali metal.

**13.** The process of claim 12, wherein said formula II is:

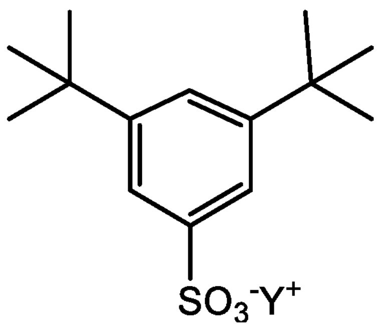

wherein $Y^+$ is hydrogen, ammonium, or alkali metal.

**14.** The process of claim 1 wherein said hydrocarbon dispersing agent is a compound of formula III:

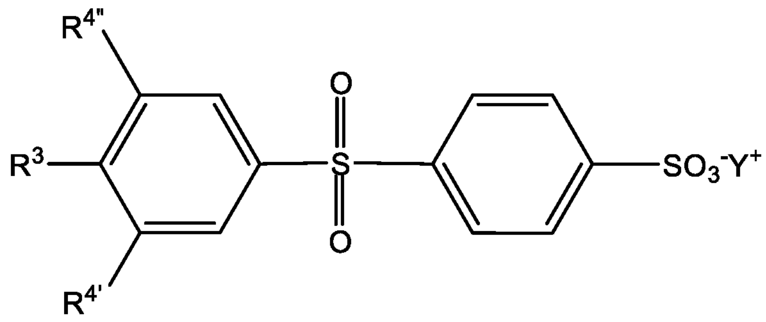

III

wherein $R^3$, $R^{4'}$, and $R^{4''}$ are the same or different and are hydrogen or saturated or unsaturated, non-cyclic or cyclic aliphatic groups having 4 to 16 carbon atoms with the percentage of total $CH_3$ groups in relation to the total of $CH_3$, $CH_2$ and CH groups the $R^3$, $R^{4'}$, and $R^{4''}$ groups being at least 70%. with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and

$Y^+$ is hydrogen, ammonium, quaternary ammonium, nitrogen heterocycle, alkali metal, or alkaline earth, preferably wherein:

(i) in the compound of formula III, $R^3$, $R^{4'}$, and $R^{4''}$ are the same or different and are hydrogen, t-butyl, t-butoxy, 2,3,3-trimethyl-2-butyl, or 2,3,3-trimethyl-2-butoxy, with the provisio that at least one of $R^3$, $R^{4'}$, and $R^{4''}$ is not hydrogen and, when $R^{4'}$ and $R^{4''}$ are hydrogen, $R^3$ is not hydrogen, and when $R^3$ is hydrogen, $R^{4'}$ and $R^{4''}$ are not hydrogen, and $Y^+$ is hydrogen, ammonium, quaternary ammonium, nitrogen heterocycle, alkali metal, or alkaline earth,
(ii) in the compound of formula III, $R^3$ is t-butyl or 2,3,3-trimethyl-2-butyl, $R^{4'}$ and $R^{4''}$ are hydrogen, and $Y^+$ is hydrogen, ammonium, or alkali metal,
(iii) in the compound of formula III, $R^3$ is t-butyl and $R^{4'}$ and $R^{4''}$ are hydrogen, and $Y^+$ is hydrogen, ammonium, or alkali metal,
(iv) in the compound of formula III, $R^{4'}$ and $R^{4''}$ are the same or different and are t-butyl or 2,3,3-trimethyl-2-butyl, $R^3$ is hydrogen and $Y^+$ hydrogen, ammonium, or alkali metal,
or
(v) in the compound of formula III, $R^{4'}$ and $R^{4''}$ are t-butyl, $R^3$ is hydrogen and $Y^+$ is hydrogen, ammonium, or alkali metal.

**15.** The process of any of the preceding claims wherein said initiator contained in the aqueous medium is an organic peroxide.

## Patentansprüche

**1.** Verfahren zum Polymerisieren mindestens eines Fluormonomers in einem wässrigen Medium, das Initiator und Kohlenwasserstoff-Dispergiermittel enthält, um eine wässrige Dispersion von Fluorpolymerteilchen zu bilden, wobei das Kohlenwasserstoff-Dispergiermittel eine Verbindung mit der Formel I umfasst:

$$R\text{-}(XZ)_n \qquad \text{I}$$

wobei R ein hydrophober Kohlenwasserstoffrest ist, der eine oder mehrere gesättigte oder ungesättigte, nichtzyklische oder zyklische aliphatische Gruppen umfasst, wobei der Prozentsatz der gesamten $CH_3$-Gruppen in Bezug auf die Gesamtheit der $CH_3$, $CH_2$ und CH-Gruppen in der einen oder den mehreren aliphatischen Gruppen mindestens 70 % beträgt, wobei der hydrophobe Rest frei von Siloxaneinheiten ist;
wobei jedes X gleich oder unterschiedlich sein kann und einen ionischen hydrophilen Rest darstellt;
wobei jedes Z gleich oder unterschiedlich sein kann und ein oder mehrere Gegenionen für den ionischen hydrophilen Rest darstellt; und

wobei n 1 bis 3 ist.

2. Verfahren nach Anspruch 1, wobei der hydrophobe Kohlenwasserstoffrest des Kohlenwasserstoff-Dispergiermittels vollständig aliphatisch ist und das Kohlenwasserstoff-Dispergiermittel ein Molekulargewicht ohne Gegenion Z von mindestens 170 g/mol aufweist.

3. Verfahren nach Anspruch 1, wobei der hydrophobe Kohlenwasserstoffrest des Kohlenwasserstoff-Dispergiermittels mindestens eine aromatische Gruppe enthält und das Kohlenwasserstoff-Dispergiermittel ein Molekulargewicht ohne Gegenion Z von mindestens 215 g/mol aufweist.

4. Verfahren nach Anspruch 1, 2 oder 3, wobei

(i) der hydrophobe Kohlenwasserstoffrest nicht mehr als 3 aufeinanderfolgende $CH_2$-Gruppen, vorzugsweise nicht mehr als 2 aufeinanderfolgende $CH_2$-Gruppen, noch bevorzugter keine aufeinanderfolgenden $CH_2$-Gruppen umfasst; und/oder
(ii) der hydrophobe Kohlenwasserstoffrest mindestens 3 $CH_3$-Gruppen, vorzugsweise mindestens 4 $CH_3$-Gruppen oder 3 bis 12 $CH_3$-Gruppen umfasst.

5. Verfahren nach einem der vorstehenden Ansprüche, wobei der ionische hydrophile Rest X ausgewählt ist aus der Gruppe bestehend aus Säuregruppen und deren Salzen, quaternären Ammoniumgruppen, Aminoxidgruppen und Tetrazolgruppen.

6. Verfahren nach einem der vorstehenden Ansprüche, wobei der hydrophile Rest und das Gegenion XY eine Gruppe mit der Formel $-A^{-}-Y^{+}$ ist, wobei $A^{-}$ Carboxylat, Sulfonat, Sulfat, Phosphonat oder Phosphat, vorzugsweise Sulfonat ist; und wobei $Y^{+}$ Wasserstoff, Ammonium, quaternäres Ammonium, Stickstoffheterocyclus, Alkalimetall oder Erdalkalimetall ist.

7. Verfahren nach einem der vorstehenden Ansprüche, wobei der hydrophile Kohlenwasserstoffrest frei von Halogenen ist und/oder wobei die eine oder mehreren aliphatischen Gruppen in dem hydrophoben Kohlenwasserstoffrest nicht-zyklische oder zyklische Alkylgruppen sind.

8. Verfahren nach einem der Ansprüche 1 oder 3 bis 7, wobei der hydrophobe Kohlenwasserstoffrest eine oder mehrere aromatische Gruppen umfasst,
vorzugsweise wobei:

(i) die eine oder mehreren aromatischen Gruppen substituiert sind und einen Hammett-Sigma-Plus-Wert von größer als -1,0, vorzugsweise größer als -0,8, bevorzugter größer als 0 aufweisen, wobei der Hammett-Sigma-Plus-Wert der substituierten aromatischen Gruppe durch Summieren der Hammett-Sigma-Plus-Werte für jeden der Substituenten an dem Ring der aromatischen Gruppe berechnet wird,
(ii) der hydrophobe Kohlenwasserstoffrest frei von Methylgruppen ist, die direkt an eine aromatische Gruppe gebunden sind,
(iii) der hydrophobe Kohlenwasserstoffrest frei von benzylischen Wasserstoffatomen ist und/oder
(iv) der hydrophobe Kohlenwasserstoffrest frei von phenolischen Hydroxygruppen ist.

9. Verfahren nach einem der Ansprüche 1 bis 8, wobei der hydrophobe Kohlenwasserstoffrest 8 bis 50 Kohlenstoffatome, vorzugsweise 10 bis 40 Kohlenstoffatome, bevorzugter 12 bis 30 Kohlenstoffatome, am bevorzugtesten 12 bis 25 Kohlenstoffatome umfasst.

10. Verfahren nach einem der vorstehenden Ansprüche, wobei der hydrophobe Kohlenwasserstoffrest frei von Kohlenstoff-Wasserstoff-Bindungen mit Bindungsdissoziationsenergien ist, die einen Kohlenwasserstoffrest von weniger als 100 kcal/mol ergeben, und/oder
wobei das Kohlenwasserstoff-Dispergiermittel einen t-Butylhydroperoxid-Oxidationsverlust von weniger als 10 Gew.-% aufweist, gemessen nach der in Reaktivitätsbeispiel 1 beschriebenen Methode.

11. Verfahren nach einem der Ansprüche 1 oder 3 bis 10, wobei das Kohlenwasserstoff-Dispergiermittel einen substituierten Rest mit der folgenden Formel umfasst:

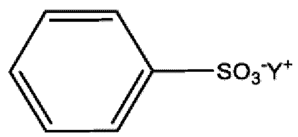

wobei $Y^+$ Wasserstoff, Ammonium, quaternäres Ammonium, Stickstoffheterocyclus, Alkalimetall oder Erdalkalimetall ist.

**12.** Verfahren nach Anspruch 1, wobei das Kohlenwasserstoff-Dispergiermittel eine Verbindung mit der Formel II ist:

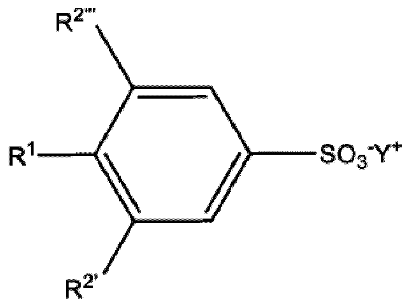

II

wobei $R^{2'}$ und $R^{2''}$ gleich oder verschieden sind und gesättigte oder ungesättigte, nicht-zyklische oder zyklische aliphatische Gruppen mit 4 bis 16 Kohlenstoffatomen sind, wobei der Prozentsatz der gesamten $CH_3$-Gruppen in Verhältnis zu den gesamten $CH_3$, $CH_2$ und CH-Gruppen in den $R^{2'}$- und $R^{2''}$-Gruppen mindestens etwa 70 % beträgt, oder $R^{2'}$ und $R^{2''}$ miteinander verbunden sein können, um einen gesättigten oder ungesättigten aliphatischen Ring zu bilden, der Ether- oder Esterbindungen enthalten kann, mit der Maßgabe, dass der prozentuale Anteil der gesamten $CH_3$-Gruppen in Verhältnis zu den gesamten $CH_3$, $CH_2$ und CH-Gruppen in dem Ring mindestens etwa 70 % beträgt;
wobei $R^1$ Wasserstoff, Methoxy, Ethoxy oder Phenoxy ist; und
wobei $Y^+$ Wasserstoff, Ammonium, quartäres Ammonium, Stickstoffheterocyclus, Alkalimetall oder Erdalkalimetall ist;
vorzugsweise wobei:

(i) in der Verbindung der Formel II $R^{2'}$, und $R^{2''}$ gleich oder verschieden sind und t-Butyl, t-Butoxy, 2,3,3-Trimethyl-2-butyl oder 2,3,3-Trimethyl-2-butoxy oder $-CO(O)C(CH_3)_3$, sind, und wobei $R^1$ Wasserstoff oder Methoxy ist und wobei $Y^+$ Wasserstoff, Ammonium oder ein Alkalimetall ist,
(ii) in der Verbindung der Formel II $R^{2'}$ und $R^{2''}$ gleich oder verschieden sind und t-Butyl oder 2,3,3-Trimethyl-2-butyl sind, $R^1$ Wasserstoff ist und $Y^+$ Wasserstoff, Ammonium oder Alkalimetall ist,
(iii) in der Verbindung der Formel II $R^{2'}$ und $R^{2''}$ gleich oder verschieden sind und t-Butyl oder 2,3,3-Trimethyl-2-butyl sind, $R^1$ Methoxy ist und $Y^+$ Wasserstoff, Ammonium oder Alkalimetall ist,
oder
(iv) in der Verbindung der Formel II $R^{2'}$ und $R^{2''}$ beide t-Butyl sind, $R^1$ Wasserstoff oder Methoxy ist und $Y^+$ Wasserstoff, Ammonium oder Alkalimetall ist.

**13.** Verfahren nach Anspruch 12, wobei die Formel II Folgende ist:

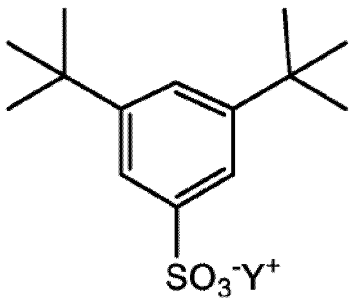

wobei $Y^+$ Wasserstoff, Ammonium oder Alkalimetall ist.

**14.** Verfahren nach Anspruch 1, wobei das Kohlenwasserstoff-Dispergiermittel eine Verbindung mit der Formel III ist:

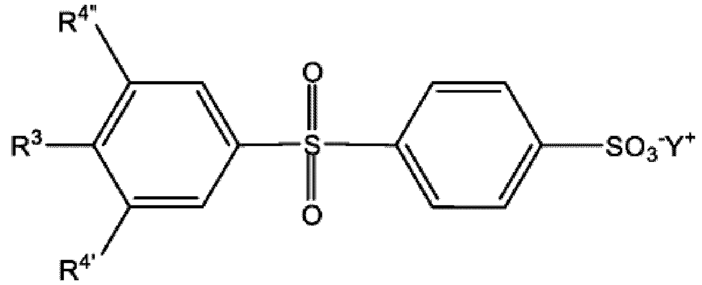

III

wobei $R^3$, $R^{4'}$ und $R^{4''}$ gleich oder verschieden sind und Wasserstoff oder gesättigte oder ungesättigte, nichtzyklische oder zyklische aliphatische Gruppen mit 4 bis 16 Kohlenstoffatomen sind, wobei der Prozentsatz der gesamten $CH_3$-Gruppen in Verhältnis zu den gesamten $CH_3$, $CH_2$ und CH-Gruppen in den $R^3$-, $R^{4'}$-, und $R^{4''}$-Gruppen mindestens 70 % beträgt, mit der Maßgabe, dass mindestens einer von $R^3$, $R^{4'}$ und $R^{4''}$ nicht Wasserstoff ist und, wenn $R^{4'}$ und $R^{4''}$ Wasserstoff sind, $R^3$ nicht Wasserstoff ist und, wenn $R^3$ Wasserstoff ist, $R^{4'}$ und $R^{4''}$ nicht Wasserstoff sind, und
$Y^+$ Wasserstoff, Ammonium, quartäres Ammonium, Stickstoffheterocyclus, Alkalimetall oder Erdalkalimetall, vorzugsweise wobei:

(i) in der Verbindung mit der Formel III, $R^3$, $R^{4'}$ und $R^{4''}$ gleich oder verschieden sind und Wasserstoff, t-Butyl, t-Butoxy, 2,3,3-Trimethyl-2-butyl oder 2,3,3-Trimethyl-2-butoxy sind, mit der Maßgabe, dass mindestens einer von $R^3$, $R^{4'}$ und $R^{4''}$ nicht Wasserstoff ist und, wenn $R^{4'}$ und $R^{4''}$ Wasserstoff sind, $R^3$ nicht Wasserstoff ist und, wenn $R^3$ Wasserstoff ist, $R^{4'}$ und $R^{4''}$ nicht Wasserstoff sind und $Y^+$ Wasserstoff, Ammonium, quartäres Ammonium, Stickstoffheterocyclus, Alkalimetall oder Erdalkalimetall ist,
(ii) in der Verbindung mit der Formel III $R^3$ t-Butyl oder 2,3,3-Trimethyl-2-butyl ist, $R^{4'}$ und $R^{4''}$ Wasserstoff sind und $Y^+$ Wasserstoff, Ammonium oder Alkalimetall ist,
(iii) in der Verbindung mit der Formel III $R^3$ t-Butyl ist und $R^{4'}$ und $R^{4''}$ Wasserstoff sind und $Y^+$ Wasserstoff, Ammonium oder Alkalimetall ist,
(iv) in der Verbindung mit der Formel III $R^{4'}$ und $R^{4''}$ gleich oder verschieden sind und t-Butyl oder 2,3,3-Trimethyl-2-butyl sind, $R^3$ Wasserstoff und $Y^+$ Wasserstoff, Ammonium oder Alkalimetall ist,
oder
(v) in der Verbindung mit der Formel III $R^{4'}$ und $R^{4''}$ t-Butyl sind, $R^3$ Wasserstoff ist und $Y^+$ Wasserstoff, Ammonium oder Alkalimetall ist.

**15.** Verfahren nach einem der vorstehenden Ansprüche, wobei der in dem wässrigen Medium enthaltene Initiator ein organisches Peroxid ist.

## Revendications

**1.** Procédé de polymérisation d'au moins un fluoromonomère dans un milieu aqueux contenant un initiateur et un agent de dispersion d'hydrocarbures pour former une dispersion aqueuse de particules de fluoropolymère, ledit agent de dispersion d'hydrocarbures comprenant un composé de formule I :

$$R\text{-}(XZ)_n \qquad I$$

dans laquelle R est une fraction hydrocarbure hydrophobe qui comprend un ou plusieurs groupes aliphatiques non-cycliques ou cycliques, saturés ou insaturés, le pourcentage de groupes $CH_3$ totaux par rapport au total des groupes $CH_3$, $CH_2$ et CH dans lesdits un ou plusieurs groupes aliphatiques étant d'au moins 70 %, ladite fraction hydrophobe étant dénuée d'unités siloxane ;
dans laquelle chaque X peut être identique ou différent et représente une fraction hydrophile ionique ;

dans laquelle chaque Z peut être identique ou différent et représente un ou plusieurs contre-ions pour ladite fraction hydrophile ionique ; et
dans laquelle n est compris entre 1 et 3.

**2.** Procédé selon la revendication 1, dans lequel ladite fraction hydrocarbure hydrophobe de l'agent de dispersion d'hydrocarbures est entièrement aliphatique et ledit agent de dispersion d'hydrocarbures présente une masse moléculaire sans un contre-ion Z d'au moins 170 g/mol.

**3.** Procédé selon la revendication 1, dans lequel ladite fraction hydrocarbure hydrophobe de l'agent de dispersion d'hydrocarbures contient au moins un groupe aromatique et ledit agent de dispersion d'hydrocarbures présente une masse moléculaire sans un contre-ion Z d'au moins 215 g/mol.

**4.** Procédé selon les revendications 1, 2 ou 3, dans lequel

(i) ladite fraction hydrocarbure hydrophobe comprend au plus 3 groupes $CH_2$ consécutifs, de préférence au plus 2 groupes $CH_2$ consécutifs, de manière davantage préférée aucuns groupes $CH_2$ consécutifs ; et/ou
(ii) ladite fraction hydrocarbure hydrophobe comprend au moins 3 groupes $CH_3$, de préférence au moins 4 groupes $CH_3$, ou 3 à 12 groupes $CH_3$.

**5.** Procédé selon l'une quelconque des revendications précédentes, dans lequel ladite fraction hydrophile ionique X est sélectionnée dans le groupe constitué de groupes acides et de leurs sels, de groupes ammonium quaternaire, de groupes d'oxyde d'amine, et de groupes tétrazole.

**6.** Procédé selon l'une quelconque des revendications précédentes, dans lequel ladite fraction hydrophile et le contre-ion XY est un groupe de formule $-A^{-}-Y^{+}$, dans laquelle $A^{-}$ est un carboxylate, un sulfonate, un sulfate, un phosphonate, ou un phosphate, de préférence un sulfonate ; et dans laquelle $Y^{+}$ est un hydrogène, un ammonium, un ammonium quaternaire, un hétérocycle d'azote, un métal alcalin ou une terre alcaline.

**7.** Procédé selon l'une quelconque des revendications précédentes, dans lequel ladite fraction hydrocarbure hydrophile est dénuée d'halogènes, et/ou dans lequel lesdits un ou plusieurs groupes aliphatiques dans ladite fraction d'hydrocarbure hydrophobe sont des groupes alkyle cycliques ou non-cycliques.

**8.** Procédé selon l'une quelconque des revendications 1 ou 3 à 7, dans lequel ladite fraction hydrocarbure hydrophobe comprend un ou plusieurs groupes aromatiques,
de préférence dans lequel :

(i) lesdits un ou plusieurs groupes aromatiques sont substitués et présentent une valeur sigma plus de Hammett supérieure à -1,0, de préférence supérieure à -0,8, de manière davantage préférée supérieure à 0, dans lequel la valeur sigma plus de Hammett du groupe aromatique substitué est calculée en ajoutant les valeurs sigma plus de Hammett pour chacun des substituants sur le cycle du groupe aromatique,
(ii) ladite fraction hydrocarbure hydrophobe est dénuée de groupes méthyle directement liés à un groupe aromatique,
(iii) ladite fraction hydrocarbure hydrophobe est dénuée d'atomes d'hydrogène benzylique, et/ou
(iv) ladite fraction hydrocarbure hydrophobe est dénuée de groupes hydroxy phénoliques.

**9.** Procédé selon l'une quelconque des revendications 1 à 8, dans lequel ladite fraction hydrocarbure hydrophobe comprend 8 à 50 atomes de carbone, de préférence 10 à 40 atomes de carbone, de manière davantage préférée de 12 à 30 atomes de carbone, de manière encore davantage préférée de 12 à 25 atomes de carbone.

**10.** Procédé selon l'une quelconque des revendications précédentes, dans lequel ladite fraction hydrocarbure hydrophobe est dénuée de liaisons carbone-hydrogène avec des énergies de dissociation de liaison pour donner un radical hydrocarbure de moins de 100 kcal/mol, et/ou
dans lequel ledit agent de dispersion d'hydrocarbure présente une perte oxydative de t-butylhydroperoxyde inférieure à 10 % en poids, comme mesuré conformément à la méthode décrite dans l'Exemple de Réactivité 1.

**11.** Procédé selon l'une quelconque des revendications 1 ou 3 à 10, dans lequel ledit agent de dispersion d'hydrocarbures comprend une fraction substituée de formule :

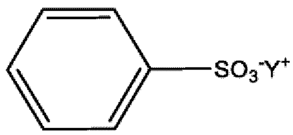

dans laquelle $Y^+$ est un hydrogène, un ammonium, un ammonium quaternaire, un hétérocycle d'azote, un métal alcalin ou une terre alcaline.

**12.** Procédé selon la revendication 1, dans lequel ledit agent de dispersion d'hydrocarbure est un composé de formule II :

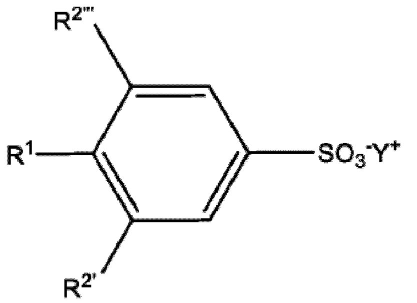

II

dans laquelle $R^{2'}$ et $R^{2''}$ sont identiques ou différents et sont des groupes aliphatiques cycliques ou non-cycliques, saturés ou insaturés, dotés de 4 à 16 atomes de carbone, le pourcentage de groupes $CH_3$ totaux relativement au total de groupes $CH_3$, $CH_2$ et CH dans les groupes $R^{2'}$ et $R^{2''}$ étant d'au moins environ 70 % ou $R^{2'}$ et $R^{2''}$ peuvent être liés ensemble pour former un cycle aliphatique saturé ou insaturé qui peut contenir des liaisons éther ou ester, à condition que le pourcentage de groupes $CH_3$ totaux relativement au total de groupes $CH_3$, $CH_2$ et CH dans ledit cycle soit d'au moins environ 70 % ;
dans laquelle $R^1$ est un hydrogène, un méthoxy, un éthoxy ou un phénoxy ; et
dans laquelle $Y^+$ est un hydrogène, un ammonium, un ammonium quaternaire, un hétérocycle d'azote, un métal alcalin ou une terre alcaline,
de préférence dans laquelle :

(i) dans le composé de formule II, $R^{2'}$ et $R^{2''}$ sont identiques ou différents et sont un t-butyle, un t-butoxy, un 2,3,3-triméthyl-2-butyle ou un 2,3,3,-triméthyl-2-butoxy, ou un $-CO(O)C(CH_3)_3$, et dans laquelle $R^1$ est un hydrogène ou un méthoxy, et $Y^+$ est un hydrogène, un ammonium ou un métal alcalin,
(ii) dans le composé de formule II, $R^{2'}$ et $R^{2''}$ sont identiques ou différents et sont un t-butyle ou un 2,3,3-triméthyl-2-butyle, $R^1$ est un hydrogène, et $Y^+$ est un hydrogène, un ammonium ou un métal alcalin,
(iii) dans le composé de formule II, $R^{2'}$ et $R^{2''}$ sont identiques ou différents et sont un t-butyle, ou un 2,3,3-triméthyl-2-butyle, $R^1$ est un méthoxy, et $Y^+$ est un hydrogène, un ammonium ou un métal alcalin,
ou
(iv) dans le composé de formule II, $R^{2'}$ et $R^{2''}$ sont tous deux un t-butyle, $R^1$ est un hydrogène ou un méthoxy, et $Y^+$ est un hydrogène, un ammonium ou un métal alcalin.

**13.** Procédé selon la revendication 12, dans lequel ladite formule II est :

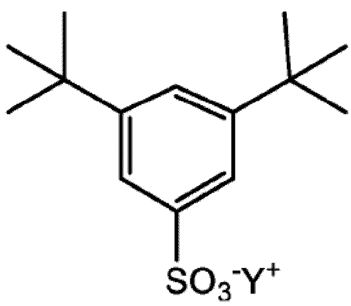

dans laquelle $Y^+$ est un hydrogène, un ammonium ou un métal alcalin.

**14.** Procédé selon la revendication 1, dans lequel ledit agent de dispersion d'hydrocarbures est un composé de formule III :

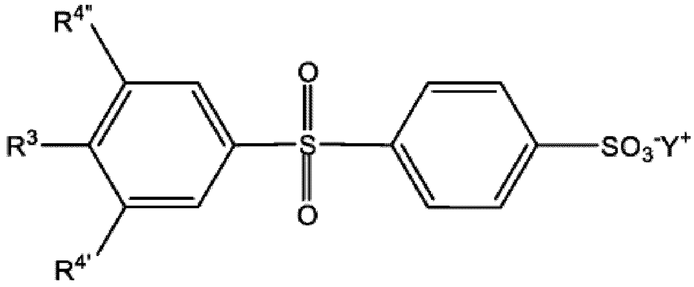

III

dans laquelle $R^3$, $R^{4'}$ et $R^{4''}$ sont identiques ou différents et sont un hydrogène ou des groupes aliphatiques cycliques ou non-cycliques, saturés ou insaturés, présentant 4 à 16 atomes de carbone, le pourcentage de groupes $CH_3$ totaux relativement au total de groupes $CH_3$, $CH_2$ et CH dans les groupes $R^3$, $R^{4'}$ et $R^{4''}$ étant d'au moins environ 70 %, à condition qu'au moins un de $R^3$, $R^{4'}$ et $R^{4''}$ ne soit pas de l'hydrogène, et, quand $R^{4'}$ et $R^{4''}$ sont un hydrogène, $R^3$ ne soit pas de l'hydrogène, et quand $R^3$ est un hydrogène, $R^{4'}$ et $R^{4''}$ ne soient pas de l'hydrogène, et

$Y^+$ est un hydrogène, un ammonium, un ammonium quaternaire, un hétérocycle d'azote, un métal alcalin, ou une terre alcaline,

de préférence, dans lequel :

(i) dans le composé de formule III, $R^3$, $R^{4'}$ et $R^{4''}$ sont identiques ou différents et sont un hydrogène, un t-butyle, un t-butoxy, un 2,3,3-triméthyl-2-butyle, ou un 2,3,3-triméthyl-2-butoxy, à condition qu'au moins un de $R^3$, $R^{4'}$ et $R^{4''}$ ne soit pas de l'hydrogène et, quand $R^{4'}$ et $R^{4''}$ sont un hydrogène, $R^3$ ne soit pas de l'hydrogène, et quand $R^3$ est un hydrogène, $R^{4'}$ et $R^{4''}$ ne soient pas de l'hydrogène, et $Y^+$ est un hydrogène, un ammonium, un ammonium quaternaire, un hétérocycle d'azote, un métal alcalin ou une terre alcaline.

(ii) dans le composé de formule III, $R^3$ est un t-butyle ou un 2,3,3-triméthyl-2-butyle, $R^{4'}$ et $R^{4''}$ sont un hydrogène, et $Y^+$ est un hydrogène, un ammonium ou un métal alcalin,

(iii) dans le composé de formule III, $R^3$ est un t-butyle et $R^{4'}$ et $R^{4''}$ sont un hydrogène, et $Y^+$ est un hydrogène, un ammonium ou un métal alcalin,

(iv) dans le composé de formule III, $R^{4'}$ et $R^{4''}$ sont identiques ou différents et sont un t-butyle ou un 2,3,3-triméthyl-2-butyle, $R^3$ est un hydrogène et $Y^+$ est un hydrogène, un ammonium, ou un métal alcalin,

ou

(v) dans le composé de formule III, $R^{4'}$ et $R^{4''}$ sont un t-butyle, $R^3$ est un hydrogène et $Y^+$ est un hydrogène, un ammonium ou un métal alcalin.

**15.** Procédé selon l'une quelconque des revendications précédentes, dans lequel ledit initiateur contenu dans le milieu aqueux est un peroxyde organique.

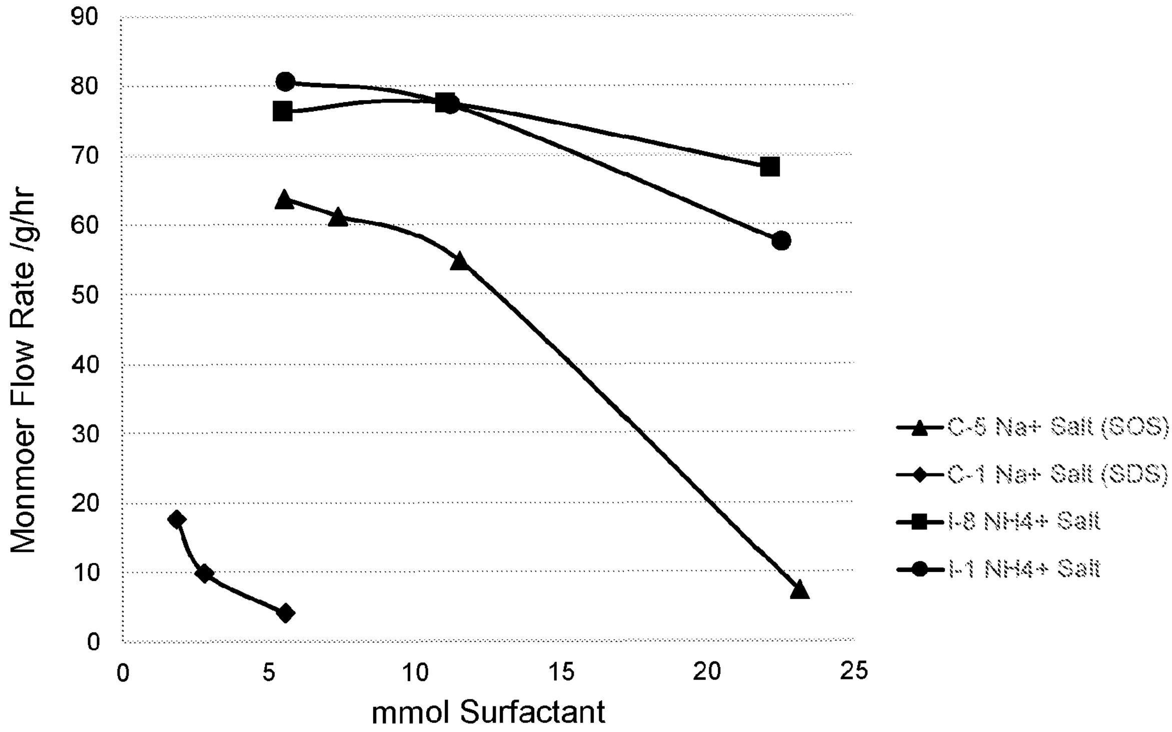
90
80
70
60
50
40
30
20
10
0
Monmoer Flow Rate /g/hr
0
5
10
15
20
25
mmol Surfactant
C-5 Na+ Salt (SOS)
C-1 Na+ Salt (SDS)
I-8 NH4+ Salt
I-1 NH4+ Salt

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7122610 B, Wille
- US 8080621 B
- US 8158734 B
- US 8338518 B, Amin-Sanayei
- US 6512063 B
- US 7521513 B, Tang
- WO 2012064846 A1
- WO 2012064858 A1
- WO 2012064841 A1, Brothers
- JP 2002308914 A
- US 3282875 A
- US 4358545 A
- US 4940525 A
- US 4552631 A
- US 5637748 A
- US 6300445 B
- US 6177196 B
- US 7763680 B
- US 4036802 A
- US 703185 A
- US 8153738 B, Leffew
- US 6150426 A, Curtin
- US 8563670 B
- US 9676929 B, Brothers
- US 7897682 B, Brothers
- US 9732212 B
- US 6916853 B


### Non-patent literature cited in the description


- **PUTS et al.** *Chem. Rev.*, 2019, vol. 119, 1763-1805
- **HANSCH et al.** *Chemical Reviews*, 1991, vol. 91, 165-195
- **KASAI**. *J. Appl. Polymer Sci.*, 1995, vol. 57, 797
- *Org. Synth.*, 1978, vol. 58, 143-146
- *Org. Process Res. Dev.*, 2007, vol. 11, 431-440
- *New J. Chem.*, 2003, vol. 27, 1603-1608
- *Tet. Lett*, 1981, vol. 22, 5023-5026
- *J. Chem. Soc.*, 1954, 2180-2200
- *Tetrahedron*, 1960, vol. 11, 39-51
- *Tetrahedron Letters*, 2017, vol. 58 (24), 2340-2343
- *Journal of Organic Chemistry*, 1966, vol. 31 (4), 1090-3
- *Journal of Organic Chemistry*, 1988, vol. 53 (19), 4626-8
- *Journal of Organic Chemistry*, 1990, vol. 55 (6), 1928-32
- *Journal of Organic Chemistry*, 1966, vol. 31 (1), 137-42